# EUROPÄISCHE PATENTSCHRIFT

(11) **EP 2 616 425 B1**
(45) Veröffentlichungstag und Bekanntmachung des Hinweises auf die Patenterteilung: **10.02.2016**
(21) Anmeldenummer: 11758432.6
(22) Anmeldetag: 09.09.2011
(51) Int. Cl.: C07C 45/38, C07C 51/353, C07C 47/04, C07C 47/052

(54) **VERFAHREN ZUR HERSTELLUNG VON ACRYLSÄURE AUS METHANOL UND ESSIGSÄURE**
METHOD FOR PRODUCING ACRYLIC ACID FROM METHANOL AND ACETIC ACID
PROCÉDÉ DE PRODUCTION D'ACIDE ACRYLIQUE À PARTIR D'ÉTHANOL ET D'ACIDE ACÉTIQUE

(30) Priorität: 16.09.2010 US 383363 P; 16.09.2010 DE 102010040921
(43) Veröffentlichungstag der Anmeldung: 24.07.2013
(73) Patentinhaber: BASF SE, 67056 Ludwigshafen (DE)
(72) Erfinder: HERZOG, Stefanie, 68163 Mannheim (DE); ALTWASSER, Stefan, 67157 Wachenheim (DE); MÜLLER-ENGEL, Klaus Joachim, 76297 Stutensee (DE)
(86) Internationale Anmeldenummer: PCT/EP2011/065593
(87) Internationale Veröffentlichungsnummer: WO 2012/034929

(56) Entgegenhaltungen:
- EP-A1- 0 122 782
- EP-A2- 2 213 370
- DE-A1- 2 145 851
- US-A- 4 677 225
- DATABASE WPI Week 200950 Thomson Scientific, London, GB; AN 2009-L06391 XP002670109, & CN 101 462 069 A (SINOPEC CORP) 24. Juni 2009 (2009-06-24)

## Beschreibung

Vorliegende Erfindung betrifft ein Verfahren zur Herstellung von Acrylsäure aus Methanol und Essigsäure. Außerdem betrifft vorliegende Erfindung die Herstellung von Folgeprodukten aus so erzeugter Acrylsäure.

Derzeit erfolgt die großtechnische Herstellung von Acrylsäure im Wesentlichen ausschließlich durch heterogen katalysierte zweistufige Partialoxidation von Propylen (siehe beispielsweise DE-A 103 36 386).

Ein Vorteil dieser Verfahrensweise besteht darin, dass sie eine vergleichsweise hohe Zielproduktselektivität bezogen auf umgesetztes Propylen aufweist, was bei Kreisfahrweise von im einfachen Durchgang nicht umgesetztem Propylen hohe Acrylsäureausbeuten aus dem eingesetzten Propylen ermöglicht. Darüber hinaus weist Propylen eine ausgesprochen wirtschaftliche Rückwärtsintegration zum fossilen Grundrohstoff Erdöl auf (d.h., Propylen ist zu vergleichsweise geringen Herstellkosten aus Erdöl zu erzeugen), was insgesamt eine kostengünstige Acrylsäureherstellung ermöglicht.

Angesichts der absehbaren Verknappung der fossilen Ressource Erdöl besteht zukünftig jedoch Bedarf an Verfahren zur Herstellung von Acrylsäure aus Rohstoffen, die auch ohne Rückwärtsintegration derselben zum fossilen Grundrohstoff Erdöl vergleichsweise wirtschaftlich durchführbar sind, und die gleichzeitig eine Rückwärtsintegration ihrer Rohstoffe zu Grundrohstoffen aufweisen, deren zeitliche Reichweite diejenige von Erdöl überragt.

Die WO 2005/093010 sieht das Propylen selbst als einen solchen Rohstoff. Sie schlägt vor, auch zukünftig an der zweistufigen heterogen katalysierten partiellen Gasphasenoxidation von Propylen zu Acrylsäure festzuhalten, das dabei benötigte Propylen jedoch ausgehend von Methanol zu gewinnen. Der Vorteil einer solchen Verfahrensweise besteht darin, dass Methanol sowohl ausgehend von fossilen Grundrohstoffen wie Kohle (beispielsweise Braunkohle und Steinkohle; vgl. z.B. WO 2010/072424) und Erdgas (vgl. z.B. WO 2010/067945), die beide über eine größere zeitliche Reichweite wie Erdöl verfügen, als auch ausgehend von dem nachwachsenden Grundrohstoff Biomasse sowie auch unmittelbar aus dem in der Erdatmosphäre enthaltenen Kohlendioxid (jeweils gegebenenfalls unter Mitverwendung von Wasserdampf bzw. molekularem Wasserstoff) zugänglich ist (vgl. z.B. G.A. Olah et al., Beyond Oil and Gas; The Methanol Economy, Wiley-VCH, 2009).

Nachteilig an der in der WO 2005/093010 vorgeschlagenen Verfahrensweise ist jedoch, dass die Selektivität der Propylenerzeugung ausgehend von Methanol mit den derzeit bekannten Herstellverfahren, bezogen auf umgesetztes Methanol, weniger als 70 mol-% beträgt, was nicht zu befriedigen vermag (neben Propylen- erfolgt z.B. auch Ethylen- und Butylenbildung).

Unter fossilen Grundrohstoffen sollen in dieser Schrift Grundrohstoffe verstanden werden, die wie z.B. Braunkohle, Steinkohle, Erdgas und Erdöl in geologischer Vorzeit aus Abbauprodukten von toten Pflanzen und toten Tieren entstanden sind.

Im Unterschied dazu sollen in dieser Schrift unter nachwachsenden Rohstoffen solche Rohstoffe verstanden werden, die aus frischer Biomasse, das heißt aus neu (in der Gegenwart) und in der Zukunft gewachsenem (neuzeitlichem) pflanzlichem und tierischem Material, gewonnen werden.

Es wurde auch schon vorgeschlagen (beispielsweise in der WO 2008/023040) Acrylsäure und deren Folgeprodukte ausgehend von dem nachwachsenden Rohstoff Glyzerin herzustellen. Nachteilig an einer solchen Verfahrensweise ist jedoch, dass Glyzerin als nachwachsender Rohstoff im Wesentlichen nur als Koppelprodukt der Biodieselherstellung wirtschaftlich zugänglich ist. Dies ist insofern nachteilig, als die derzeitige Energiebilanz der Biodieselherstellung nicht zu befriedigen vermag.

Weiterhin wird im Stand der Technik die Herstellung von Acrylsäure aus Propan vorgeschlagen (beispielsweise in der DE-A 102006024901), welches einen Rohbestandteil von Erdgas bildet. Nachteilig an einer solchen Herstellweise von Acrylsäure ist jedoch, zum einen die vergleichsweise hohe Reaktionsträgheit von Propan, sowie der Sachverhalt, dass Propan auch einen gut handhabbaren nachgefragten Energieträger bildet.

EP 2 213 370 offenbart ein Verfahren zur Herstellung von Acrylsäure aus Formaldehyd oder Methanol und Essigsäure.

Die Aufgabe der vorliegenden Erfindung bestand daher darin, ein alternatives Verfahren zur Herstellung von Acrylsäure zur Verfügung zu stellen, das die beschriebenen Nachteile der Verfahren des Standes der Technik nicht hat und ausgehend von den zu seiner Herstellung eingesetzten Rohstoffen insbesondere eine befriedigende Selektivität der Zielproduktbildung aufweist.

Demgemäß wird ein Verfahren zur Herstellung von Acrylsäure aus Methanol und Essigsäure zur Verfügung gestellt.

Der Charme einer solchen Verfahrensweise besteht insbesondere darin, dass die Essigsäure selbst in einfacher und großtechnisch erprobter Weise ausgehend von Methanol durch Carbonylierung desselben mit Kohlenmonoxid erhältlich ist (vgl. z.B. Industrielle Organische Chemie, Klaus Weissermehl und Hans-Jürgen Arpe, Wiley-VCH, Weinheim, 5. Auflage (1998), S. 194 bis 198).

Insgesamt wird somit im Wesentlichen ein Verfahren zur Herstellung von Acrylsäure aus Methanol zur Verfügung gestellt. Im Unterschied zum ebenfalls auf dem Rohstoff Methanol basierenden Verfahren der WO 2005/093010 verfügt das erfindungsgemäße Verfahren über eine erhöhte Selektivität der Acrylsäurebildung bezogen auf die umgesetzte Menge an Methanol. Die Vorteilhaftigkeit eines im Wesentlichen ausschließlich auf dem Rohstoff Methanol basierten Acrylsäureherstellungsverfahrens ist nicht zuletzt darin begründet, dass das Methanol über Synthesegas (Gasgemische aus Kohlenmonoxid und molekularem Wasserstoff) im Prinzip aus allen kohlenstoffhaltigen fossilen Grundstoffen und allen kohlenstoffhaltigen nachwachsenden Rohstoffen erzeugt werden kann (der erforderliche molekulare Wasserstoff kann wie im Fall des Methans (ein Verfahren zur Gewinnung von Methan aus Biogas bzw. Biomasse beschreibt beispielsweise die DE-A 102008060310 bzw. EP-A 2220004) bereits im Kohlenstoffträger enthalten sein; alternativ steht als Wasserstoffquelle Wasser zur Verfügung, aus dem der molekulare Wasserstoff z.B. mittels Elektrolyse gewonnen werden kann; Sauerstoffquelle ist in der Regel Luft; vgl. z.B. WO 10-060236 und WO 10-060279). Als nachwachsender kohlenstoffhaltiger Rohstoff eignet sich z.B. Lignocellulose zur Synthesegaserzeugung (vgl. z.B. WO 10-062936). Auch kann zur Synthesegasgewinnung die Pyrolyse von Biomasse direkt mit einem Wasserdampfreforming gekoppelt werden.

Gegenstand der vorliegenden Erfindung ist somit ein Verfahren zur Herstellung von Acrylsäure aus Methanol und Essigsäure, das folgende Maßnahmen umfasst:
- durch eine erste Reaktionszone A, die mit wenigstens einem Oxidationskatalysator A beschickt ist, wird ein Strom eines die Reaktanden Methanol und molekularer Sauerstoff sowie wenigstens ein von Wasserdampf verschiedenes inertes Verdünnungsgas enthaltenden Reaktionsgaseingangsgemischs A hindurchgeführt und beim Durchströmen der Reaktionszone A im Reaktionsgaseingangsgemisch A enthaltenes Methanol heterogen katalysiert zu Formaldehyd und Wasserdampf oxidiert, so dass ein Formaldehyd, Wasserdampf, wenigstens ein von Wasserdampf verschiedenes inertes Verdünnungsgas sowie gegebenenfalls überschüssigen molekularen Sauerstoff enthaltendes Produktgasgemisch A entsteht und ein Strom an Produktgasgemisch A die Reaktionszone A verlässt, wobei dem durch die Reaktionszone A strömenden Reaktionsgasgemisch A auf seinem Weg durch die Reaktionszone A wahlweise weiterer molekularer Sauerstoff und/oder weiteres inertes Verdünnungsgas zugeführt werden kann,
- wahlweise wird der die Reaktionszone A verlassende Strom an Produktgasgemisch A einer Trennzone T* zugeführt und in der Trennzone T* im Produktgasgemisch A gegebenenfalls noch enthaltenes nicht umgesetztes Methanol vom Produktgasgemisch A abgetrennt, wobei ein Formaldehyd enthaltendes Produktgasgemisch A* verbleibt und ein Strom an Produktgasgemisch A* die Reaktionszone A verlässt,
- aus dem Strom an Produktgasgemisch A oder aus dem Strom an Produktgasgemisch A* sowie wenigstens einem weiteren, Essigsäure enthaltenden, Stoffstrom wird ein Strom eines Essigsäure, Wasserdampf, wenigstens ein von Wasserdampf verschiedenes inertes Verdünnungsgas, Formaldehyd und gegebenenfalls molekularen Sauerstoff enthaltenden Reaktionsgaseingangsgemischs B erzeugt, in welchem die enthaltene molare Menge n_{HAc} an Essigsäure größer ist, als die in ihm enthaltene molare Menge n_{Fd} an Formaldehyd,
- durch eine zweite Reaktionszone B, die mit wenigstens einem Aldolkondensationskatalysator B beschickt ist, wird der Strom des Reaktionsgaseingangsgemischs B hindurchgeführt und beim Durchströmen der Reaktionszone B im Reaktionsgaseingangsgemisch B enthaltenes Formaldehyd mit im Reaktionsgaseingangsgemisch B enthaltener Essigsäure heterogen katalysiert zu Acrylsäure und H₂O kondensiert, so dass ein Acrylsäure, Essigsäure, Wasserdampf, wenigstens ein von Wasserdampf verschiedenes inertes Verdünnungsgas sowie gegebenenfalls molekularen Sauerstoff enthaltendes Produktgasgemisch B entsteht und ein Strom an Produktgasgemisch B die Reaktionszone B verlässt, wobei dem durch die Reaktionszone B strömenden Reaktionsgasgemisch B auf seinem Weg durch die Reaktionszone B wahlweise weiterer molekularer Sauerstoff und/oder weiteres inertes Verdünnungsgas zugeführt werden kann,
- der die Reaktionszone B verlassende Strom an Produktgasgemisch B wird einer Trennzone T zugeführt und in der Trennzone T in wenigstens drei Stoffströme X, Y und Z aufgetrennt, wobei
- der im Stoffstrom X enthaltene Acrylsäurestrom größer ist, als der in den Stoffströmen Y und Z zusammengenommen enthaltene Acrylsäurestrom,
- der im Stoffstrom Y enthaltene Essigsäurestrom größer ist, als der in den Stoffströmen X und Z zusammengenommen enthaltene Essigsäurestrom,
- der im Stoffstrom Z enthaltene Strom an von Wasserdampf verschiedenem inertem Verdünnungsgas größer ist, als der in den Stoffströmen X und Y zusammengenommen enthaltene Stoffstrom an von Wasserdampf verschiedenem inertem Verdünnungsgas,
   und
- der Stoffstrom Y wird in die Reaktionszone B rückgeführt und zur Erzeugung des Reaktionsgaseingangsgemischs B mitverwendet.

Ein wesentlicher Vorteil der erfindungsgemäßen Verfahrensweise ist darin begründet, dass das im Produktgasgemisch A enthaltene Formaldehyd nicht aus dem Produktgasgemisch A abgetrennt werden muss, um es zur Erzeugung des Reaktionsgaseingangsgemischs B einsetzen zu können.

Vielmehr kann der die Reaktionszone A verlassende, Formaldehyd enthaltende Strom an Produktgasgemisch A als solcher (d.h., ohne zuvor an ihm ein Abtrennverfahren durchzuführen) verwendet werden, um das Reaktionsgaseingangsgemisch B zu erzeugen. In der Regel wird man zu diesem Zweck das Produktgasgemisch A beim Verlassen der Reaktionszone A zunächst abkühlen (abschrecken), um unerwünschte Folgereaktionen im Produktgasgemisch A vorab seiner Einbringung in das Reaktionsgaseingangsgemisch B zu mindern. In typischer Weise wird möglichst rasch auf Temperaturen von 150 bis 350°C, bzw. 200 bis 250°C abgekühlt.

Wahlweise kann aus dem Produktgasgemisch A aber auch in einer Trennzone T* zunächst eine Teil- oder die Gesamtmenge von darin gegebenenfalls noch enthaltenem, in der Reaktionszone A nicht umgesetztem, Methanol abgetrennt, und anschließend das dabei verbleibende, Formaldehyd enthaltende Produktgasgemisch A* (das im Rahmen der Abtrennung den flüssigen Aggregatzustand durchlaufen kann) zur Erzeugung des Reaktionsgaseingangsgemischs B verwendet werden. Anwendungstechnisch vorteilhaft wird man die Abtrennung rektifikativ vornehmen. Zu diesem Zweck kann das Produktgasgemisch A, wahlweise nach zuvor erfolgter direkter oder indirekter Abkühlung, der entsprechenden mit Kühlkreisen versehenen Rektifikationskolonne gasförmig zugeführt werden. Selbstverständlich können aber aus dem Produktgasgemisch A heraus zunächst diejenigen Bestandteile, deren Siedepunkt bei Normaldruck (10⁵ Pa) kleiner oder gleich groß wie der Siedepunkt von Formaldehyd ist, in die flüssige Phase überführt (z.B. durch Kondensation) und die Rektifikation aus der flüssigen Phase heraus vorgenommen werden. In der Regel geht mit einer solchen Methanolabtrennung auch eine Abtrennung von im Produktgasgemisch A enthaltenem Wasserdampf einher. Zum Zweck der vorstehend erwähnten Direktkühlung kann z.B. der Rektifikationskolonne aus deren Sumpfbereich entnommene und gegebenenfalls durch indirekten Wärmeaustausch zusätzlich abgekühlte Flüssigphase eingesetzt werden, die über entsprechende Düsen in feinteilige Tröpfchen versprüht wird, die die erforderliche große Wärmeaustauscherfläche für das heiße Produktgasgemisch A zur Verfügung stellen. Das abgetrennte Methanol wird man erfindungsgemäß zweckmäßig in die Reaktionszone A rückführen und zur Erzeugung des Reaktionsgaseingangsgemischs A mitverwenden (vgl. DE-A 1618413). Eine Abtrennung von Methanol vom Produktgasgemisch A vorab dessen Verwendung zur Erzeugung des Reaktionsgaseingangsgemischs B wird in der Regel dann vorgenommen, wenn die Reaktionszone A so gestaltet wird, dass der resultierende Umsatz an Methanol in der Reaktionszone A, bezogen auf einen Einmaldurchgang des Produktgasgemischs A durch die Reaktionszone A, nicht mehr als 90 mol-% beträgt. Selbstverständlich kann eine solche Methanolabtrennung aber auch bei entsprechenden Methanolumsätzen von nicht mehr als 95 mol-% angewendet werden. Beispielsweise kann eine solche Methanolabtrennung wie in Ullmann's Encyclopedia of Industrial Chemistry, Vol. A11, 5th Ed., VCH Weinheim auf Seite 626 ff beschrieben vorgenommen werden.

Die zur Beschickung der Reaktionszone A besonders geeigneten Oxidationskatalysatoren A lassen sich im Wesentlichen in zwei Gruppen aufteilen.

Die erste der beiden Gruppen umfasst die sogenannten Silberkontakte (Silberkatalysatoren), die als Aktivmasse elementares Silber aufweisen, dessen Reinheit vorzugsweise ≥ 99,7 Gew.-% vorteilhaft ≥ 99,8 Gew.-%, vorzugsweise ≥ 99,9 Gew.-% und ganz besonders bevorzugt ≥ 99,99 Gew.-% beträgt. Die zugehörigen Verfahren der heterogen katalysierten partiellen Gasphasenoxidation von Methanol zu Formaldehyd an diesen "Silberkontakten" werden im Stand der Technik als Silberverfahren bezeichnet (vgl. z.B. "A. Nagy, G. Mestl: High temperature partial oxidation reactions over silver catalysts, Appl. Catal. 188 (1999), S. 337 bis 353", "H. Schubert, U. Tegtmayr, R. Schlögl: On the mechanism of the selective oxidation of methanol over elemental silver, Catalyst Letters, 28 (1994), S. 383 bis 395", "L. Lefferts, Factors controlling the selectivity of silver catalysts for methanol oxidation, Dissertation, Universität Twente (1987)" und die DE-A 2334981).

Erfindungsgemäß vorteilhafte Silber-Oxidationskatalysatoren A zur Beschickung der Reaktionszone A sind z.B. in Ullmann's Encyclopedia of Industrial Chemistry, Vol. A11, 5th Ed., VCH, Weinheim, S. 619 bis 652, oder in Encyclopedia of Chemical Technology, Vol. 11, 4th Ed., Wiley & Sons, New York, S. 929 bis 949, in der DE-AS 1231229, in der DE-AS 1294360, in der DE-A 1903197 und in der BE-Patentschrift 683130 offenbart. Üblicherweise handelt es sich um durch Elektrolyse wässriger Silbersalzlösungen abgeschiedene Kristalle (deren Form auch rund sein kann) von elementarem Silber (vorzugsweise der oben genannten Reinheit), die auf einer perforierten Unterlage (z.B. eine Lochplatte, ein Sieb oder ein Maschennetzwerk (vorzugsweise ebenfalls aus Silber gefertigt)) als Katalysatorfestbett aufgeschüttet werden (typische Schütthöhen betragen 10 bis 50 mm, häufig 15 bis 30 mm). Der Gesamtgehalt an im katalytisch aktiven Silber von Ag verschiedenen elementar vorliegenden Metallen (z.B. Cu, Pd, Pb, Bi, Fe, Pt und Au) beträgt vorteilhaft ≤ 2000 Gew.ppm, besser ≤ 1000 Gew.ppm, vorzugsweise ≤ 100 Gew.ppm und besonders bevorzugt ≤ 50 Gew.ppm oder ≤ 30 Gew.ppm. Die Längstausdehnung der Silberkristalle liegt üblicherweise im Bereich von 0,1 bis 5 mm und nimmt vorzugsweise in Strömungsrichtung des Reaktionsgasgemischs A zu. Vorzugsweise wird das Silberfestbett als ein Zweischichtenbett ausgeführt, wobei die untere Schicht z.B. 15 bis 40 mm, vorzugsweise 20 bis 30 mm stark ist und zu wenigstens 50 Gew.-% aus Silberkristallen der Korngröße 1 bis 4 mm, vorzugsweise 1 bis 2,5 mm besteht. Die obere Schicht kann z.B. über eine Stärke (Schichtdicke) von 0,75 bis 3 mm, vorzugsweise 1 bis 2 mm verfügen und aus Kristallen mit Korngrößen (Längstausdehnungen) von 0,1 bis 1 mm, vorzugsweise 0,2 bis 0,75 mm bestehen. Die Anströmung mit Reaktionsgaseingangsgemisch A erfolgt in diesem Fall von oben nach unten.

Um einem die Performance des Katalysatorfestbetts mindernden Zusammensintern der Silberkristalle mit zunehmender Betriebsdauer (bei vergleichsweise hohen Reaktionstemperaturen) entgegenzuwirken, empfiehlt die WO 2010/022923 die Silberkristalle mit einer dünnen porösen Schicht aus oxidischem Material wenigstens eines der Elemente Al, Si, Zr und Ti zu überziehen (die Schichtdicke kann 0,3 bis 10 µm, vorzugsweise 1,0 bis 5,0 µm, besonders bevorzugt 2,0 bis 4,0 µm und am besten etwa 3 µm betragen), und auf diese Weise eine Verlängerung der Standzeit des Katalysatorfestbetts zu erreichen.

Der Gehalt des Reaktionsgaseingangsgemischs A an Methanol beträgt beim Silberverfahren normalerweise wenigstens 5 Vol.-%, meist wenigstens 10 Vol.-% und kann sich auf bis zu 60 Vol.-% erstrecken. Vorzugsweise beträgt der vorgenannte Methanolgehalt beim Silberverfahren 15 bis 50 Vol.-% und besonders bevorzugt 20 bis 40 bzw. bis 30 Vol.-%.

Darüber hinaus beträgt das Verhältnis der im Reaktionsgaseingangsgemisch A enthaltenen molaren Menge an molekularem Sauerstoff (n_{O}) zur im Reaktionsgaseingangsgemisch A enthaltenen molaren Menge an Methanol (n_{Me}), n_{O} : n_{Me}, beim Silberverfahren normalerweise weniger als 1 (< 1), vorzugsweise ≤ 0,8. Besonders bevorzugt wird es 0,2 bis 0,6 und ganz besonders bevorzugt 0,3 bis 0,5 oder 0,4 bis 0,5 betragen. In der Regel wird n_{O} : n_{Me} beim Silberverfahren nicht weniger als 0,1 betragen.

Als ein inertes Verdünnungsgas soll in dieser Schrift ein Reaktionsgaseingangsgemischbestandteil verstanden werden, der sich unter den Bedingungen in der jeweiligen der beiden Reaktionszonen A, B als inert verhält und -jeder inerte Reaktionsgasbestandteil für sich betrachtet - in der jeweiligen Reaktionszone zu mehr als 95 mol-%, vorzugsweise zu mehr als 97, oder zu mehr als 98, oder zu mehr als 99 mol-% chemisch unverändert erhalten bleibt.

Beispiele für inerte Verdünnungsgäse sowohl für die Reaktionszone A als auch die Reaktionszone B sind H₂O, CO₂, N₂ und Edelgase wie Ar sowie Gemische aus den vorgenannten Gasen. Den inerten Verdünnungsgasen wächst unter anderem die Aufgabe zu, in der Reaktionszone A frei werdende Reaktionswärme aufzunehmen, und dadurch die sogenannte Heißpunkttemperatur in der Reaktionszone A zu begrenzen sowie das Zündverhalten des Reaktionsgasgemischs A günstig zu gestalten. Unter der Heißpunkttemperatur wird dabei die höchste Temperatur des Reaktionsgasgemischs A auf seinem Weg durch die Reaktionszone A verstanden.

Als von Wasserdampf verschiedenes inertes Verdünnungsgas wird beim Silberverfahren für das Reaktionsgaseingangsgemisch A molekularer Stickstoff bevorzugt. Dessen Vorteilhaftigkeit beruht nicht zuletzt darauf, dass molekularer Stickstoff in Luft als natürlicher Begleiter von molekularem Sauerstoff auftritt, was Luft zu einer bevorzugten Quelle des in der Reaktionszone A benötigten molekularen Sauerstoffs macht. Selbstverständlich kann beim Silberverfahren erfindungsgemäß aber auch reiner molekularer Sauerstoff, oder mit molekularem Sauerstoff angereicherte Luft, oder ein sonstiges Gemisch aus molekularem Sauerstoff und inertem Verdünnungsgas als Sauerstoffquelle verwendet werden.

In typischer Weise enthält das Reaktionsgaseingangsgemisch A beim Silberverfahren 20 bis 80 Vol.-%, oder 30 bis 70 Vol.-%, oder 40 bis 60 Vol.-% an inertem Verdünnungsgas. Selbiges kann völlig frei von Wasserdampf sein. D.h., das Reaktionsgaseingangsgemisch A kann beim Silberverfahren 20 bis 80 Vol.-%, oder 30 bis 70 Vol.-%, oder 40 bis 60 Vol.-% an molekularem Stickstoff enthalten.

Grundsätzlich kann das Reaktionsgaseingangsgemisch A beim Silberverfahren > 0 bis 50 Vol.-% an H₂O enthalten.

Wasserdampf ist als Bestandteil des Reaktionsgaseingangsgemischs A insofern vorteilhaft, als Wasserdampf im Vergleich mit z.B. N₂ und Edelgasen eine erhöhte molare Wärmekapazität aufweist. In der Regel ist Wasserdampf als Bestandteil des Reaktionsgasgemischs A auch förderlich für die Desorption des gewünschten Partialoxidationsprodukts von der Katalysatoroberfläche, was sich positiv auf die Selektivität der erwünschten Produktbildung auswirkt. Da ein Beisein von Wasserdampf in der Reaktionszone B die gewünschte Aldolkondensation in der Regel jedoch in gewissem Umfang mindert und außerdem den erforderlichen Energieaufwand zur Abtrennung eines Acrylsäure angereichert enthaltenden Stoffstroms X aus dem Produktgasgemisch B in der Trennzone T erhöht (Acrylsäure weist eine erhöhte Affinität zu H₂O auf), werden erfindungsgemäß zweckmäßig vergleichsweise beschränkte Wasserdampfgehalte des Reaktionsgaseingangsgemischs A bevorzugt.

D.h., vorzugsweise enthält das Reaktionsgaseingangsgemisch A beim Silberverfahren ≥ 5 bis 45 Vol.-% an H₂O, mit Vorteil ≥ 10 bis 40 Vol-% und besonders vorteilhaft 15 bis 35 Vol.-%, oder 20 bis 30 Vol.-% an H₂O. Der Siedepunkt der von Wasserdampf verschiedenen inerten Verdünnungsgase (bezogen auf einen Druck von 10⁵ Pa = 1 bar) liegt normalerweise deutlich unterhalb desjenigen von Wasserdampf (bezogen auf denselben Druck), weshalb der Stoffstrom Z beim erfindungsgemäßen Verfahren die von Wasserdampf verschiedenen inerten Verdünnungsgase (z.B. N₂ und CO₂) in der Regel angereichert enthält.

Anwendungstechnisch vorteilhaft wird man die Auftrennung des Produktgasgemischs B in der Trennzone T so durchführen, dass der Stoffstrom Z auch einen angemessenen Anteil an Wasserdampf aufweist. Im letzteren Fall kann der Stoffstrom Z sowohl als Quelle für von Wasserdampf verschiedene Inertgase als auch für Wasserdampf fungieren.

Als Inertgasquelle kann beim Silberverfahren für das Reaktionsgaseingangsgemisch A somit auch der in der Trennzone T anfallende Stoffstrom Z verwendet werden. Anwendungstechnisch zweckmäßig wird daher beim Silberverfahren ein Teilstrom des Stoffstroms Z zur Erzeugung des Reaktionsgaseingangsgemischs A in die Reaktionszone A rückgeführt (Kreisgasfahrweise). Selbstverständlich kann ein Teilstrom des Stoffstroms Z auch in die Reaktionszone B rückgeführt werden.

D.h., erfindungsgemäß geeignete Reaktionsgaseingangsgemische A können beim Silberverfahren z.B. 10 bis 50 Vol.-% H₂O und 20 bis 60 Vol.-% an von Wasserdampf verschiedenem inertem Verdünnungsgas (z.B. N₂, oder N₂+CO₂, oder N₂+Edelgas (z.B. Ar), oder N₂+CO₂+Edelgas (z.B. Ar)) enthalten.

Selbstverständlich können Reaktionsgaseingangsgemische A beim Silberverfahren auch 10 bis 40 Vol.-% H₂O und 30 bis 60 Vol.-% an von Wasserdampf verschiedenen inerten Verdünnungsgasen (z.B. die vorgenannten) enthalten.

Natürlich kann das Reaktionsgaseingangsgemisch A beim Silberverfahren aber auch 20 bis 40 Vol.-% H₂O und 30 bis 50 Vol.-% an von Wasserdampf verschiedenen inerten Verdünnungsgasen (z.B. die vorgenannten) enthalten.

Prinzipiell kann beim Silberverfahren das Reaktionsgasgemisch A durch die Reaktionszone A sowohl hindurchgedrückt als auch hindurchgesaugt werden. Dem entsprechend kann der Arbeitsdruck beim Silberverfahren innerhalb der Reaktionszone A sowohl ≥ 10⁵ Pa als auch < 10⁵ Pa betragen. Anwendungstechnisch zweckmäßig wird der Arbeitsdruck beim Silberverfahren in der Reaktionszone A 10³ bis 10⁶ Pa, vorzugsweise 10⁴ bis 5·10⁵ Pa, besonders bevorzugt 10⁴ bis 2·10⁵ Pa und besonders bevorzugt 0,5·10⁵ Pa bis 1,8·10⁵ Pa betragen.

Die Temperatur des Reaktionsgasgemischs A (der Begriff des Reaktionsgasgemischs A umfasst in vorliegender Anmeldung alle in der Reaktionszone A auftretenden Gasgemische, die zwischen dem Reaktionsgaseingangsgemisch A und dem Produktgasgemisch A liegen) wird beim Silberverfahren innerhalb der Reaktionszone A normalerweise im Bereich von 400 bis 800°C, vorzugsweise im Bereich von 450 bis 800°C und besonders bevorzugt im Bereich von 500 bis 800°C liegen. Der Begriff der Temperatur des Reaktionsgasgemischs A (in dieser Schrift auch als Reaktionstemperatur in der Reaktionszone A bezeichnet) meint dabei in erster Linie diejenige Temperatur, die das Reaktionsgasgemisch A ab Erreichen eines Umsatzes des im Reaktionsgaseingangsgemisch A enthaltenen Methanols von wenigstens 5 mol-% bis zum Erreichen des entsprechenden Endumsatzes des Methanols innerhalb der Reaktionszone A aufweist.

Erfindungsgemäß vorteilhaft liegt die Temperatur des Reaktionsgaseingangsgemischs A beim Silberverfahren über die gesamte Reaktionszone A in den vorgenannten Temperaturbereichen.

Mit Vorteil wird beim Silberverfahren auch das Reaktionsgaseingangsgemisch A der Reaktionszone A bereits mit einer im vorgenannten Bereich liegenden Temperatur zugeführt. Häufig befindet sich beim Silberverfahren am Eingang in die Reaktionszone A in Strömungsrichtung vorab der eigentlichen katalytisch aktiven Katalysatorbeschickung (die auch mit inerten Formkörpern verdünnt sein kann) eine Beschickung der Reaktionszone A mit festem inertem Material oder von mit derartigem inertem Material hoch verdünnter katalytisch aktiver Katalysatorbeschickung. Beim Durchströmen einer solchen Vorabbeschickung der Reaktionszone A kann die Temperatur des der Reaktionszone A beim Silberverfahren zugeführten Reaktionsgaseingangsgemischs A vergleichsweise einfach auf den Wert eingestellt werden, mit dem das Reaktionsgasgemisch A beim Silberverfahren in die eigentliche katalytisch aktive Katalysatorbeschickung der Reaktionszone A eintreten soll.

Wird die Temperatur des Reaktionsgasgemischs A beim Silberverfahren innerhalb der Reaktionszone A auf Werte von 450 bis 650°C, vorzugsweise 500 bis 600°C begrenzt, wird der Umsatz an Methanol in der Regel ≤ 90 mol-%, häufig ≤ 85 mol-% oder ≤ 80 mol-% betragen, während die Selektivität der Formaldehydbildung bei Werten ≥ 90 mol-%, vielfach ≥ 93 mol-% oder ≥ 95 mol-% liegt. In diesem Fall (bei dem der Wasserdampfgehalt des Reaktionsgaseingangsgemischs vorzugsweise < 10 Vol.-% beträgt) ist es erfindungsgemäß zweckmäßig, vom Produktgasgemisch A vorab seiner Verwendung zur Erzeugung des Reaktionsgaseingangsgemischs B wenigstens eine Teilmenge an nicht umgesetztem Methanol abzutrennen und in die Erzeugung des Reaktionsgaseingangsgemischs A rückzuführen.

Erfindungsgemäß vorteilhaft wird die Temperatur des Reaktionsgasgemischs A beim Silberverfahren innerhalb der Reaktionszone A daher 550 bis 800°C, vorzugsweise 600 bis 750°C und besonders bevorzugt 650 bis 750°C betragen.

Gleichzeitig wird der Wasserdampfgehalt des Reaktionsgaseingangsgemischs A beim Silberverfahren vorteilhaft auf Werte ≥ 10 Vol.-%, vorzugsweise ≥ 15 Vol.-% und besonders vorteilhaft ≥ 20 Vol.-% eingestellt. Sowohl die erhöhte Temperatur als auch der erhöhte Wasserdampfgehalt des Reaktionsgaseingangsgemischs A wirken sich beim Silberverfahren vorteilhaft auf den Methanolumsatz (bezogen auf einen Einmaldurchgang des Reaktionsgasgemischs A durch die Reaktionszone A) aus. In der Regel wird dieser Umsatz > 90 mol-%, vielfach ≥ 92 mol-%, oder ≥ 95 mol-% und häufig sogar ≥ 97 mol-% (vgl. z.B. Ullmann's Encyclopedia of Industrial Chemistry, Vol. A 11, 5th Ed., VCH Weinheim auf Seite 625 ff) betragen (die trotz der vergleichsweise geringen Verhältnisse n_{O} : n_{Me} im Reaktionsgaseingangsgemisch A beim Silberverfahren zu erreichenden hohen Methanolumsätze sind vor allem darauf zurückzuführen, dass mit zunehmender Temperatur des Reaktionsgasgemischs A in der Reaktionszone A die exotherme Partialoxidation CH₃OH+0,5 O₂ → HCHO+H₂O zunehmend von der endothermen Dehydrierung CH₃OH ⇄ HCHO+H₂ begleitet wird). Auf diese Weise können beim Silberverfahren regelmäßig auf einen Einmaldurchgang des Reaktionsgasgemischs A durch die Reaktionszone A sowie die dabei umgesetzte molare Menge an Methanol bezogene Ausbeuten an Formaldehyd von ≥ 85 mol-%, meist ≥ 87 mol-% und vielfach ≥ 89 mol-% erzielt werden. Im Übrigen kann man das Silberverfahren wie in den diesbezüglich bereits erwähnten Schriften des Standes der Technik oder wie in den Schriften US-A 4080383, US-A 3994977, US-A 3987107, US-A 4584412 und US-A 4343954 beschrieben durchführen. Selbstverständlich kann beim beschriebenen Silberverfahren nicht nur vergleichsweise reines Methanol als Rohstoff (Quelle) eingesetzt werden. Erfindungsgemäß diesbezüglich geeignete Methanol-Rohstoffe sind auch wässrige Methanollösungen und technisches Methanol, die nach entsprechendem Verdampfen zur Erzeugung des Reaktionsgaseingangsgemischs A verwendet werden können.

Zur Ausführung des Silberverfahrens eignen sich in der Reaktionszone A neben den im vorgenannten Stand der Technik empfohlenen Reaktoren unter anderem auch Wärmeaustauscherreaktoren.

Ein Wärmeaustauscherreaktor weist wenigstens einen Primärraum und wenigstens einen Sekundärraum auf, die beide durch eine Trennwand voneinander getrennt sind. In dem wenigstens einen Primärraum wird die Katalysatorbeschickung platziert, die wenigstens einen Oxidationskatalysator A umfasst und vom Reaktionsgasgemisch A durchströmt wird. Gleichzeitig wird der Sekundärraum von einem fluiden Wärmeträger durchströmt und durch die Trennwand hindurch findet zwischen den beiden Räumen der Wärmeaustausch statt, der den Zweck verfolgt, die Temperatur des Reaktionsgasgemischs A auf dessen Weg durch das Katalysatorbett zu kontrollieren und zu steuern (die Reaktionszone A zu temperieren).

Beispielhaft seien als erfindungsgemäß geeignete Wärmeaustauscherreaktoren für die Realisierung der Reaktionszone A der Rohrbündelreaktor (wie er z.B. in der EP-A 700714 und dem in jener Schrift zitierten Stand der Technik offenbart ist) und der Thermoplattenreaktor (wie er z.B. in den Schriften EP-A 1651344, DE-A 10361456, DE-A 102004017150 und dem in diesen Schriften gewürdigten Stand der Technik offenbart ist) genannt. Im Fall des Rohrbündelreaktors befindet sich das vom Reaktionsgasgemisch A durchströmte Katalysatorbett vorzugsweise in dessen Rohren (den Primärräumen) und durch den die Reaktionsrohre umgebenden Raum (den Sekundärraum) wird wenigstens ein Wärmeträger geführt. Als Wärmeträger kommen für die Wärmeaustauscherreaktoren beispielsweise Salzschmelzen, Wärmeträgeröle, ionische Flüssigkeiten und Wasserdampf in Betracht. Im Allgemeinen enthalten großtechnisch eingesetzte Rohrbündelreaktoren wenigstens dreitausend bis zu mehreren zehntausend parallel geschalteter Reaktionsrohre (Reaktorrohre). Selbstverständlich kann die Ausführung der Reaktionszone A aber auch in einem Wirbelbettreaktor oder in einem Mikroreaktor verwirklicht werden.

Herkömmliche Reaktoren und Mikroreaktoren unterscheiden sich durch ihre charakteristische Dimension und insbesondere durch die charakteristische Dimension des das Katalysatorbett, das vom Reaktionsgasgemisch durchströmt wird, beherbergenden Reaktionsraums.

Die Belastung des mit Silberkristallen beschickten Reaktors mit im Reaktionsgaseingangsgemisch A enthaltenem Methanol wird in der Regel (0,5 bis 6)·10³ kg Methanol pro m² des Reaktorquerschnitts bzw. des Querschnitts des Katalysatorfestbetts betragen.

Erfindungsgemäß bevorzugt wird die heterogen katalysierte partielle Gasphasenoxidation von Methanol zu Formaldehyd in der Reaktionszone A jedoch nach dem FORMOX-Verfahren durchgeführt.

Im Unterschied zum Silberverfahren wird das FORMOX-Verfahren an Oxidationskatalysatoren A durchgeführt, deren Aktivmasse ein Mischoxid ist, das wenigstens ein Übergangsmetall im oxidierten Zustand aufweist (vgl. z.B. WO 03/053556 und die EP-A 2213370). Der Begriff "Übergangsmetalle" meint dabei die chemischen Elemente des Periodensystems mit den Ordnungszahlen von 21 bis 30, von 39 bis 48 und von 57 bis 80.

Erfindungsgemäß bevorzugt enthalten vorgenannte Mischoxidaktivmassen wenigstens eines der Übergangsmetalle Mo und V in oxidiertem Zustand. Erfindungsgemäß ganz besonders bevorzugt handelt es sich bei den vorgenannten Aktivmassen um wenigstens die Elemente Fe und Mo im oxidierten Zustand aufweisende Mischoxide (vgl. z.B. US-A 3983073, US-A 3978136, US-A 3975302, US-A 3846341, US-A 3716497, US-A 4829042, EP-A 2213370 sowie WO 2005/063375, US 3408309, US-A 3198753, US-A 3152997, WO 2009/1489809, DE-A 2145851, WO 2010/034480, WO 2007/059974 und "Methanol Selective Oxidation to Formaldehyde over Iron-Molybdate Catalysts, Ana Paula Vieira Soares and Manuel Farinha Portela and Alain Kiennemann in Catalysis Review 47, pages 125 to 174 (2004)" und den in diesen Schriften zitierten Stand der Technik).

Ein weiterer Unterschied zwischen dem Silber- und dem FORMOX-Verfahren besteht darin, dass das Verhältnis der im Reaktionsgaseingangsgemisch A enthaltenen molaren Menge an molekularem Sauerstoff (n_{O}) zur im Reaktionsgaseingangsgemisch A enthaltenen molaren Menge an Methanol (n_{Me}), n_{O}: n_{Me}, normalerweise wenigstens 1 oder größer als 1 (≥ 1), vorzugsweise ≥ 1,1 ist. In der Regel wird das Verhältnis no: n_{Me} im Reaktionsgaseingangsgemisch A beim FORMOX-Verfahren jedoch nicht mehr als 5, häufig nicht mehr als 4 betragen. Erfindungsgemäß vorteilhafte Verhältnisse n_{O} : n_{Me} betragen im Reaktionsgaseingangsgemisch A 1,5 bis 3,5, vorzugsweise 2 bis 3. Ein Sauerstoffüberschuß ist erfindungsgemäß insofern vorteilhaft, als er bei der erfindungsgemäßen Verfahrensweise über das Produktgasgemisch A in das Reaktionsgaseingangsgemisch B, und damit in die Reaktionszone B, hineingetragen wird, was sich auf die Standzeit des Aldolkondensationskatalysators B vorteilhaft auswirkt. Darüber hinaus wird der Gehalt des Reaktionsgaseingangsgemischs A an Methanol beim FORMOX-Verfahren üblicherweise nicht mehr als 15 Vol.-%, meist nicht mehr als 11 Vol.-% betragen. Dies ist darauf zurückzuführen, dass Gasgemische aus molekularem Stickstoff, molekularem Sauerstoff und Methanol mit einem Gehalt an molekularem Sauerstoff von nicht mehr als ca. 11 Vol-% molekularem Sauerstoff außerhalb des Explosionsbereichs liegen. Normalerweise wird der Methanolgehalt des Reaktionsgaseingangsgemischs A beim FORMOX-Verfahren ≥ 2 Vol.-%, vorzugsweise 4 bis 10 Vol.-% und besonders bevorzugt 6 bis 9 Vol.-% oder 5 bis 7 Vol.-% betragen. Gasgemische aus molekularem Stickstoff, molekularem Sauerstoff und Methanol, deren Methanolgehalt ≤ 6,7 Vol.-% beträgt, liegen unabhängig von ihrem Gehalt an molekularem Sauerstoff außerhalb des Explosionsbereichs, weshalb in diesem Konzentrationsbereich besonders hohe Verhältnisse n_{O} : n_{Me} im Reaktionsgaseingangsgemisch A angewendet werden können.

Das FORMOX-Verfahren unterscheidet sich aber auch dadurch vom Silberverfahren, dass die mit diesem Verfahren, bezogen auf einen Einmaldurchgang des Reaktionsgasgemischs A durch die Reaktionszone A, erzielten Methanolumsätze im Wesentlichen unabhängig vom im Reaktionsgaseingangsgemisch A mitverwendeten inerten Verdünnungsgas regelmäßig > 90 mol-%, üblicherweise ≥ 92 mol-%, meist ≥ 95 mol-% und vielfach sogar ≥ 97 mol-% bzw. ≥ 98 mol-%, bzw. ≥ 99 mol-% betragen. Die damit einhergehenden Selektivitäten der Formaldehydbildung betragen regelmäßig ≥ 90 mol-%, meist ≥ 92 mol-% und vielfach ≥ 94 mol% und häufig sogar ≥ 96 mol-%.

Als inerte Verdünnungsgase im Reaktionsgaseingangsgemisch A kommen erfindungsgemäß für das FORMOX-Verfahren (sowie für das Silberverfahren) in der Reaktionszone A ebenfalls Gase wie H₂O, N₂, CO₂ und Edelgase wie Ar sowie Gemische aus vorgenannten Gasen in Betracht. Bevorzugtes von Wasserdampf verschiedenes inertes Verdünnungsgas ist auch beim FORMOX-Verfahren im Reaktionsgaseingangsgemisch A molekularer Stickstoff.

Der Gehalt des Reaktionsgaseingangsgemischs A an inertem Verdünnungsgas kann beim FORMOX-Verfahren 70 bis 95 Vol.-%, häufig 70 bis 90 Vol.-% und vorteilhaft 70 bis 85 Vol.-% betragen. D.h., der Gehalt des Reaktionsgaseingangsgemischs A an molekularem Stickstoff kann bei Anwendung des FORMOX-Verfahrens im Reaktionsgaseingangsgemisch A 70 bis 95 Vol.-%, oder 70 bis 90 Vol.-%, oder 70 bis 85 Vol.-% betragen. Erfindungsgemäß vorteilhaft kann das Reaktionsgaseingangsgemisch A beim FORMOX-Verfahren frei von Wasserdampf sein. Anwendungstechnisch zweckmäßig wird das Reaktionsgaseingangsgemisch A bei Anwendung eines FORMOX-Verfahrens in der Reaktionszone A aus ähnlichen Gründen wie im Fall des Silberverfahrens einen geringen Wasserdampfgehalt aufweisen. In der Regel beträgt der Wasserdampfgehalt des Reaktionsgaseingangsgemischs A beim FORMOX-Verfahren in der Reaktionszone A ≥ 0,1 Vol.-% und ≤ 20 Vol.-% bzw. ≤ 10 Vol.-%, vorteilhaft ≥ 0,2 Vol.-% und ≤ 7 Vol.-%, vorzugsweise ≥ 0,5 und ≤ 5 Vol.-%.

Ein weiterer Vorteil der Anwendung eines FORMOX-Verfahrens in der Reaktionszone A liegt erfindungsgemäß darin begründet, dass sich die beschriebenen hohen Methanolumsätze bei wesentlich niedrigeren Reaktionstemperaturen im Vergleich zur Anwendung eines Silberverfahrens einstellen.

Die Temperatur des Reaktionsgasgemischs A wird beim FORMOX-Verfahren in der Reaktionszone A normalerweise im Bereich von 250 bis 500°C, vorzugsweise im Bereich von 300 bis 450°C und häufig im Bereich von 270 bis 400°C liegen. Die Begriffsbedeutung der "Temperatur des Reaktionsgasgemischs A" entspricht beim FORMOX-Verfahren dabei derjenigen, die in dieser Schrift bereits beim Silberverfahren gegeben wurde.

Erfindungsgemäß vorteilhaft liegt die Temperatur des Reaktionsgasgemischs A (in dieser Schrift auch als Reaktionstemperatur in der Reaktionszone A bezeichnet) beim FORMOX-Verfahren über die gesamte Reaktionszone A in den vorgenannten Temperaturbereichen. Mit Vorteil wird auch beim FORMOX-Verfahren das Reaktionsgaseingangsgemisch A der Reaktionszone A bereits mit einer im vorgenannten Bereich liegenden Temperatur zugeführt. Häufig befindet sich beim FORMOX-Verfahren am Eingang in die Reaktionszone A in Strömungsrichtung vorab der eigentlichen katalytisch aktiven Katalysatorbeschickung (die auch mit inerten Formkörpern verdünnt sein kann) eine Beschickung der Reaktionszone A mit festem inertem Material oder von mit derartigem inertem Material hochverdünnter katalytisch aktiver Katalysatorbeschickung. Beim Durchströmen einer solchen Vorabbeschickung der Reaktionszone A kann die Temperatur des der Reaktionszone A beim FORMOX-Verfahren zugeführten Reaktionsgaseingangsgemischs A vergleichsweise einfach auf den Wert eingestellt werden, mit dem das Reaktionsgasgemisch A beim FORMOX-Verfahren in die eigentliche katalytisch aktive Katalysatorbeschickung der Reaktionszone A eintreten soll.

Hinsichtlich des Arbeitsdrucks in der Reaktionszone A gilt für das FORMOX-Verfahren das beim Silberverfahren Gesagte in entsprechender Weise.

Für das FORMOX-Verfahren besonders geeignete Mischoxidaktivmassen sind solche der allgemeinen Formel I,

[Fe₂(MoO₄)₃]₁ [M¹ₘOₙ]_{q} (I),

in der die Variablen folgende Bedeutung aufweisen:
- M¹ =: Mo und/oder Fe, oder
Mo und/oder Fe sowie bezogen auf die molare Gesamtmenge aus Mo und Fe eine molare Gesamtmenge von bis zu 10 mol-% (z.B. 0,01 bis 10 mol-%, oder 0,1 bis 10 mol-%), vorzugsweise nicht mehr als 5 mol-%,
von einem oder mehr als einem Element aus der Gruppe bestehend aus Ti, Sb, Sn, Ni, Cr, Ce, Al, Ca, Mg, V, Nb, Ag, Mn, Cu, Co, Si, Na, K, Tl, Zr, W, Ir, Ta, As, P und B,
- q =: 0 bis 5, oder 0,5 bis 3, oder 1 bis 2,
- m =: 1 bis 3, und
- n =: 1 bis 6,
mit der Maßgabe, dass der Inhalt beider eckiger Klammern elektrisch neutral ist, d.h., keine elektrische Ladung aufweist.

Erfindungsgemäß vorteilhaft enthalten Mischoxidaktivmassen I weniger als 50 mol-%, besonders bevorzugt weniger als 20 mol-% und besonders bevorzugt weniger als 10 mol-% des in der Mischoxidaktivmasse I enthaltenen Fe in der Oxidationsstufe +2, und die jeweils restliche Menge des in ihnen enthaltenen Fe in der Oxidationsstufe +3. Ganz besonders bevorzugt enthält die Mischoxidaktivmasse I das gesamte in ihr enthaltene Fe in der Oxidationsstufe +3.

Das Verhältnis n_{Mo}: n_{Fe} von in einer Mischoxidaktivmasse I enthaltener molarer Menge an Mo (n_{Mo}) zu in derselben Mischoxidaktivmasse enthaltener molarer Menge an Fe (n_{Fe}) beträgt vorzugsweise 1:1 bis 5:1.

Ferner ist es erfindungsgemäß vorteilhaft wenn M¹ = Mo und m = 1 sowie n = 3 ist. Erfindungsgemäß vorteilhafte Mischoxidaktivmassen sind auch dann gegeben, wenn M¹ = Fe und m = 2 sowie n = 3 ist.

Erfindungsgemäß günstige Mischoxidaktivmassen I sind ferner solche, deren Stöchiometrie so beschaffen ist, dass sie sich formal als ein Gemisch aus MoO₃ und Fe₂O₃ betrachten (darstellen) lassen und der MoO₃-Gehalt des Gemischs 65 bis 95 Gew.-% und der Fe₂O₃-Gehalt des Gemischs 5 bis 35 Gew.-% beträgt.

Die Herstellung von Mischoxidaktivmassen I kann wie in den angeführten Schriften des Standes der Technik beschrieben erfolgen.

In der Regel wird man dabei so vorgehen, dass man aus Quellen der katalytisch aktiven Oxidmasse I ein möglichst inniges, vorzugsweise feinteiliges, der Stöchiometrie der gewünschten Oxidmasse I entsprechend zusammengesetztes, Trockengemisch (eine Vorläufermasse) erzeugt und dieses bei Temperaturen von 300 bis 600°C, vorzugsweise 400 bis 550°C calciniert (thermisch behandelt). Die Calcination kann sowohl unter Inertgas als auch unter einer oxidativen Atmosphäre wie z.B. Luft (oder ein anderes Gemisch aus Inertgas und Sauerstoff) sowie auch unter reduzierender Atmosphäre (z.B. ein Gemisch aus Inertgas und reduzierenden Gasen wie NH₃ und CO) durchgeführt werden. Die Calcinationsdauer wird in der Regel einige Stunden betragen und nimmt üblicherweise mit der Höhe der Calcinationstemperatur ab.

Als Quellen für die elementaren Konstituenten der Mischoxidaktivmassen I kommen insbesondere solche Verbindungen in Betracht, bei denen es sich bereits um Oxide handelt und/oder um solche Verbindungen, die durch Erhitzen, wenigstens in Anwesenheit von Sauerstoff, in Oxide überführbar sind. Das innige Vermischen der Ausgangsverbindungen (Quellen) kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form von wässrigen Suspensionen und/oder Lösungen miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird.

Als Lösungsmittel wird vorzugsweise Wasser eingesetzt. Bevorzugt werden aus den Ausgangsverbindungen wenigstens zwei wässrige Lösungen hergestellt, von denen wenigstens eine eine saure Lösung und wenigstens eine eine ammoniakalische (basische) Lösung ist.

Beim Zusammengeben der wässrigen Lösungen kommt es in der Regel zu Fällungsreaktionen, bei denen sich Vorläuferverbindungen der Multimetalloxidaktivmasse I ausbilden.

Anschließend wird die erhaltene wässrige Masse getrocknet, wobei der Trocknungsprozess beispielsweise durch Sprühtrocknung erfolgen kann.

Die nach dem Calcinieren der Trockenmasse erhaltene katalytisch aktive Oxidmasse kann in feinteiliger Form als solche, oder mit Hilfe eines flüssigen Bindemittels auf eine äußere Oberfläche eines Trägerformkörpers aufgebracht als Schalenkatalysator zur Beschickung der Reaktionszone A für das FORMOX-Verfahren verwendet werden. Die Schalenkatalysatorherstellung kann aber auch so erfolgen, dass mit Hilfe eines flüssigen Bindemittels feinteiliges Vorläuferpulver auf die äußere Oberfläche von Trägerformkörpern aufgebracht wird und die Calcination der Vorläufersubstanz erst nach erfolgter Aufbringung und Trocknung erfolgt.

Die Multimetalloxidaktivmassen I können aber auch in reiner, unverdünnter Form oder mit einem oxidischen, im Wesentlichen inerten, Verdünnungsmaterial verdünnt als sogenannte Vollkatalysatoren in der Reaktionszone A zum Einsatz kommen (dies ist erfindungsgemäß bevorzugt). Als erfindungsgemäß geeignete inerte Verdünnungsmaterialien seien z.B. feinteiliges Aluminiumoxid, Siliziumdioxid, Alumosilikate, Zirkondioxid, Titandioxid oder Gemische davon genannt. Unverdünnte Vollkatalysatoren sind erfindungsgemäß bevorzugt.

Im Fall von Vollkatalysatorformkörpern erfolgt die Formgebung vorteilhaft mit Vorläuferpulver, das erst nach der Formgebung calciniert wird. Die Formgebung erfolgt üblicherweise unter Zusatz von Formgebungshilfsmittel wie z.B. Graphit (Gleitmittel) oder mineralischen Fasern (Verstärkungshilfsmittel). Geeignete Formgebungsverfahren sind das Tablettieren, das Strangpressen und das Extrudieren. Selbstverständlich kann die Formgebung aber auch z.B. mit einem Gemisch aus Aktivmassenpulver und Vorläuferpulver durchgeführt werden, dem vorab der Formgebung wiederum Formgebungshilfsmittel sowie wahlweise inerte Verdünnungspulver zugesetzt werden. Nach der Formgebung wird wieder calciniert. Grundsätzlich kann die Formgebung zu Vollkatalysatoren auch nur mit bereits vorgefertigtem Aktivmassenpulver sowie wahlweise den genannten Hilfsmitteln durchgeführt werden. Diese Verfahrensweise ist weniger vorteilhaft. Nach der Formgebung wird auch hier in der Regel nochmals calciniert.

Eine günstige Mo-Quelle ist z.B. Ammoniumheptamolybdattetrahydrat (NH₄)₆ (Mo₇O₂₄)·4H₂O. Vorteilhafte Eisenquellen sind z.B. Eisen-III-nitrat [Fe(NO₃)₃], Eisen-(III)-chlorid [FeCl₃] oder Hydrate des Eisen-(III)-nitrats wie z.B. Fe(NO₃)₃·9 H₂O.

Bevorzugte Geometrien der Trägerformkörper für Schalenkatalysatoren der Mischoxidaktivmassen I sind Kugeln und Ringe, deren Längstausdehnung 1 bis 10 mm, häufig 2 bis 8 mm oder 3 bis 6 mm beträgt (unter der Längstausdehnung eines Formkörpers wird in dieser Schrift generell die längste direkte Verbindungslinie zweier auf der Oberfläche des Formkörpers befindlicher Punkte verstanden).

Erfindungsgemäß günstige Ringgeometrien haben hohlzylindrische Trägerformkörper mit einer Länge von 2 bis 10 mm, einem Außendurchmesser von 4 bis 10 mm und einer Wanddicke von 1 bis 4 mm. Bevorzugt haben die hohlzylindrischen Trägerformkörper eine Länge von 3 bis 6 mm, einen Außendurchmesser von 4 bis 8 mm und eine Wanddicke von 1 bis 2 mm. Grundsätzlich können die Trägerformkörper auch unregelmäßig geformt sein.

Als Materialien für die inerten Trägerformkörper eignen sich beispielsweise Quarz, Kieselglas, gesinterte Kieselsäure, Sinter- oder Schmelztonerde, Porzellan, Sinter- oder Schmelzsilikate wie Aluminiumsilicat, Magnesiumsilicat, Zinksilicat, Zirkonsilicat und insbesondere Steatit (z.B. Steatit C220 der Fa. Ceram Tec).

Die inerten Trägerformkörper unterscheiden sich von der katalytischen Aktivmasse (dazu Synonym wird in dieser Schrift ganz allgemein auch "katalytisch aktive Masse" verwendet) normalerweise dadurch, dass sie eine wesentlich geringere spezifische Oberfläche aufweisen. In der Regel beträgt ihre spezifische Oberfläche weniger als 3 m²/g Trägerformkörper. An dieser Stelle sei festgehalten, dass sich alle Angaben in dieser Schrift zu spezifischen Oberflächen auf Bestimmungen nach DIN 66131 (Bestimmungen der spezifischen Oberfläche von Feststoffen durch Gasadsorption (N₂) nach Brunauer-Emmet-Teller (BET)) beziehen.

Die Beschichtung der inerten Trägerformkörper mit dem jeweiligen feinteiligen Pulver wird in der Regel in einem geeigneten drehbaren Behälter ausgeführt, z.B. in einer Dragiertrommel). Das flüssige Bindemittel wird anwendungstechnisch zweckmäßig auf die inerten Trägerformkörper versprüht und die mit dem Bindemittel befeuchtete Oberfläche der in der Dragiertrommel bewegten Trägerformkörper mit dem jeweiligen Pulver bestäubt (vgl. z.B. EP-A 714700). Anschließend wird die Haftflüssigkeit in der Regel wenigstens teilweise aus dem beschichteten Trägerformkörper entfernt (z.B. durch ein Durchleiten von heißem Gas durch die beschichteten Trägerformkörper, wie es die WO 2006/094765 beschreibt). Grundsätzlich können aber auch alle anderen in der EP-A 714700 als Stand der Technik gewürdigten Aufbringungsverfahren zur Herstellung der relevanten Schalenkatalysatoren angewendet werden. Als flüssige Bindemittel kommen z.B. Wasser und wässrige Lösungen (z.B. von Glyzerin in Wasser) in Betracht. Beispielsweise kann die Beschichtung der Trägerformkörper auch dadurch vorgenommen werden, dass man eine Suspension der aufzubringenden pulverförmigen Masse in flüssigem Bindemittel (z.B. Wasser) auf die Oberfläche der inerten Trägerformkörper aufsprüht (in der Regel unter Einwirkung von Wärme und einem trocknenden Schleppgas). Grundsätzlich kann die Beschichtung auch in einer Wirbelschicht- oder Pulverbeschichtungsanlage vorgenommen werden.

Die Dicke der auf die Oberfläche des inerten Trägerformkörpers aufgebrachten Schale aus katalytisch aktiver Oxidmasse liegt im Fall der Mischoxidaktivmassen I anwendungstechnisch zweckmäßig in der Regel bei 10 bis 1000 µm. Bevorzugt beträgt die Schalendicke 10 bis 500 µm, besonders bevorzugt 100 bis 500 µm und ganz besonders bevorzugt 200 bis 300 µm. Über das bereits Gesagte hinaus eignen sich für die erfindungsgemäßen Zwecke in der Reaktionszone A alle in der DE-A 102010028328 und in der DE-A 102010023312 sowie alle in der EP-A 714700 offenbarten Ringgeometrien für mögliche inerte Trägerformkörper von ringförmigen Oxidationsschalenkatalysatoren A.

Bevorzugte Vollkatalysatorformkörper umfassend Mischoxidaktivmassen I sind Vollzylinder, Hohlzylinder und Trilobe. Der äußere Durchmesser von zylindrischen Vollkatalysatoren beträgt anwendungstechnisch zweckmäßig 3 bis 10 mm, bevorzugt 4 bis 8 mm und vor allem 5 bis 7 mm.

Ihre Höhe beträgt vorteilhaft 1 bis 10 mm, bevorzugt 2 bis 6 mm und vor allem 3 bis 5 mm. Im Fall von Hohlzylindern gilt das selbe. Zusätzlich beträgt der Innendurchmesser der von oben nach unten hindurchlaufenden Öffnung vorteilhaft 1 bis 8 mm, bevorzugt 2 bis 6 mm und ganz besonders bevorzugt 2 bis 4 mm. Anwendungstechnisch zweckmäßig beträgt die Wanddicke von Hohlzylindern 1 bis 3 mm.

Die Formgebung kann im Fall von Vollkatalysatorformkörpern (Vollkatalysatoren) z.B. so erfolgen, dass aus der pulverförmigen Aktivmasse oder ihrer uncalcinierten Vorläufermasse (letzteres ist erfindungsgemäß bevorzugt) durch Verdichten (z.B. durch Tablettieren oder Extrudieren oder Strangpressen) zur gewünschten Katalysatorgeometrie unmittelbar Vollkatalysatoren oder Vollkatalysatorvorläufer hergestellt werden, wobei vorab der Formgebung wahlweise Hilfsmittel wie z.B. Graphit oder Stearinsäure als Gleitmittel und/oder Formgebungshilfsmittel sowie Verstärkungshilfsmittel wie Mikrofasern aus Glas, Asbest, Siliciumcarbid oder Kaliumtitanat zugesetzt werden können. Im Fall von ringförmigen Geometrien kann die Tablettierung vorteilhaft wie in den Schriften WO 2008/152079, WO 2008/087116, DE-A 102008040094, DE-A 102008040093 und WO 2010/000720 beschrieben vorgenommen werden. Auch sind alle in den vorgenannten Schriften aufgeführten Geometrien für erfindungsgemäße Vollkatalysator-Oxidationskatalysatoren A geeignet.

Mischoxidaktivmassen-I-Oxidationskatalysatoren A können in der Reaktionszone A aber auch als Trägerkatalysatoren zur Anwendung kommen. Im Unterschied zu Trägerformkörpern für die Schalenoxidationskatalysatoren A, die vorzugsweise nicht porös bzw. arm an Poren sind, wird bei Trägerkatalysatoren A die Aktivmasse in das Porengefüge der Trägerformkörper eingebracht. In diesem Fall wird deshalb von vergleichsweise porösen Trägerformkörpern ausgegangen, die z.B. nacheinander mit den wenigstens zwei Lösungen der Vorläuferverbindungen getränkt werden. Die beschriebene Fällungsreaktion erfolgt in den Poren des Trägerformkörpers und die sich dabei in diesen ausbildenden Vorläuferverbindungen können nachfolgend durch Calcination in die gewünschte Mischoxidaktivmasse-I überführt werden. Alternativ kann auch mit einer alle erforderlichen Quellen gelöst enthaltenden Lösung getränkt, getrocknet und nachfolgend calciniert werden (vgl. z.B. DE-A 2442311). Im Übrigen kann zur Herstellung von Mischoxidaktivmasse-I-Oxidationskatalysatoren wie in den Schriften des Standes der Technik, auf die in dieser Anmeldung diesbezüglich Bezug genommen wird, vorgegangen werden.

Dies sind insbesondere die Schriften US-A 3716497, die US-A 3846341, die EP-A 199359, die DE-A 2145851, die US-A 3983073, die DE-A 2533209, die EP-A 2213370 und Catalysis Review, 47, pages 125-174 (2004).

Selbstverständlich kann auch beim FORMOX-Verfahren nicht nur vergleichsweise reines Methanol zur Erzeugung des Reaktionsgaseingangsgemischs A eingesetzt werden. Erfindungsgemäß diesbezüglich geeignete Methanol-Rohstoffe sind auch wässrige Methanollösungen sowie technisches Methanol, die nach entsprechendem Verdampfen zur Erzeugung des Reaktionsgaseingangsgemischs A verwendet werden können.

Auch kann die Reaktionszone A mit einem Katalysatorfestbett beschickt werden, das FORMOX-Oxidationskatalysatoren A in mit inerten Formkörpern verdünnter Form aufweist.

Die Belastung des in der Reaktionszone A befindlichen Katalysatorfestbetts mit Reaktionsgaseingangsgemisch A wird bei einem erfindungsgemäß angewandten FORMOX-Verfahren in der Regel 3500 Nl/I·h bis 75000 Nl/I·h, vorzugsweise 25000 Nl/I·h bis 35000 Nl/I·h betragen. Der Begriff der Belastung wird dabei so wie in der DE-A 19927624 definiert verwendet.

Zur Ausführung des FORMOX-Verfahrens eignen sich in der Reaktionszone A insbesondere auch die Wärmeaustauscherreaktoren, die bereits zur Verwirklichung der Reaktionszone A beim Silberverfahren empfohlen wurden (vgl. z.B. WO 2005/063375).

Das FORMOX-Verfahren ist in der Raktionszone A erfindungsgemäß auch deshalb bevorzugt, weil sein Produktgasgemisch A im Unterschied zu einem Produktgasgemisch A nach dem Silberverfahren frei von molekularem Wasserstoff ist.

D.h., das Produktgasgemisch A einer heterogen katalysierten partiellen Gasphasenoxidation von Methanol zu Formaldehyd nach dem FORMOX-Verfahren ist (ohne es zuvor einem Abtrennverfahren zu unterwerfen, ohne zuvor ein Abtrennverfahren an ihm durchzuführen) die ideale Formaldehydquelle für im Reaktionsgaseingangsgemisch B benötigtes Formaldehyd.

Häufig fällt das Produktgasgemisch A beim FORMOX-Verfahren mit einer Temperatur an, mit der es ohne weitere thermische Vorabbehandlung zur Erzeugung des Reaktionsgaseingangsgemischs B eingesetzt werden kann. Vielfach ist die Temperatur des die Reaktionszone A verlassenden Produktgasgemischs A sowohl beim Silberverfahren als auch beim FORMOX-Verfahren aber von derjenigen Temperatur verschieden, mit der es zur Erzeugung des Reaktionsgaseingangsgemischs B eingesetzt werden soll. Vor diesem Hintergrund kann der Strom des Produktgasgemischs A auf seinem Weg aus der Reaktionszone A in die Reaktionszone B einen indirekten Wärmeaustauscher durchströmen, um seine Temperatur an die für die zur Erzeugung des Reaktionsgaseingangsgemischs B vorgesehene Zumischtemperatur anzupassen.

Der Vollständigkeit halber sei noch ergänzt, dass auch bei einer Anwendung des FORMOX-Verfahrens in der Reaktionszone A der beim erfindungsgemäßen Verfahren in der Trennzone T erzeugte Stoffstrom Z eine geeignete Inertgasquelle für das im Reaktionsgaseingangsgemisch A benötigte Inertgas bildet und anwendungstechnisch zweckmäßig ein Teilstrom des Stoffstroms Z zur Erzeugung des Reaktionsgaseingangsgemischs A in die Reaktionszone A rückgeführt wird.

Als Quelle für die im Reaktionsgaseingangsgemisch B benötigte Essigsäure kommt für das erfindungsgemäße Verfahren insbesondere die Carbonylierung von Methanol in der Flüssigphase in Betracht:

CH₃OH+CO → CH₃ COOH.

Die Umsetzung wird mit einem Katalysator durchgeführt (homogene Katalyse). In typischer Weise umfasst der Katalysator wenigstens eines der Elemente Fe, Co, Ni, Ru, Rh, Pd, Cu, Os, Ir und Pt, ein ionisches Halogenid (z.B. KI) und/oder ein kovalentes Halogenid (z.B. CH₃I) als Promotor (wobei die Jodide normalerweise die bevorzugten Promotoren bilden) sowie gegebenenfalls einem Liganden wie beispielsweise PR₃ oder NR₃, wobei R ein organischer Rest ist. Entsprechende Carbonylierungsverfahren sind z.B. in den Schriften EP-A 1506151, DE 3889233 T2, EP-A 277824, EP-A 656811, DE-A 1941449, US-Patent 6420304, EP-A 161874, US-A 3769329, EP-A 55618, EP-A 87870, US-A 5001259, US-A 5466874 und US-A 502698 sowie dem in diesen Schriften zitierten Stand der Technik offenbart. Die Arbeitsbedingungen erfordern hohe Drucke (wenigstens 3 Mpa (abs.)) und erhöhte Temperaturen (wenigstens 150°C bzw. 250°C). Das derzeit in großtechnischen Verfahren bevorzugt angewandte Katalysatorsystem ist Rh in Kombination mit HJ/CH₃J als Promotorsystem (vgl. DE 68916718 T2 und US 3769329). Die dabei erzielten Selektivitäten der Essigsäurebildung, bezogen auf umgesetztes Methanol, betragen ≥ 99 mol-% (Industrielle Organische Chemie, Klaus Weissermel und Hans-Jürgen Arpe, Wiley-VCH, 5. Auflage, 1998, Seite 196 und Ullmann's Encyclopedia of Industrial Chemistry, Sixth Edition, Volume 6 (2003), Seite 467 bis 491).

Da die Flüssgphasen-Carbonylierung von Methanol, wie vorstehend beschrieben, die Mitverwendung von Halogenid-Promotoren erfordert, die stark korrodierend wirken und die Verwendung von teuren korrosionsbeständigen Baumaterialien erfordern, wird die gebildete Essigsäure zur Verwendung im erfindungsgemäßen Verfahren vom bei der Carbonylierung des Methanols anfallenden Produktgemisch rektifikativ abgetrennt. Üblicherweise erfolgt dies in einer Reinheit von wenigstens 99,8 Gew.-% Essigsäuregehalt (vgl. Industrielle Organische Chemie, Klaus Weissermel und Hans-Jürgen Arpe, Wiley-VCH, 5. Auflage, 1998, Seite 196).

Durch Überführen von solcher rektifikativ abgetrennter Essigsäure in die Gasphase (Dampfphase) und Vereinigen mit Produktgasgemisch A oder Produktgasgemisch A* kann erfindungsgemäß vergleichsweise einfach das für die Reaktionszone B erforderliche Reaktionsgaseingangsgemisch B erzeugt werden.

Grundsätzlich kann die Carbonylierung von Methanol zu Essigsäure in der Flüssigphase auch unter Ausschluß von Halogenid enthaltenden Promotoren durchgeführt werden (vgl. z.B. DE-A 3606169). In diesem Fall muss die im Rohprodukt der Carbonylierung des Methanols enthaltene Essigsäure nicht in notwendiger Weise von selbigem rektifikativ abgetrennt werden, um zur Erzeugung des Reaktionsgaseingangsgemischs B herangezogen werden zu können. Vielmehr kann in diesem Fall das Rohprodukt auch als solches in die Dampfphase überführt und zur Erzeugung des Reaktionsgaseingangsgemischs B verwendet werden.

Erfindungsgemäß besonders geschickt wird man die Carbonylierung des Methanols mit Kohlenmonoxid jedoch in der Gasphase durchführen, und unmittelbar das dabei resultierende, die gebildete Essigsäure enthaltende, Produktgasgemisch zur Erzeugung des Reaktionsgaseingangsgemischs B einsetzen.

Mit besonderem Vorteil wird man dabei heterogen katalysierte Gasphasencarbonylierungsverfahren des Methanols zu Essigsäure anwenden, die kein Beisein von Halogen-haltigen Promotoren erfordern. Entsprechende Gasphasencarbonylierungen von Methanol zu Essigsäure offenbaren die US-A 4612387 und die EP-A 596632. Charakteristisch für diese Verfahren ist, dass als Katalysatoren Zeolithe (aluminosilicates) mit anionischer Gerüstladung zur Anwendung kommen, die vorzugsweise an ihrer inneren und/oder äußeren Oberfläche wenigstens eine Kationensorte aus der Gruppe der Kationen der Elemente Kupfer, Iridium, Nickel, Rhodium und Kobalt aufweisen, um die negative Gerüstladung auszugleichen (zu neutralisieren). Besonders vorteilhaft sind dabei Zeolithe, die eine Mordenitgerüststruktur aufweisen (vgl. Studies in Surface, Science and Catalysis, Vol. 101, 11th International Congress on Catalysis - 40th Anniversary), 1996, Elsevier, Science B.V., Lausanne, Seite 771 bis 779).

Selbstverständlich kann als Essigsäurequelle (als Rohstoff) für das Reaktionsgaseingangsgemisch B auch eine wässrige Essigsäurelösung oder technische Essigsäurelösung verwendet werden, die nach entsprechendem Verdampfen zur Erzeugung des Reaktionsgaseingangsgemischs B eingesetzt werden können.

Aus dem die Reaktionszone A verlassenden Strom an Produktgasgemisch A oder dem die Trennzone T* verlassenden Strom an Produktgasgemisch A* sowie der in die Dampfphase überführten Essigsäurequelle als wenigstens einem weiteren Stoffstrom und dem Stoffstrom Y sowie wahlweise weiteren Stoffströmen wie z.B. zusätzlichem Wasserdampf oder zusätzlichem von Wasserdampf verschiedenem inertem Verdünnungsgas (in dieser Schrift verkürzt auch nur Inertgas genannt) kann das Reaktionsgaseingangsgemisch B erzeugt werden. Bei Bedarf, z.B. wenn das Produktgasgemisch A keinen überschüssigen molekularen Sauerstoff enthält, kann zur Erzeugung des Reaktionsgaseingangsgemischs B auch noch molekularer Sauerstoff oder ein Gemisch aus Inertgas und molekularem Sauerstoff mitverwendet werden, da sich ein geringer (begrenzter) Sauerstoffgehalt im Reaktionsgaseingangsgemisch B auf die Standzeit des Aldolkondensationskatalysators B in der Regel vorteilhaft auswirkt.

Die Temperatur des Reaktionsgasgemischs B wird beim erfindungsgemäßen Verfahren innerhalb der Reaktionszone B normalerweise im Bereich von 260 bis 400°C, vorzugsweise im Bereich von 270 bis 390°C, besonders bevorzugt im Bereich 280 bis 380°C, mit Vorteil im Bereich 300 bis 370°C und mit besonderem Vorteil im Bereich von 300 bis 340°C liegen.

Der Begriff der Temperatur des Reaktionsgasgemischs B (in dieser Schrift auch als Reaktionstemperatur in der Reaktionszone B bezeichnet) meint dabei in erster Linie diejenige Temperatur, die das Reaktionsgasgemisch B ab Erreichen eines Umsatzes des im Reaktionsgaseingangsgemisch B enthaltenen Formaldehyds von wenigstens 5 mol-% bis zum Erreichen des entsprechenden Endumsatzes des Formaldehyds innerhalb der Reaktionszone B aufweist. Erfindungsgemäß vorteilhaft liegt die Temperatur des Reaktionsgasgemischs B über die gesamte Reaktionszone B in den vorgenannten Temperaturbereichen. Mit Vorteil wird das Reaktionsgaseingangsgemisch B der Reaktionszone B bereits mit einer im Bereich von 260 bis 400°C liegenden Temperatur zugeführt. Häufig befindet sich jedoch am Eingang in die Reaktionszone B in Strömungsrichtung vorab der eigentlichen katalytisch aktiven Katalysatorbeschickung der Reaktionszone B eine Beschickung der Reaktionszone B mit festem inertem Material oder von mit derartigem inertem Material hoch verdünnter katalytisch aktiver Katalysatorbeschickung. Beim Durchströmen einer solchen Vorabbeschickung der Reaktionszone B kann die Temperatur des der Reaktionszone B zugeführten Reaktionsgaseingangsgemischs B vergleichsweise einfach auf den Wert eingestellt werden, mit dem das Reaktionsgasgemisch B in die eigentliche katalytisch aktive Katalysatorbeschickung der Reaktionszone B eintreten soll. In der Regel ist die Temperatur des die Reaktionszone A verlassenden Produktgasgemischs A von dieser Temperatur verschieden. Vor diesem Hintergrund kann der Strom des Produktgasgemischs A auf seinem Weg aus der Reaktionszone A in die Reaktionszone B einen indirekten Wärmeaustauscher durchströmen, um seine Temperatur an die für das Reaktionsgaseingangsgemisch B vorgesehene Eintrittstemperatur in die Reaktionszone B anzunähern, oder es auf diese Temperatur zu bringen.

Grundsätzlich kann die Beschickung der Reaktionszone B mit wenigstens einem Aldolkondensationskatalysator B als Wirbelbett ausgeführt sein. Anwendungstechnisch vorteilhaft ist die Beschickung der Reaktionszone B mit Aldolkondensationskatalysator B jedoch als Festbett ausgeführt.

Bezüglich des in der Reaktionszone B herrschenden Arbeitsdrucks gilt das über den in der Reaktionszone A herrschenden Arbeitsdruck bereits Gesagte in entsprechender Weise. In der Regel ist der Arbeitsdruck in der Reaktionszone B aufgrund des beim Durchströmen des Reaktionsgasgemischs A durch die Reaktionszone A eintretenden Druckverlusts geringer als der Arbeitsdruck in der Reaktionszone A. Auch kann die Ausführung der Reaktionszone B in entsprechenden Wärmeaustauscherreaktoren wie die Reaktionszone A erfolgen, wobei die gleichen Vorzugsregeln gültig sind.

Der Gehalt des Reaktionsgaseingangsgemischs B an Formaldehyd wird beim erfindungsgemäßen Verfahren in der Regel 0,5 bis 10 Vol.-%, vorzugsweise 0,5 bis 7 Vol.-% und besonders bevorzugt 1 bis 5 Vol.-% betragen.

Das Verhältnis n_{Hac} : n_{Fd} aus im Reaktionsgaseingangsgemisch B enthaltener molarer Menge an Essigsäure (n_{HAc}) zu in ihm enthaltener molarer Menge an Formaldehyd (n_{Fd}) ist beim erfindungsgemäßen Verfahren größer als 1 und kann bis zu 10 betragen (unter n_{Fd} wird dabei die Summe aus im Reaktionsgaseingangsgemisch B monomer (bevorzugt) und gegebenenfalls oligomer sowie polymer vorliegenden (Formaldehyd neigt zu derartigen Formationsbildungen) Formaldehydeinheiten verstanden, da letzte unter den Reaktionsbedingungen in der Reaktionszone B eine Rückspaltung zu monomerem Formaldehyd erfahren). Erfindungsgemäß vorteilhaft beträgt das Verhältnis n_{HAc} : n_{Fd} im Reaktionsgaseingangsgemisch B 1,1 bis 5 und besonders bevorzugt 1,5 bis 3,5. Häufig wird sich der Essigsäuregehalt des Reaktionsgaseingangsgemischs B im Bereich von 1 oder von 1,5 bis 20 Vol.-%, mit Vorteil im Bereich von 2 bis 15 Vol.-% und besonders vorteilhaft im Bereich von 3 bis 10 Vol.-% bewegen. Der Gehalt des Reaktionsgaseingangsgemischs B an molekularem Sauerstoff bewegt sich beim erfindungsgemäßen Verfahren anwendungstechnisch zweckmäßig im Bereich von 0,5 bis 5 Vol.-%, vorzugsweise im Bereich von 1 bis 5 Vol.-% und besonders bevorzugt im Bereich von 2 oder von 3 bis 5 Vol.-%. Ein Beisein von molekularem Sauerstoff im Reaktionsgaseingangsgemisch B wirkt sich vorteilhaft auf die Standzeit der Katalysatorbeschickung der Reaktionszone B aus. Ist der Sauerstoffgehalt des Reaktionsgasgemischs B jedoch zu hoch, kommt es in der Reaktionszone B zu unerwünschter Kohlenoxidbildung. Grundsätzlich kann der Gehalt des Reaktionsgaseingangsgemischs B an molekularem Sauerstoff beim erfindungsgemäßen Verfahren aber auch verschwindend sein.

Der Wasserdampfgehalt des Reaktionsgaseingangsgemischs B sollte beim erfindungsgemäßen Verfahren 30 Vol.-% nicht überschreiten, da ein Beisein von Wasserdampf im Reaktionsgasgemisch B sich auf die Gleichgewichtslage der Aldolkondensation ungünstig auswirkt. Anwendungstechnisch zweckmäßig wird der Wasserdampfgehalt des Reaktionsgaseingangsgemischs B daher 25 Vol.-% und vorzugsweise 20 Vol.-% in der Regel nicht überschreiten. In der Regel wird der Wasserdampfgehalt des Reaktionsgaseingangsgemischs B wenigstens 0,5 oder wenigstens 1 Vol.-% betragen. Mit Vorteil beträgt der Wasserdampfgehalt des Reaktionsgaseingangsgemischs B 0,5 bis 15 Vol.-% und unter Berücksichtigung seiner Wirkung und Bildung (Entstehung) in der Reaktionszone A vor allem 1 bis 10 Vol.-%. Der Volumenanteil an von Wasserdampf verschiedenen inerten Verdünnungsgasen im Reaktionsgaseingangsgemisch B wird normalerweise wenigstens 30 Vol.-% betragen. Bevorzugt beträgt der vorgenannte Inertgasanteil wenigstens 40 Vol.-% bzw. wenigstens 50 Vol.-%. In der Regel wird der Anteil an von Wasserdampf verschiedenem inertem Verdünnungsgas im Reaktionsgaseingangsgemisch B 95 Vol.-% bzw. meist 90 Vol.-% nicht überschreiten. Anwendungstechnisch besonders vorteilhaft enthält das Reaktionsgaseingangsgemisch B 60 bis 90 Vol.-%, besonders vorteilhaft 70 bis 80 Vol.-% an von Wasserdampf verschiedenem inertem Verdünnungsgas. Erfindungsgemäß bevorzugtes von Wasserdampf verschiedenes inertes Verdünnungsgas ist auch im Reaktionsgaseingangsgemisch B molekularer Stickstoff (N₂).

Damit kann der Gehalt des Reaktionsgaseingangsgemischs B an molekularem Stickstoff wenigstens 30 Vol.-%, bevorzugt wenigstens 40 Vol.-% oder wenigstens 50 Vol.-% betragen. In der Regel enthält das Reaktionsgaseingangsgemisch B nicht mehr als 95 Vol.-% und meist nicht mehr als 90 Vol.-% an molekularem Stickstoff. Vorteilhaft enthält das Reaktionsgaseingangsgemisch B 60 bis 90 Vol.-%, besonders vorteilhaft 70 bis 80 Vol.-% an molekularem Stickstoff.

Als Katalysatoren kommen zur Beschickung der Reaktionszone B z.B. diejenigen in Betracht, die in I & EC PRODUCT RESEARCH AND DEVELOPMENT, Vol. 5, No. 1, March 1966, Seite 50 bis 53 offenbart sind. Diese Gruppe an basischen Katalysatoren umfasst zum einen Zeolithe (aluminosilicates) mit anionischer Gerüstladung, an deren innerer und äußerer Oberfläche sich wenigstens eine Kationensorte aus der Gruppe der Alkali- und Erdalkaliionen befindet (vorzugsweise Na⁺, K⁺, Ca²⁺ und/oder Mg²⁺), um die negative Gerüstladung auszugleichen (zu neutralisieren). Sie umfasst aber auch auf inerte Träger (z.B. amorphes Siliziumdioxid (Silicagel)) aufgebrachtes Hydroxid aus der Gruppe bestehend aus den Alkalihydroxiden, Erdalkalihydroxiden und Aluminiumhydroxid (vorzugsweise KOH, NaOH, Ca(OH)₂ und Mg(OH)₂).

Zur Beschickung der Reaktionszone B eignen sich aber auch die sauren Katalysatoren, die in der EP-A 164614 offenbart werden.

Dabei handelt es sich um Katalysatoren, die
- als Bestandteil a) wenigstens ein Oxid von wenigstens einem der Elemente Si, Al, Ti, Zr, Cd, Sn, Ga, Y und La und/oder Zeolith,
   und
- als Bestandteil b) wenigstens ein Oxid ausgewählt aus Boroxid und Phosphoroxid,
   sowie wahlweise
- als Bestandteil c) ein oder mehr als ein Oxid von wenigstens einem der Elemente V, Cr, Co, Ni, Mo und Pb und/oder eine oder mehr als eine Heteropolysäure mit wenigstens einem Polyatom ausgewählt aus V, Mo und W
   umfassen. Als Boroxid wird dabei B₂O₃ und als Phosphoroxid P₂O₅ bevorzugt.

Bevorzugt werden dabei Katalysatoren, deren Boroxidgehalt (gerechnet als B₂O₃ (bezogen auf die enthaltene Menge an B)) 1 bis 50 Gew.-% beträgt. Erfindungsgemäß günstige Katalysatoren sind aber auch solche, deren Phosphoroxidgehalt (gerechnet als P₂O₅ (bezogen auf die enthaltene Menge an P)) 1 bis 50 Gew.-% beträgt. Für das erfindungsgemäße Verfahren kommen als Aldolkondensationskatalysatoren B aber auch solche der vorgenannten Katalysatoren in Betracht, deren Gesamtgehalt an Phosphoroxid (gerechnet als P₂O₅) und an Boroxid (gerechnet als B₂O₃) 1 bis 50 Gew.-% beträgt. Vorzugsweise betragen vorgenannte Gehalte an Phosphoroxid und/oder Boroxid 5 bis 30 Gew.-%.

Des Weiteren handelt es sich beim Bestandteil a) bevorzugt um wenigstens ein Oxid von wenigstens einem der Elemente Si, Al, Ti und Zr.

Erfindungsgemäß besonders günstig sind die Kombinationen Titanoxid als Bestandteil a) sowie Bor- und Phosphoroxid als Bestandteil b), bzw. Siliziumdioxid-Aluminiumoxid als Bestandteil a) sowie Boroxid als Bestandteil b), bzw. Aluminiumoxid als Bestandteil a) sowie Boroxid oder Phosphoroxid als Bestandteil b). Umfassen die vorstehend ausgeführten Katalysatoren zusätzlich eine Heteropolysäure, so enthält diese bevorzugt wenigstens eines der Elemente P, B und Si als Heteroatom. Umfassen die vorgenannten Katalysatoren einen Bestandteil c), so beläuft sich dessen Menge normalerweise auf 0,01 bis 10 mmol pro Gramm Katalysator und vielfach auf 0,03 bis 5 mmol pro Gramm Katalysator. Dabei ist es günstig, wenn die Katalysatoren als Bestandteil c) sowohl wenigstens eines der Oxide als auch wenigstens eine der Heteropolysäuren aufweisen.

Erfindungsgemäß besonders bevorzugt wird die Reaktionszone B jedoch mit Aldolkondensationskatalysatoren B beschickt, deren Aktivmasse ein Vanadin-Phosphoroxid und/oder ein mit von Vanadin und Phosphor verschiedenen Elementen dotiertes Vanadin-Phosphoroxid ist (zusammengefasst in der Literatur auch als V-P-O-Katalysatoren bezeichnet).

Derartige Katalysatoren sind in der Literatur vorbeschrieben und werden dort insbesondere als Katalysatoren für die heterogen katalysierte partielle Gasphasenoxidation von Kohlenwasserstoffen mit mindestens vier Kohlenstoffatomen (insbesondere n-Butan, n-Butene und/oder Benzol) zu Maleinsäureanhydrid empfohlen.

Überraschenderweise eignen sich diese aus dem Stand der Technik für vorgenannte Partialoxidationen bekannten Katalysatoren grundsätzlich als Aldolkondensationskatalysatoren B zur Beschickung der Reaktionszone B. Sie zeichnen sich durch besonders hohe Selektivitäten der Zielproduktbildung (der Acrylsäurebildung) aus (bei gleichzeitig hohen Formaldehydumsätzen).

Demgemäß können als Aldolkondensationskatalysatoren B beim erfindungsgemäßen Verfahren beispielsweise alle diejenigen eingesetzt werden, die in den Schriften US-A 5,275,996, US-A 5,641,722, US-A 5,137,860, US-A 5,095,125, DE-69702728 T2, WO 2007/012620, WO 2010/072721, WO 2001/68245, US-A 4,933,312, WO 2003/078310, Journal of Catalysis 107, Seite 201-208 (1987), DE-A 102008040094, WO 97/12674, "Neuartige Vanadium (IV)-phosphate für die Partialoxidation von kurzkettigen Kohlenwasserstoffen-Synthesen, Kristallstrukturen, Redox-Verhalten und katalytische Eigenschaften, Dissertation von Dipl.Chem. Ernst Benser, 2007, Rheinische Friedrichs-Wilhelms-Universität Bonn", WO 2010/072723, "Untersuchung von V-P-O-Katalysatoren für die partielle Oxidation von Propan zu Acrylsäure, Dissertation von Dipl.Chem. Thomas Quandt, 1999, Ruhr-Universität.Bochum", WO 2010/000720, WO 2008/152079, WO 2008/087116, DE-A 102008040093, DE-A 102005035978 und DE-A 102007005602 sowie in dem in diesen Schriften gewürdigten Stand der Technik offenbart sind. Im besonderen gilt dies für alle beispielhaften Ausführungsformen des vorstehenden Standes der Technik, insbesondere diejenigen der WO 2007/012620.

Das Phosphor/Vanadin-Atomverhältnis in den undotierten oder dotierten Vanadin-Phosphoroxiden beträgt erfindungsgemäß vorteilhaft 0,9 bis 2,0, bevorzugt 0,9 bis 1,5, besonders bevorzugt 0,9 bis 1,2 und ganz besonders bevorzugt 1,0 bis 1,1. Die arithmetisch mittlere Oxidationsstufe des Vanadins beträgt in ihnen vorzugsweise +3,9 bis +4,4 und besonders bevorzugt 4,0 bis 4,3. Ferner besitzen diese Aktivmassen vorteilhaft eine spezifische BET-Oberfläche von ≥15 m²/g, bevorzugt von ≥15 bis 50 m²/g und ganz besonders bevorzugt von ≥ 15 bis 40 m²/g. Sie weisen vorteilhaft ein Porengesamtvolumen von ≥ 0,1 ml/g, bevorzugt von 0,15 bis 0,5 ml/g und ganz besonders bevorzugt von 0,15 bis 0,4 ml/g auf. Angaben zu Porengesamtvolumina beziehen sich in dieser Schrift auf Bestimmungen mit der Methode der Quecksilberporosimetrie in Anwendung des Messgerätes Auto Pore 9220 der Fa. Micromeritics GmbH, DE-4040 Neuss (Bandbreite 30 Å bis 0,3 mm). Wie bereits ausgeführt, können die Vanadin-Phosphoroxid-Aktivmassen mit von Vanadin und Phosphor verschiedenen Promotorelementen dotiert sein. Als derartige Promotorelemente kommen die von P und V verschiedenen Elemente der 1. bis 15. Gruppe des Periodensystems in Betracht. Dotierte Vanadin-Phosphoroxide offenbaren beispielsweise die WO 97/12674, die WO 95/26817, die US-A 5,137,860, die US-A 5,296,436, die US-A 5,158,923, die US-A 4,795,818 und die WO 2007/012620.

Erfindungsgemäß bevorzugte Promotoren sind die Elemente Lithium, Kalium, Natrium, Rubidium, Cäsium, Thallium, Molybdän, Zink, Hafnium, Zirkon, Titan, Chrom, Mangan, Nickel, Kupfer, Eisen, Bor, Silizium, Zinn, Niob, Kobalt und Wismut, unter denen neben Eisen insbesondere Niob, Molybdän, Zink und Wismut bevorzugt sind. Die Vanadin-Phosphoroxid-Aktivmassen können eines oder mehrere Promotorelemente enthalten. Der Gesamtgehalt an Promotoren in der katalytischen Aktivmasse beträgt, auf deren Gewicht bezogen, in der Regel nicht mehr als 5 Gew.-% (das einzelne Promotorelement jeweils als elektrisch neutrales Oxid gerechnet, in welchem das Promotorelement die gleiche Ladungszahl (Oxidationszahl) wie in der Aktivmasse aufweist).

Als Aktivmassen für Aldolkondensationskatalysatoren B zur Beschickung der Reaktionszone B kommen somit insbesondere Multielementoxidaktivmassen der allgemeinen Formel II,

V₁P_{b}Fe_{c}X¹_{d}X²ₑOₙ (II),

in der die Variablen folgende Bedeutung aufweisen:
- X¹ =: Mo, Bi, Co, Ni, Si, Zn, Hf, Zr, Ti, Cr, Mn, Cu, B, Sn und/oder Nb, vorzugsweise Nb, Mo, Zn und/oder Hf,
- X² =: Li, K, Na, Rb, Cs und/oder Tl,
- b =: 0,9 bis 2,0, vorzugsweise 0,9 bis 1,5, besonders bevorzugt 0,9 bis 1,2 und ganz besonders bevorzugt 1,0 bis 1,1,
- c =: ≥ 0 bis 0,1,
- d =: ≥ 0 bis 0,1,
- e =: ≥ 0 bis 0,1, und
- n =: der stöchiometrische Koeffizient des Elementes Sauerstoff, der durch die stöchiometrischen Koeffizienten der von Sauerstoff verschiedenen Elemente sowie deren Ladungszahl in II bestimmt wird,

### in Betracht.

Unabhängig von den stöchiometrischen Koeffizienten d, e und b beträgt der stöchiometrische Koeffizient c erfindungsgemäß vorteilhaft in Aktivmassen der allgemeinen Formel II 0,005 bis 0,1, vorzugsweise 0,005 bis 0,05 und besonders vorteilhaft 0,005 bis 0,02.

Die Aldolkondensationskatalysatoren B können die Multimetalloxidaktivmassen der allgemeinen Formel II beispielsweise in reiner, unverdünnter Form oder mit einem oxidischen, im Wesentlichen inerten, Verdünnungsmaterial verdünnt als sogenannte Vollkatalysatoren enthalten. Als erfindungsgemäß geeignete inerte Verdünnungsmaterialien seien z.B. feinteiliges Aluminiumoxid, Siliziumdioxid, Alumosilikate, Zirkondioxid, Titandioxid oder Gemische davon genannt. Unverdünnte Vollkatalysatoren sind erfindungsgemäß bevorzugt. Die Vollkatalysatoren können grundsätzlich jedwede Form aufweisen. Bevorzugte Vollkatalysatorformkörper sind Kugeln, Vollzylinder, Hohlzylinder und Trilobe, deren Längstausdehnung in allen Fällen vorteilhaft 1 bis 10 mm beträgt.

Im Fall von Vollkatalysatorformkörpern erfolgt die Formgebung vorteilhaft mit Vorläuferpulver, das erst nach der Formgebung calciniert wird. Die Formgebung erfolgt üblicherweise unter Zusatz von Formgebungshilfsmitteln wie z.B. Graphit (Gleitmittel) oder mineralischen Fasern (Verstärkungshilfsmittel). Geeignete Formgebungsverfahren sind die Tablettierung, das Strangpressen und das Extrudieren.

Der äußere Durchmesser von zylindrischen Vollkatalysatoren beträgt anwendungstechnisch zweckmäßig 3 bis 10 mm, bevorzugt 4 bis 8 mm und vor allem 5 bis 7 mm. Ihre Höhe beträgt vorteilhaft 1 bis 10 mm, bevorzugt 2 bis 6 mm und vor allem 3 bis 5 mm. Im Fall von Hohlzylindern gilt dasselbe. Zusätzlich beträgt der Innendurchmesser der von oben nach unten hindurchlaufenden Öffnung vorteilhaft 1 bis 8 mm, bevorzugt 2 bis 6 mm und ganz besonders bevorzugt 2 bis 4 mm. Eine Wanddicke von 1 bis 3 mm ist bei Hohlzylindern anwendungstechnisch zweckmäßig. Selbstverständlich können die dotierten oder undotierten Vanadin-Phosphoroxid-Aktivmassen auch in Pulverform oder als Schalenkatalysatoren mit einer auf die Oberfläche von inerten Trägerformkörpern aufgebrachten Aktivmassenschale als Aldolkondensationskatalysatoren B in der Reaktionszone B zum Einsatz kommen. Die Herstellung der Schalenkatalysatoren, die Schalendicke und die Geometrie der inerten Trägerformkörper kann dabei wie im Fall der Schalenkatalysatoren für die Reaktionszone A beschrieben gewählt werden.

Im Übrigen kann die Herstellung von dotierten oder undotierten Vanadin-Phosphoroxid-Aktivmassen sowie von aus selbigen gefertigten Vollkatalysatoren wie in den Schriften des Standes der Technik beschrieben erfolgen, auf die in dieser Patentanmeldung Bezug genommen wird.

Dies sind insbesondere die Schriften WO 2007/012620, WO 2010/07273, WO 2010/000720 und WO 2010/000764.

Beispielsweise kann wie folgt vorgegangen werden:
a) Umsetzung einer fünfwertigen Vanadinverbindung (z.B. V₂O₅) mit einem organischen, reduzierenden Lösungsmittel (z.B. iso-Butanol) in Gegenwart einer fünfwertigen Phosphorverbindung (z.B. Ortho- und/oder Pyrophosphorsäure) unter Erwärmen auf 75 bis 205°C, bevorzugt auf 100 bis 120°C;
b) Abkühlen des Reaktionsgemisches auf vorteilhaft 40 bis 90°C;
c) Wahlweise Zugabe von Dotierelemente enthaltenden Verbindungen wie z.B. Eisen(III)phosphat;
d) Erneutes Erwärmen auf 75 bis 205°C, bevorzugt 100 bis 120°C;
e) Isolierung der gebildeten festen V, P, O und z.B. Fe enthaltenden Vorläufermasse (z.B. durch Filtrieren);
f) Trocknung und/oder thermische Vorbehandlung der Vorläufermasse (wahlweise bis zur beginnenden Vorformierung durch Wasserabspaltung aus der Vorläufermasse);
g) Zugabe von Formgebungshilfsmittel wie z.B. feinteiligem Graphit bzw. mineralischen Fasern und anschließend Formgebung zum Vollkatalysatorvorläuferformkörper durch z.B. Tablettieren;
h) Daran anschließend thermische Behandlung der gebildeten Katalysatorvorläuferformkörper durch Erhitzen in einer Atmosphäre, welche Sauerstoff, Stickstoff, Edelgase, Kohlendioxid, Kohlenmonoxid und/oder Wasserdampf enthält (z.B. wie in der WO 2003/078310 auf Seite 20, Zeile 16 bis Seite 21, Zeile 35 beschrieben). Die Temperatur der thermischen Behandlung überschreitet in der Regel 250°C, vielfach 300°C oder 350°C, jedoch normalerweise nicht 600°C, vorzugsweise nicht 550°C und ganz besonders bevorzugt nicht 500°C.

Die Belastung der Katalysatorbeschickung der Reaktionszone B mit im Reaktionsgaseingangsgemisch B enthaltenem Formaldehyd kann erfindungsgemäß z.B. 1 bis 100, vorzugsweise 2 bis 50 und besonders bevorzugt 3 bis 30 oder 4 bis 10 Nl/I·h betragen. Der Begriff der Belastung wird dabei so wie in der DE-A 19927624 definiert verwendet. Sowohl in der Reaktionszone A als auch in der Reaktionszone B kann das jeweilige Katalysatorfestbett nur aus Aktivmasse aufweisenden Katalysatoren als auch aus einem Gemisch aus Aktivmasse aufweisenden Katalysatoren und inerten Formkörpern bestehen.

Insbesondere bei Verwendung von V-P-O-Katalysatoren als Aldolkondensationskatalysatoren in der Reaktionszone B, werden beim erfindungsgemäßen Verfahren, bezogen auf einen einmaligen Durchgang des Reaktionsgasgemischs B durch die Reaktionszone B, wenigstens 95 mol-%, meist wenigstens 98 mol-% des im Reaktionsgaseingangsgemisch B enthaltenen Formaldehyds umgesetzt. Die Selektivität der Acrylsäurebildung, bezogen auf umgesetztes Formaldehyd, beträgt dabei in der Regel ≥ 95 mol-%, häufig ≥ 98 mol-%.

Zur Ausführung der Reaktionszone B eignen sich erfindungsgemäß diejenigen Wärmeaustauscherreaktoren, die bereits zur Verwirklichung der Reaktionszone A empfohlen wurden.

Die Auftrennung des die Reaktionszone B verlassenden, die gebildete Acrylsäure, nicht umgesetzte Essigsäure, wenigstens ein von Wasserdampf verschiedenes inertes Verdünnungsgas, Wasserdampf, sowie gegebenenfalls (wahlweise) molekularen Sauerstoff enthaltenden Produktgasgemischs B in einer Trennzone T in die wenigstens drei Stoffströme X, Y und Z, kann in an sich bekannter Weise erfolgen.

Beispielsweise kann die Auftrennung durch fraktionierende Kondensation erfolgen, wie es in den Schriften DE-A 102007004960, DE-A 10 2007055086, DE-A 10243625, DE-A 10235847 und DE-A 19924532 empfohlen wird. Bei dieser Verfahrensweise wird die Temperatur des Produktgasgemischs B gegebenenfalls zunächst durch direkte und/oder indirekte Kühlung verringert und das Produktgasgemisch B anschließend in eine mit trennwirksamen Einbauten (z.B. Stoffaustauschböden) ausgerüstete und wahlweise mit Kühlkreisen versehene Kondensationskolonne geleitet und innerhalb der Kondensationskolonne in sich selbst aufsteigend fraktionierend kondensiert. Durch entsprechende Wahl der Anzahl der theoretischen Trennstufen (theoretischen Böden) in der Kondensationskolonne, können die Stoffströme X, Y und Z als voneinander getrennte Fraktionen mit dem jeweils gewünschten Anreicherungsgrad aus der Kondensationskolonne herausgeführt werden.

Anwendungstechnisch zweckmäßig wird der Stoffstrom X in der Regel mit einem Acrylsäuregehalt von ≥ 90 Gew.-%, vorzugsweise ≥ 95 Gew-% abgetrennt und aus der Kondensationskolonne herausgeführt. Bei erhöhtem Reinheitsbedarf kann der Stoffstrom X anwendungstechnisch vorteilhaft durch Kristallisation (vorzugsweise Suspensionskristallisation) weitergereinigt werden (vgl. die vorgenannten Schriften des Standes der Technik und die WO 01/77056). Selbstverständlich kann der aus der Kondensationskolonne herausgeführte Stoffstrom X auch rektifikativ weitergereinigt werden. Auf beiden Wegen können so mit vergleichsweise geringem Aufwand Acrylsäurereinheiten ≥ 99,9 Gew.-% erreicht werden, die zur Herstellung wasserabsorbierender Harze durch radikalische Polymerisation von Acrylsäure und/oder deren Natriumsalz umfassenden Monomerengemischen geeignet sind.

Die Herstellung der wasserabsorbierenden Harze kann z.B. wie in den Schriften WO 2008/116840, DE-A 102005062929, DE-A 102004057874, DE-A 102004057868, DE-A 102004004496 und DE-A 19854575 beschrieben erfolgen.

In entsprechender Weise wird auch der Stoffstrom Y normalerweise mit einem Essigsäuregehalt ≥ 90 Gew.-%, vorzugsweise ≥ 95 Gew.-% aus der Kondensationskolonne herausgeführt. Der so abgetrennte Stoffstrom Y kann als solcher zur Erzeugung des Reaktionsgaseingangsgemischs B in die Reaktionszone B rückgeführt werden. Selbstverständlich ist es aber auch möglich, vorab der Rückführung des wie beschrieben abgetrennten Stoffstroms Y in die Reaktionszone B dessen Essigsäuregehalt rektifikativ und/oder kristallisativ weiter anzureichern (z.B. auf Essigsäuregehalte ≥ 99 Gew.-%), oder durch eine Erhöhung der Anzahl der theoretischen Trennstufen in der Kondensationskolonne den Stoffstrom Y in selbiger unmittelbar mit einer solchen erhöhten Reinheit abzutrennen. Der Stoffstrom Z verlässt die Kondensationskolonne normalerweise über Kopf.

Alternativ kann auch wie in den Schriften DE-A 102009027401 und DE-A 10336386 empfohlen verfahren werden. Nach gegebenenfalls zuvor erfolgter direkter und/oder indirekter Abkühlung wird das Produktgasgemisch B bei dieser Verfahrensweise in einer vorteilhaft mit trennwirksamen Einbauten ausgestatteten Absorptionskolonne im Gegenstrom zu einem bei Normaldruck (10⁵ Pa) höher als Acrylsäure siedenden organischen Lösungsmittel (als solche kommen beispielsweise die in der DE-A 102009027401 und in der DE-A 10336386 genannten organischen Lösungsmittel in Betracht) geführt und die im Produktgasgemisch B enthaltene Essigsäure und Acrylsäure in das organische Lösungsmittel absorbiert, während ein Stoffstrom Z die Absorptionskolonne an deren Kopf verlässt. Vom die Essigsäure und Acrylsäure enthaltenden Absorbat können Stoffströme X und Y durch entsprechende Wahl der Anzahl der theoretischen Trennstufen (der theoretischen Böden) in an sich bekannter Weise durch Rektifikation (fraktionierende Destillation) in einer Rektifikationskolonne mit dem jeweils gewünschten Anreicherungsgrad abgetrennt werden. In der Regel wird dieser Anreicherungsgrad an Acrylsäure bzw. Essigsäure wenigstens 90 Gew.-%, vorzugsweise wenigstens 95 Gew.-% betragen. Eine nachfolgende kristallisative Weiterreinigung des abgetrennten Stoffstroms X (z.B. wie in der WO 01/77056 offenbart) führt mit vergleichsweise geringem Aufwand zu Acrylsäurereinheiten ≥ 99,9 Gew.-%, die zur Herstellung wasserabsorbierender Harze durch radikalische Polymerisation von Acrylsäure und/oder deren Natriumsalz umfassenden Monomerengemischen geeignet sind. Der wie beschrieben rektifikativ abgetrennte Stoffstrom Y kann als solcher oder nach gegebenenfalls erfolgter kristallisativer und/oder rektifikativer Weiterreinigung (z.B. auf Essigsäuregehalte ≥ 99 Gew.-%) zur Erzeugung des Reaktionsgaseingangsgemischs B in die Reaktionszone B rückgeführt werden. Durch entsprechende Erhöhung der Anzahl der theoretischen Trennstufen kann der Stoffstrom Y auch unmittelbar mit einem solchen Anreicherungsgrad vom Absorbat rektifikativ abgetrennt werden.

Anstelle ein organisches Absorptionsmittel zu verwenden, können der Lehre der EP-A 551111 oder der EP-A 778255 folgend die im Produktgasgemisch B enthaltene Acrylsäure sowie Essigsäure aus selbigem in einer Absorptionskolonne auch in ein wässriges Absorptionsmittel aufgenommen werden, während ein Stoffstrom Z die Absorptionskolonne an deren Kopf verlässt. Nachfolgende rektifikative Auftrennung des wässrigen Absorbats, unter optionalem Einbezug eines azeotropen Schleppmittels, ergibt die gewünschten Stoffströme X und Y.

Die Überführung der im Reaktionsgasgemisch B enthaltenen Essigsäure und Acrylsäure in die kondensierte Phase, unter Verbleib eines gasförmigen Stoffstroms Z, kann auch z.B. durch einstufige Kondensation derjenigen im Reaktionsgasgemisch B enthaltenen Bestandteile, deren Siedepunkt bei Normaldruck nicht oberhalb desjenigen von Essigsäure liegt, erfolgen. Nachfolgend kann das Acrylsäure und Essigsäure enthaltende Kondensat wieder im jeweils erwünschten Anreicherungsgrad in wenigstens einen Stoffstrom Y und wenigstens einen Stoffstrom X aufgetrennt werden.

Anwendungstechnisch zweckmäßig werden beim erfindungsgemäßen Verfahren wenigstens 90 mol-%, vorzugsweise wenigstens 95 mol-%, besonders bevorzugt wenigstens 98 mol-% oder wenigstens 99 mol-% der im Produktgasgemisch B enthaltenen Essigsäure zur Erzeugung des Reaktionsgaseingangsgemischs B in die Reaktionszone B rückgeführt.
Anstelle dass sich an das erfindungsgemäße Verfahren ein Verfahren anschließt, bei dem im Stoffstrom X enthaltene Acrylsäure oder ein Gemisch aus im Stoffstrom X enthaltener Acrylsäure und einem oder mehreren von Acrylsäure verschiedenen, wenigstens einfach ethylenisch ungesättigten Monomeren zu Polymerisaten polymerisiert werden (z.B. radikalisch; die Polymerisation kann z.B. eine Lösungspolymerisation oder eine wässrige Emulsionspolymerisation oder eine Suspensionspolymerisation sein), kann sich an das erfindungsgemäße Verfahren auch ein Verfahren anschließen, bei dem im Stoffstrom X enthaltene Acrylsäure mit wenigstens einem, z.B. 1- bis 8 C-Atome aufweisenden, Alkohol (z.B. ein solches Alkanol wie Methanol, Ethanol, n-Butanol, tert.-Butanol und 2-Ethylhexanol) zum entsprechenden Acrylsäureester (Acrylat) verestert wird. An das Verfahren der Acrylsäureesterherstellung kann sich dann wieder ein Verfahren anschließen, bei dem der hergestellte Acrylsäureester oder ein Gemisch aus dem hergestellten Acrylsäureester und einem oder mehreren von dem hergestellten Acrylsäureester verschiedenen, wenigstens einfach ethylenisch ungesättigten Monomeren zu Polymerisaten polymerisiert werden (z.B. radikalisch; die Polymerisation kann z.B. eine Lösungspolymerisation oder eine wässrige Emulsionspolymerisation oder eine Suspensionspolymerisation sein).

Der guten Ordnung halber sei noch festgehalten, dass einer Deaktivierung der verschiedenen Katalysatoren in den verschiedenen Reaktionszonen des erfindungsgemäßen Verfahrens dadurch entgegenwirken kann, dass man die Reaktionstemperatur in der jeweiligen Reaktionszone entsprechend erhöht (um den auf einen Einmaldurchgang des Reaktionsgasgemischs durch die Katalysatorbeschickung bezogenen Reaktandenumsatz stabil zu halten). Auch können die oxidischen Aktivmassen der Reaktionszonen A und B in entsprechender Weise wie für vergleichbare oxidische Katalysatoren in der WO 2005/042459 beschrieben durch Überleiten eines oxidierend wirkenden Sauerstoff enthaltenden Gases bei erhöhter Temperatur regeneriert werden.

Ein sicherer Betrieb, insbesondere in der Reaktionszone A, kann beim erfindungsgemäßen Verfahren durch eine analoge Anwendung der in der WO 2004/007405 beschriebenen Verfahrensweise gewährleistet werden.

Das erfindungsgemäße Verfahren besticht zum einen durch seine breite und zeitlich weitreichende Rohstoffbasis. Zum anderen handelt es sich um ein Verfahren, das im Unterschied zu den Verfahren des Standes der Technik unter Beibehalt der Verfahrensweise einen gleitenden Übergang von "fossiler Acrylsäure" zu "nachwachsender Acrylsäure" ermöglicht.

Unter "fossiler Acrylsäure" wird dabei Acrylsäure verstanden, für die das Verhältnis aus der in dieser Acrylsäure enthaltenen molaren Menge an ¹⁴C-Atomkernen zu der in derselben Acrylsäure enthaltenen molaren Menge an ¹²C-Atomkernen, n¹⁴C : n¹²C, verschwindend ist.

Unter "nachwachsender Acrylsäure" wird dabei Acrylsäure verstanden, für die das Verhältnis n¹⁴C : n¹²C dem im CO₂ in der Atmosphäre auf der Erde vorliegenden Verhältnis V* von n¹⁴C : n¹²C entspricht, wobei die Bestimmung des Verhältnisses n¹⁴C : n¹²C nach der von Willard Frank Libby entwickelten Verfahrensweise erfolgt
(http://de.wikipedia.orgn/wiki/Radikohlenstoffdatierung).

In entsprechender Weise werden in dieser Schrift die Begriffe "nachwachsender Kohlenstoff" und "fossiler Kohlenstoff" verwendet.

Das von Libby entwickelte Verfahren beruht darauf, dass im Vergleich zu den beiden Kohlenstoffatomkernen ¹²C und ¹³C, der dritte natürlich vorkommende Kohlenstoffkern ¹⁴C nicht stabil ist und deswegen auch Radiokohlenstoff genannt wird (Halbwertszeit = ca. 5700 Jahre).

In den oberen Schichten der Erdatmosphäre wird ¹⁴C durch Kernreaktion ständig neu gebildet. Gleichzeitig zerfällt ¹⁴C mit einer Halbwertszeit von 5700 Jahren durch β-Zerfall. Zwischen ständiger Neubildung und ständigem Zerfall bildet sich in der Atmosphäre auf der Erde ein Gleichgewicht aus, so dass der Anteil der ¹⁴C-Kerne am Kohlenstoff in der Atmosphäre auf der Erde über große Zeiträume konstant ist, in der Atmosphäre auf der Erde ein stabiles Verhältnis V* vorliegt.

Der in der Atmosphäre erzeugte Radiokohlenstoff verbindet sich mit dem Luftsauerstoff zu CO₂, der durch die Photosynthese anschließend in die Biosphäre gelangt. Da Lebewesen (Pflanzen, Tiere, Menschen) bei ihrem Stoffwechsel auf diese Weise ständig Kohlenstoff mit der sie umgebenden Atmosphäre austauschen, stellt sich in lebenden Organismen dasselbe Verteilungsverhältnis der drei Kohlenstoff-Isotope und damit dasselbe Verhältnis n¹⁴C : n¹²C ein, wie es in der sie umgebenden Atmosphäre vorliegt.

Wird dieser Austausch zum Zeitpunkt des Todes des Lebewesens unterbrochen, ändert sich das Verhältnis zwischen ¹⁴C und ¹²C im abgestorbenen Organismus, weil die zerfallenden ¹⁴C-Atomkerne nicht mehr durch neue ersetzt werden (der im abgestorbenen Organismus enthaltene Kohlenstoff wird fossil).

Liegt der Tod des Organismus (Lebewesens) mehr als 50000 Jahre zurück, liegt dessen ¹⁴C-Gehalt unterhalb der Nachweisgrenze. Heutige und zukünftige biologische ("nachwachsende") Rohstoffe und aus diesen hergestellte Chemikalien weisen die jeweils aktuelle ¹⁴C-Konzentration im CO₂ der Atmosphäre auf der Erde auf (dieses n¹⁴C : n¹²C Verhältnis = V*). Fossile Kohlenstoffquellen wie Kohle, Erdöl oder Erdgas lagern jedoch schon mehrere Millionen Jahre "tot" in der Erde, weshalb sie, ebenso wie aus ihnen erzeugte Chemikalien, kein ¹⁴C mehr enthalten.

Wird beim erfindungsgemäßen Verfahren fossile Essigsäure (aus fossilen Rohstoffen erzeugte Essigsäure) und nachwachsendes Formaldehyd (aus aus nachwachsenden Rohstoffen gewonnenem Methanol erzeugtes Formaldehyd) eingesetzt, wird eine Acrylsäure erzeugt, deren Verhältnis n¹⁴C : n¹²C lediglich (1/3) x V* ist.

Wird beim erfindungsgemäßen Verfahren dagegen aus nachwachsenden Rohstoffen erzeugte Essigsäure und aus fossilem Methanol erzeugtes Formaldehyd eingesetzt, wird eine Acrylsäure erzeugt, deren Verhältnis n¹⁴C : n¹²C = (2/3) x V* ist.

Wird beim erfindungsgemäßen Verfahren sowohl fossile (nachwachsende) Essigsäure als auch fossiles (nachwachsendes) Formaldehyd eingesetzt, wird eine Acrylsäure erzeugt, deren Verhältnis n¹⁴C : n¹²C = 0 (= V*) ist.

Bezieht man zusätzlich die Möglichkeit des Abmischens von nachwachsenden und fossilen Ausgangsstoffen (Rohstoffen) beim erfindungsgemäßen Verfahren mit ein, vermag der Hersteller von Acrylsäure bei Anwendung der erfindungsgemäßen Verfahrensweise somit ohne Änderung des Herstellverfahrens (d.h., mit ein- und derselben Produktionsanlage) dem Kundenwunsch entsprechend (z.B. dem Hersteller von Superabsorbern (= wasserabsorbierende Harze)) den diesem zu liefernden "nachwachsenden Grad" der Acrylsäure (das vom Kunden für die zu liefernde Acrylsäure gewünschte Verhältnis n¹⁴C : n¹²C) nach Belieben einzustellen.

Durch Veresterung einer Acrylsäure, für die V = V* gilt, mit Biomethanol oder Bioethanol, können Acrylsäureester erhalten werden, deren n¹⁴C zu n¹²C-Verhältnis ebenfalls V* ist.

Ein weiterer Vorteil der erfindungsgemäßen Verfahrensweise besteht darin, dass das Zielprodukt der Reaktionszone A keiner Abtrennung aus dem Produktgasgemisch A bedarf, um zur Erzeugung des Reaktionsgaseingangsgemischs B herangezogen werden zu können. Dies gewährleistet für das erfindungsgemäße Verfahren sowohl eine hohe Ökonomie als auch eine effiziente Energiebilanz. Darüber hinaus wird bei einer Kondensation von Essigsäure mit Formaldehyd weder Glyoxal noch Propionsäure als Nebenprodukt gebildet, wie es bei einer heterogen katalysierten Partialoxidation von Propylen, Propan, Acrolein, Propionaldehyd und/oder Glyzerin zu Acrylsäure zwingend der Fall ist (vgl. WO 2010/074177).

Darüber hinaus gewährleistet das erfindungsgemäße Verfahren eine hohe Raum-Zeit-Ausbeute bei gleichzeitig hoher, auf die umgesetzten Edukte bezogener Zielproduktselektivität.

Damit umfasst die vorliegende Anmeldung insbesondere die nachfolgenden erfindungsgemäßen Ausführungsformen:
1. Verfahren zur Herstellung von Acrylsäure aus Methanol und Essigsäure, das folgende Maßnahmen umfasst:
   - durch eine erste Reaktionszone A, die mit wenigstens einem Oxidationskatalysator A beschickt ist, wird ein Strom eines die Reaktanden Methanol und molekularer Sauerstoff sowie wenigstens ein von Wasserdampf verschiedenes inertes Verdünnurigsgas enthaltenden Reaktionsgaseingangsgemischs A hindurchgeführt und beim Durchströmen der Reaktionszone A im Reaktionsgaseingangsgemisch A enthaltenes Methanol heterogen katalysiert zu Formaldehyd und Wasserdampf oxidiert, so dass ein Formaldehyd, Wasserdampf, wenigstens ein von Wasserdampf verschiedenes inertes Verdünnungsgas sowie gegebenenfalls überschüssigen molekularen Sauerstoff enthaltendes Produktgasgemisch A entsteht und ein Strom an Produktgasgemisch A die Reaktionszone A verlässt, wobei dem durch die Reaktionszone A strömenden Reaktionsgasgemisch A auf seinem Weg durch die Reaktionszone A wahlweise weiterer molekularer Sauerstoff und/oder weiteres inertes Verdünnungsgas zugeführt werden kann,
   - wahlweise wird der die Reaktionszone A verlassende Strom an Produktgasgemisch A einer Trennzone T* zugeführt und in der Trennzone T* im Produktgasgemisch A gegebenenfalls noch enthaltenes nicht umgesetztes Methanol vom Produktgasgemisch A abgetrennt, wobei ein Formaldehyd enthaltendes Produktgasgemisch A* verbleibt und ein Strom an Produktgasgemisch A* die Reaktionszone A verlässt,
   - aus dem Strom an Produktgasgemisch A oder aus dem Strom an Produktgasgemisch A* sowie wenigstens einem weiteren, Essigsäure enthaltenden, Stoffstrom, wird ein Strom eines Essigsäure, Wasserdampf, wenigstens ein von Wasserdampf verschiedenes inertes Verdünnungsgas, Formaldehyd und gegebenenfalls molekularen Sauerstoff enthaltenden Reaktionsgaseingangsgemischs B erzeugt, in welchem die enthaltene molare Menge n_{HAc} an Essigsäure größer ist, als die in ihm enthaltene molare Menge n_{Fd} an Formaldehyd,
   - durch eine zweite Reaktionszone B, die mit wenigstens einem Aldolkondensationskatalysator B beschickt ist, wird der Strom des Reaktionsgaseingangsgemischs B hindurchgeführt und beim Durchströmen der Reaktionszone B im Reaktionsgaseingangsgemisch B enthaltenes Formaldehyd mit im Reaktionsgaseingangsgemisch B enthaltener Essigsäure heterogen katalysiert zu Acrylsäure und H₂O kondensiert, so dass ein Acrylsäure, Essigsäure, Wasserdampf, wenigstens ein von Wasserdampf verschiedenes inertes Verdünnungsgas sowie gegebenenfalls molekularen Sauerstoff enthaltendes Produktgasgemisch B entsteht und ein Strom an Produktgasgemisch B die Reaktionszone B verlässt, wobei dem durch die Reaktionszone B strömenden Reaktionsgasgemisch B auf seinem Weg durch die Reaktionszone B wahlweise weiterer molekularer Sauerstoff und/oder weiteres inertes Verdünnungsgas zugeführt werden kann,
   - der die Reaktionszone B verlassende Strom an Produktgasgemisch B wird einer Trennzone T zugeführt und in der Trennzone T in wenigstens drei Stoffströme X, Y und Z aufgetrennt, wobei
   - der im Stoffstrom X enthaltene Acrylsäurestrom größer ist, als der in den Stoffströmen Y und Z zusammengenommen enthaltene Acrylsäurestrom,
   - der im Stoffstrom Y enthaltene Essigsäurestrom größer ist, als der in den Stoffströmen X und Z zusammengenommen enthaltene Essigsäurestrom,
   - der im Stoffstrom Z enthaltene Strom an von Wasserdampf verschiedenem inertem Verdünnungsgas größer ist, als der in den Stoffströmen X und Y zusammengenommen enthaltene Stoffstrom an von Wasserdampf verschiedenem inertem Verdünnungsgas,
      und
   - der Stoffstrom Y wird in die Reaktionszone B rückgeführt und zur Erzeugung des Reaktionsgaseingangsgemischs B mitverwendet.
2. Verfahren gemäß Ausführungsform 1, dadurch gekennzeichnet, dass in der Trennzone T* abgetrenntes Methanol zur Erzeugung des Reaktionsgaseingangsgemischs A in die Reaktionszone A rückgeführt wird.
3. Verfahren gemäß Ausführungsform 1 oder 2, dadurch gekennzeichnet, dass die Abtrennung des Methanols in der Trennzone T* rektifikativ erfolgt.
4. Verfahren gemäß einer der Ausführungsformen 1 bis 3, dadurch gekennzeichnet, dass der wenigstens eine Oxidationskatalysator A eine katalytisch aktive Masse aufweist, die wenigstens elementares Silber umfasst.
5. Verfahren gemäß der Ausführungsform 4, dadurch gekennzeichnet, dass die Reinheit des elementaren Silbers ≥ 99,7 Gew.-% beträgt.
6. Verfahren gemäß der Ausführungsform 4, dadurch gekennzeichnet, dass die Reinheit des elementaren Silbers ≥ 99,9 oder ≥ 99,99 Gew.-% beträgt.
7. Verfahren gemäß einer der Ausführungsformen 4 bis 6, dadurch gekennzeichnet, dass der wenigstens eine Oxidationskatalysator A Silberkristalle umfasst, deren Längstausdehnung im Bereich von 0,1 bis 5 mm liegt.
8. Verfahren gemäß Ausführungsform 7, dadurch gekennzeichnet, dass die Silberkristalle mit einer porösen Schicht aus oxidischem Material wenigstens eines der Elemente Al, Si, Zr und Ti überzogen sind, deren Dicke im Bereich 0,3 bis 10 µm liegt.
9. Verfahren gemäß einer der Ausführungsformen 4 bis 8, dadurch gekennzeichnet, dass der Gehalt des Reaktionsgaseingangsgemischs A an Methanol wenigstens 5 Vol.-% beträgt.
10. Verfahren gemäß Ausführungsform 9, dadurch gekennzeichnet, dass der Gehalt des Reaktionsgaseingangsgemischs A an Methanol nicht mehr als 60 Vol-% beträgt.
11. Verfahren gemäß einer der Ausführungsformen 4 bis 8, dadurch gekennzeichnet, dass der Gehalt des Reaktionsgaseingangsgemischs A an Methanol 15 bis 50 Vol.-% beträgt.
12. Verfahren gemäß einer der Ausführungsformen 4 bis 8, dadurch gekennzeichnet, dass der Gehalt des Reaktionsgaseingangsgemischs A an Methanol 20 bis 40 Vol.-% oder 20 bis 30 Vol.-% beträgt.
13. Verfahren gemäß einer der Ausführungsformen 4 bis 12, dadurch gekennzeichnet, dass das Reaktionsgaseingangsgemisch A den molekularen Sauerstoff in einer molaren Menge n_{O} und das Methanol in einer molaren Menge n_{Me} enthält und das Verhältnis no : n_{Me} kleiner als 1 ist.
14. Verfahren gemäß Ausführungsform 13, dadurch gekennzeichnet, dass n_{O} : n_{Me} 0,1 bis 0,8 oder 0,2 bis 0,6 beträgt.
15. Verfahren gemäß einer der Ausführungsformen 4 bis 14, dadurch gekennzeichnet, dass n_{O :} n_{Me} 0,3 bis 0,5 beträgt.
16. Verfahren gemäß einer der Ausführungsformen 4 bis 15, dadurch gekennzeichnet, dass das Reaktionsgaseingangsgemisch A ≥ 0 bis 50 Vol.-% an H₂O enthält.
17. Verfahren gemäß Ausführungsform 16, dadurch gekennzeichnet, dass das Reaktionsgaseingangsgemisch A 15 bis 35 Vol.-% oder 20 bis 30 Vol.-% an H₂O enthält.
18. Verfahren gemäß einer der Ausführungsformen 4 bis 17, dadurch gekennzeichnet, dass das Reaktionsgaseingangsgemisch A als wenigstens ein von Wasserdampf verschiedenes inertes Verdünnungsgas N₂ enthält.
19. Verfahren gemäß Ausführungsform 18, dadurch gekennzeichnet, dass das Reaktionsgaseingangsgemisch A 20 bis 80 Vol.-% N₂ enthält.
20. Verfahren gemäß Ausführungsform 18 oder 19, dadurch gekennzeichnet, dass das Reaktionsgaseingangsgemisch A 30 bis 70 Vol.-% N₂ enthält.
21. Verfahren gemäß einer der Ausführungsformen 18 bis 20, dadurch gekennzeichnet, dass das Reaktionsgaseingangsgemisch A 40 bis 60 Vol.-% N₂ enthält.
22. Verfahren gemäß einer der Ausführungsformen 4 bis 21, dadurch gekennzeichnet, dass das Methanol in der Reaktionszone A bei einer Reaktionstemperatur im Bereich von 400 bis 800°C zu Formaldehyd und Wasser oxidiert wird.
23. Verfahren gemäß einer der Ausführungsformen 4 bis 22, dadurch gekennzeichnet, dass das Methanol in der Reaktionszone A bei einer Reaktionstemperatur im Bereich von 500 bis 800°C zu Formaldehyd und Wasser oxidiert wird.
24. Verfahren gemäß einer der Ausführungsformen 4 bis 22, dadurch gekennzeichnet, dass das Methanol in der Reaktionszone A bei einer Reaktionstemperatur im Bereich von 450 bis 650°C, oder von 500 bis 600°C zu Formaldehyd und Wasser oxidiert wird.
25. Verfahren gemäß einer der Ausführungsformen 4 bis 22, dadurch gekennzeichnet, dass das Methanol in der Reaktionszone A bei einer Reaktionstemperatur im Bereich von 600 bis 750°C zu Formaldehyd und Wasser oxidiert wird.
26. Verfahren gemäß einer der Ausführungsformen 4 bis 25, dadurch gekennzeichnet, dass das Methanol in der Reaktionszone A bei einem Arbeitsdruck im Bereich von 10³ bis 10⁶ Pa oder von 10⁴ bis 2·10⁵ Pa zu Formaldehyd und Wasser oxidiert wird.
27. Verfahren gemäß einer der Ausführungsformen 1 bis 3, dadurch gekennzeichnet, dass der wenigstens eine Oxidationskatalysator A eine katalytisch aktive Masse aufweist, die ein Mischoxid ist, das wenigstens ein Übergangsmetall im oxidierten Zustand aufweist.
28. Verfahren gemäß Ausführungsform 27, dadurch gekennzeichnet, dass das wenigstens eine Übergangsmetall Mo und/oder V umfasst.
29. Verfahren gemäß Ausführungsform 27, dadurch gekennzeichnet, dass das wenigstens eine Übergangsmetall Mo und Fe umfasst.
30. Verfahren gemäß Ausführungsform 27, dadurch gekennzeichnet, dass die katalytisch aktive Masse ein Mischoxid der allgemeinen Formel I,

   [Fe₂ (MoO₄)₃]₁ [M¹ₘOₙ]_{q} (I),

   in der die Variablen folgende Bedeutung aufweisen:
   - M¹ =: Mo und/oder Fe, oder
   Mo und/oder Fe sowie bezogen auf die molare Gesamtmenge aus Mo und Fe eine molare Gesamtmenge von bis zu 10 mol-% (z.B. 0,01 bis 10 mol-%, oder 0,1 bis 10 mol-%), vorzugsweise zu nicht mehr als 5 mol-%, eines oder mehrere Elemente aus der Gruppe bestehend aus Ti, Sb, Sn, Ni, Cr, Ce, Al, Ca, Mg, V, Nb, Ag, Mn, Cu, Co, Si, Na, K, Tl, Zr, W, Ir, Ta, As, P und B,
   - q =: 0 bis 5,
   - m =: 1 bis 3,
   - n =: 1 bis 6,
   ist.
31. Verfahren gemäß Ausführungsform 30, dadurch gekennzeichnet, dass q = 0,5 bis 3 ist.
32. Verfahren gemäß Ausführungsform 30 oder 31, dadurch gekennzeichnet, dass q = 1 bis 2 ist.
33. Verfahren gemäß einer der Ausführungsformen 30 bis 32, dadurch gekennzeichnet, dass M¹ = Mo, m = 1 und n = 3 ist.
34. Verfahren gemäß einer der Ausführungsformen 30 bis 33, dadurch gekennzeichnet, dass M¹ = Fe, m = 2 und n = 3 ist.
35. Verfahren gemäß einer der Ausführungsformen 30 bis 34, dadurch gekennzeichnet, dass weniger als 50 mol-% des im Mischoxid I enthaltenen Fe in der Oxidationsstufe +2 vorliegt.
36. Verfahren gemäß einer der Ausführungsformen 30 bis 34, dadurch gekennzeichnet, dass weniger als 20 mol-% des im Mischoxid I enthaltenen Fe in der Oxidationsstufe +2 vorliegt.
37. Verfahren gemäß einer der Ausführungsformen 30 bis 34, dadurch gekennzeichnet, dass weniger als 10 mol-% des im Mischoxid I enthaltenen Fe in der Oxidationsstufe +2 vorliegt.
38. Verfahren gemäß einer der Ausführungsformen 30 bis 34, dadurch gekennzeichnet, dass die Gesamtmenge des im Mischoxid I enthaltenen Fe in der Oxidationsstufe +3 vorliegt.
39. Verfahren gemäß einer der Ausführungsformen 30 bis 38, dadurch gekennzeichnet, dass das Verhältnis n_{Mo} : n_{Fe}, gebildet aus der im Mischoxid I enthaltenen molaren Menge an Mo und der in demselben Mischoxid I enthaltenen molaren Menge an Fe, 1:1 bis 5:1 beträgt.
40. Verfahren gemäß einer der Ausführungsformen 30 bis 38, dadurch gekennzeichnet, dass die katalytisch aktive Masse sich formal als ein Gemisch aus MoO₃ und Fe₂O₃ darstellen lässt, wobei der Gehalt des Gemischs an MoO₃ 65 bis 95 Gew.-% und der Gehalt des Gemischs an Fe₂O₃ 5 bis 35 Gew.-% beträgt.
41. Verfahren gemäß einer der Ausführungsformen 27 bis 40, dadurch gekennzeichnet, dass der wenigstens eine Oxidationskatalysator A ein Vollkatalysator ist.
42. Verfahren gemäß Ausführungsform 41, dadurch gekennzeichnet, dass die Geometrie des Vollkatalysators ausgewählt ist aus der Gruppe bestehend aus Kugel, Ring und Vollzylinder.
43. Verfahren gemäß Ausführungsform 42, dadurch gekennzeichnet, dass die Längstausdehnung des Vollkatalysators 1 bis 10 mm beträgt.
44. Verfahren gemäß Ausführungsform 41, dadurch gekennzeichnet, dass der Vollkatalysator die Geometrie eines Rings mit einem Aussendurchmesser von 3 bis 10 mm, einer Höhe von 1 bis 10 mm und einem Innendurchmesser von 1 bis 8 mm aufweist.
45. Verfahren gemäß Ausführungsform 44, dadurch gekennzeichnet, dass der Ring eine Wanddicke von 1 bis 3 mm aufweist.
46. Verfahren gemäß einer der Ausführungsformen 27 bis 40, dadurch gekennzeichnet, dass der wenigstens eine Oxidationskatalysator A ein Schalenkatalysator ist, der das katalytisch aktive Mischoxid als Schale auf die Oberfläche eines inerten Trägerformkörpers aufgebracht aufweist.
47. Verfahren gemäß Ausführungsform 46, dadurch gekennzeichnet, das der Trägerformkörper eine Kugel oder ein Ring ist.
48. Verfahren gemäß Ausführungsform 47, dadurch gekennzeichnet, dass die Längstausdehnung des Trägerformkörpers 1 bis 10 mm beträgt.
49. Verfahren gemäß Ausführungsform 46, dadurch gekennzeichnet, dass der inerte Trägerformkörper ein Ring mit einer Länge von 2 bis 10 mm, einem Außendurchmesser von 4 bis 10 mm und einer Wanddicke von 1 bis 4 mm ist.
50. Verfahren gemäß einer der Ausführungsformen 46 bis 49 dadurch gekennzeichnet, dass der inerte Trägerformkörper aus Steatit ist.
51. Verfahren gemäß einer der Ausführungsformen 46 bis 50, dadurch gekennzeichnet, dass die Schale aus katalytisch aktivem Mischoxid eine Dicke von 10 bis 2000 µm, oder 10 bis 500 µm, oder 100 bis 500 µm, oder 200 bis 300 µm hat.
52. Verfahren gemäß einer der Ausführungsformen 27 bis 51, dadurch gekennzeichnet, dass das Reaktionsgaseingangsgemisch A nicht mehr als 15 Vol.-% Methanol enthält.
53. Verfahren gemäß einer der Ausführungsformen 27 bis 51, dadurch gekennzeichnet, dass das Reaktionsgaseingangsgemisch A nicht mehr als 11 Vol.-% Methanol enthält.
54. Verfahren gemäß einer der Ausführungsformen 27 bis 53, dadurch gekennzeichnet, dass das Reaktionsgaseingangsgemisch A 2 bis 10 Vol.-% Methanol enthält.
55. Verfahren gemäß einer der Ausführungsformen 27 bis 54, dadurch gekennzeichnet, dass das Reaktionsgaseingangsgemisch A 6 bis 9 Vol.-% Methanol enthält.
56. Verfahren gemäß einer der Ausführungsformen 27 bis 55, dadurch gekennzeichnet, dass das Reaktionsgaseingangsgemisch A den molekularen Sauerstoff in einer molaren Menge n_{O} und das Methanol in einer molaren Menge n_{Me} enthält und das Verhältnis n_{O} : n_{Me} wenigstens 1 oder größer als 1 ist.
57. Verfahren gemäß Ausführungsform 56, dadurch gekennzeichnet, dass das Verhältnis n_{O} : n_{Me} 1,1 bis 5 beträgt.
58. Verfahren gemäß Ausführungsform 56 oder 57, dadurch gekennzeichnet, dass das Verhältnis n_{O} : n_{Me} 1,5 bis 3,5 beträgt.
59. Verfahren gemäß einer der Ausführungsformen 27 bis 58, dadurch gekennzeichnet, dass das Reaktionsgaseingangsgemisch A als wenigstens ein von Wasserdampf verschiedenes inertes Verdünnungsgas N₂ enthält.
60. Verfahren gemäß Ausführungsform 59, dadurch gekennzeichnet, dass das Reaktionsgaseingangsgemisch A 70 bis 95 Vol.-% N₂ enthält.
61. Verfahren gemäß einer der Ausführungsformen 27 bis 60, dadurch gekennzeichnet, dass das Reaktionsgaseingangsgemisch A 0 bis 20 Vol.-% H₂O enthält.
62. Verfahren gemäß Ausführungsform 61, dadurch gekennzeichnet, dass das Reaktionsgaseingangsgemisch A 0,1 bis 10 Vol.-% H₂O enthält.
63. Verfahren gemäß Ausführungsform 61 oder 62, dadurch gekennzeichnet, dass das Reaktionsgaseingangsgemisch A 0,2 bis 7 Vol.-% H₂O enthält.
64. Verfahren gemäß einer der Ausführungsformen 60 bis 62, dadurch gekennzeichnet, dass das Reaktionsgaseingangsgemisch A 0,5 bis 5 Vol.-% H₂O enthält.
65. Verfahren gemäß einer der Ausführungsformen 27 bis 64, dadurch gekennzeichnet, dass das Methanol in der Reaktionszone A bei einer Reaktionstemperatur im Bereich von 250 bis 500°C zu Formaldehyd und Wasser oxidiert wird.
66. Verfahren gemäß Ausführungsform 65, dadurch gekennzeichnet, dass das Methanol in der Reaktionszone A bei einer Reaktionstemperatur im Bereich von 250 bis 400°C zu Formaldehyd und Wasser oxidiert wird.
67. Verfahren gemäß einer der Ausführungsformen 27 bis 66, dadurch gekennzeichnet, dass das Methanol in der Reaktionszone A bei einem Arbeitsdruck im Bereich von 10³ bis 10⁶ Pa oder von 10⁴ bis 2·10⁵ Pa zu Formaldehyd und Wasser oxidiert wird.
68. Verfahren gemäß einer der Ausführungsformen 67, dadurch gekennzeichnet, dass eine Teilmenge des Stoffstroms Y zur Erzeugung des Reaktionsgaseingangsgemischs A in die Reaktionszone A rückgeführt wird.
69. Verfahren gemäß einer der Ausführungsformen 1 bis 68, dadurch gekennzeichnet, dass die in dem wenigstens einen weiteren Stoffstrom enthaltene Essigsäure eine durch homogene Katalyse katalysierte Carbonylierung von Methanol in der Flüssigphase erzeugte Essigsäure ist.
70. Verfahren gemäß Ausführungsform 69, dadurch gekennzeichnet, dass der Katalysator Rh in Kombination mit HI und CH₃I umfasst.
71. Verfahren gemäß Ausführungsform 69 oder 70, dadurch gekennzeichnet, dass die in dem wenigstens einen weiteren Stoffstrom enthaltene Essigsäure eine vom Produktgemisch der homogen katalysierten Carbonylierung von Methanol zu Essigsäure in der Flüssigphase rektifikativ abgetrennte Essigsäure ist.
72. Verfahren gemäß einer der Ausführungsformen 1 bis 68, dadurch gekennzeichnet, dass der wenigstens eine weitere, Essigsäure enthaltende, Stoffstrom das Produktgasgemisch einer in Abwesenheit von Halogen-haltigen Verbindungen heterogen katalysierten Gasphasencarbonylierung von Methanol zu Essigsäure ist.
73. Verfahren gemäß einer der Ausführungsformen 1 bis 72, dadurch gekennzeichnet, dass die Reaktionstemperatur in der Reaktionszone B 200 bis 400°C beträgt.
74. Verfahren gemäß einer der Ausführungsformen 1 bis 72, dadurch gekennzeichnet, dass die Reaktionstemperatur in der Reaktionszone B 280 bis 380°C beträgt.
75. Verfahren gemäß einer der Ausführungsformen 1 bis 72, dadurch gekennzeichnet, dass die Reaktionstemperatur in der Reaktionszone B 300 bis 370°C beträgt.
76. Verfahren gemäß einer der Ausführungsformen 1 bis 75, dadurch gekennzeichnet, dass der Arbeitsdruck in der Reaktionszone B 1,2·10⁵ Pa bis 50·10⁵ Pa beträgt.
77. Verfahren gemäß einer der Ausführungsformen 1 bis 76, dadurch gekennzeichnet, dass der Gehalt des Reaktionsgaseingangsgemischs B an Formaldehyd 0,5 bis 10 Vol-% beträgt.
78. Verfahren gemäß einer der Ausführungsformen 1 bis 76, dadurch gekennzeichnet, dass der Gehalt des Reaktionsgaseingangsgemischs B an Formaldehyd 0,5 bis 7 Vol-% beträgt.
79. Verfahren gemäß einer der Ausführungsformen 1 bis 76, dadurch gekennzeichnet, dass der Gehalt des Reaktionsgaseingangsgemischs B an Formaldehyd 1 bis 5 Vol-% beträgt.
80. Verfahren gemäß einer der Ausführungsformen 1 bis 79, dadurch gekennzeichnet, dass das Reaktionsgaseingangsgemisch B die Essigsäure in einer molaren Menge n_{HAc} und das Formaldehyd in einer molaren Menge n_{Fd} enthält und das Verhältnis n_{HAc} : n_{Fd} größer als 1 und ≤ 10 ist.
81. Verfahren gemäß Ausführungsform 80, dadurch gekennzeichnet, dass das Verhältnis n_{HAc} : n_{Fd} 1,1 bis 5 beträgt.
82. Verfahren gemäß Ausführungsform 80, dadurch gekennzeichnet, dass das Verhältnis n_{HAc} : n_{Fd} 1,5 bis 3,5 beträgt.
83. Verfahren gemäß einer der Ausführungsformen 1 bis 82, dadurch gekennzeichnet, dass der Essigsäuregehalt des Reaktionsgaseingangsgemischs B 1,5 bis 20 Vol.-% beträgt.
84. Verfahren gemäß einer der Ausführungsformen 1 bis 82, dadurch gekennzeichnet, dass der Essigsäuregehalt des Reaktionsgaseingangsgemischs B 2 bis 15 Vol.-% beträgt.
85. Verfahren gemäß einer der Ausführungsformen 1 bis 82, dadurch gekennzeichnet, dass der Essigsäuregehalt des Reaktionsgaseingangsgemischs B 3 bis 10 Vol.-% beträgt.
86. Verfahren gemäß einer der Ausführungsformen 1 bis 85, dadurch gekennzeichnet, dass der Gehalt des Reaktionsgaseingangsgemischs B an molekularem Sauerstoff 0,5 bis 5 Vol-% beträgt.
87. Verfahren gemäß einer der Ausführungsformen 1 bis 85, dadurch gekennzeichnet, dass der Gehalt des Reaktionsgaseingangsgemischs B an molekularem Sauerstoff 2 bis 5 Vol-% beträgt.
88. Verfahren gemäß einer der Ausführungsformen 1 bis 87, dadurch gekennzeichnet, dass der Wasserdampfgehalt des Reaktionsgaseingangsgemischs B 30 Vol.-% nicht überschreitet und 0,5 Vol.-% nicht unterschreitet.
89. Verfahren gemäß einer der Ausführungsformen 1 bis 87, dadurch gekennzeichnet, dass der Wasserdampfgehalt des Reaktionsgaseingangsgemischs B 20 Vol.-% nicht überschreitet und 1 Vol.-% nicht unterschreitet.
90. Verfahren gemäß einer der Ausführungsformen 1 bis 87, dadurch gekennzeichnet, dass der Wasserdampfgehalt des Reaktionsgaseingangsgemischs B 0,5 bis 15 Vol.-% oder 1 bis 10 Vol.-% beträgt.
91. Verfahren gemäß einer der Ausführungsformen 1 bis 90, dadurch gekennzeichnet, dass der Gehalt des Reaktionsgaseingangsgemischs B an von Wasserdampf verschiedenem inertem Verdünnungsgas wenigstens 30 Vol.-% oder wenigstens 40 Vol.-% beträgt.
92. Verfahren gemäß einer der Ausführungsformen 1 bis 90, dadurch gekennzeichnet, dass der Gehalt des Reaktionsgaseingangsgemischs B an von Wasserdampf verschiedenem inertem Verdünnungsgas wenigstens 50 Vol.-% beträgt.
93. Verfahren gemäß einer der Ausführungsformen 1 bis 92, dadurch gekennzeichnet, dass das Reaktionsgaseingangsgemisch B als wenigstens ein von Wasserdampf verschiedenes inertes Verdünnungsgas wenigstens 30 Vol.-% oder wenigstens 40 Vol.-% N₂ enthält.
94. Verfahren gemäß einer der Ausführungsformen 1 bis 92, dadurch gekennzeichnet, dass das Reaktionsgaseingangsgemischs B als wenigstens ein von Wasserdampf verschiedenes inertes Verdünnungsgas wenigstens 50 Vol.-% N₂ enthält.
95. Verfahren gemäß einer der Ausführungsformen 1 bis 94, dadurch gekennzeichnet, dass der wenigstens eine Aldolkondensationskatalysator B ein Zeolith mit anionischer Gerüstladung ist, an dessen innerer und äußerer Oberfläche sich wenigstens eine Kationensorte aus der Gruppe der Alkali- und Erdalkaliionen befindet, um die negative Gerüstladung zu neutralisieren.
96. Verfahren gemäß einer der Ausführungsformen 1 bis 94, dadurch gekennzeichnet, dass der wenigstens eine Aldolkondensationskatalysator B auf amorphes Siliziumdioxid aufgebrachtes Hydroxid aus der Gruppe bestehend aus den Alkalihydroxiden, Erdalkalihydroxiden und Aluminiumhydroxid ist.
97. Verfahren gemäß der Ausführungsform 96, dadurch gekennzeichnet, dass das auf das amorphe Siliziumdioxid aufgebrachte Hydroxid KOH, NaOH, Ca(OH)₂ oder Mg(OH)₂ ist.
98. Verfahren gemäß einer der Ausführungsformen 1 bis 94, dadurch gekennzeichnet, dass der wenigstens eine Alkolkondensationskatalysator B ein Katalysator ist, der
   - als Bestandteil a) wenigstens ein Oxid von wenigstens einem der Elemente Si, Al, Ti, Zr, Cd, Sn, Ga, Y und La und/oder Zeolith,
      und
   - als Bestandteil b) wenigstens ein Oxid ausgewählt aus Boroxid und Phosphoroxid sowie wahlweise
   - als Bestandteil c) ein oder mehr als ein Oxid von wenigstens einem der Elemente V, Cr, Co, Ni, Mo und Pb und/oder mehr als eine Heteropolysäure mit wenigstens einem Polyatom ausgewählt aus V, Mo und W
      umfasst.
99. Verfahren gemäß Ausführungsform 98, dadurch gekennzeichnet, dass der wenigstens eine Aldolkondenstionskatalysator B 1 bis 50 Gew.-% Boroxid, oder 1 bis 50 Gew.-% Phosphoroxid, oder 1 bis 50 Gew.-% Boroxid und Phosphoroxid aufweist, wobei das Boroxid, bezogen auf die enthaltene Menge an B, stets als B₂O₃ und das Phosphoroxid, bezogen auf die enthaltene Menge an P, stets als P₂O₅ gerechnet wird.
100. Verfahren gemäß einer der Ausführungsformen 1 bis 94, dadurch gekennzeichnet, dass der wenigstens eine Aldokondensationskatalysator B eine katalytisch aktive Masse aufweist, die ein Vanadin- Phosphoroxid oder ein mit von Vanadin und Phosphor verschiedenen Elementen dotiertes Vanadin-Phosphoroxid ist.
101. Verfahren gemäß Ausführungsform 100, dadurch gekennzeichnet, dass die katalytisch aktive Masse eine Multielementoxidaktivmasse der allgemeinen Formel II,

   V₁P_{b}Fe_{c}X¹_{d}X²ₑOₙ (II),

   in der die Variablen folgende Bedeutung aufweisen:
   - X¹ =: Mo, Bi, Co, Ni, Si, Zn, Hf, Zr, Ti, Cr, Mn, Cu, B, Sn und/oder Nb,
   - X² =: Li, K, Na, Rb, Cs und/oder Tl,
   - b =: 0,9 bis 2,0
   - c =: ≥ 0 bis 0,1,
   - d =: ≥ 0 bis 0,1,
   - e =: ≥ 0 bis 0,1, und
   - n =: der stöchiometische Koeffizient des Elements Sauerstoff, der durch die stöchiometrischen Koeffezienten der von Sauerstoff verschiedenen Elemente sowie deren Ladungzahl in II bestimmt wird,
   ist.
102. Verfahren gemäß Ausführungsform 101, dadurch gekennzeichnet, dass X¹ = Nb, Mo, Zn und/oder Hf ist.
103. Verfahren gemäß Ausführungsform 101 oder 102, dadurch gekennzeichnet, dass b 0,9 bis 1,5 ist.
104. Verfahren gemäß Ausführungsform 101 oder 102, dadurch gekennzeichnet, dass b 0,9 bis 1,2 ist.
105. Verfahren gemäß einer der Ausführungsformen 101 bis 104, dadurch gekennzeichnet, dass X¹ = Mo ist.
106. Verfahren gemäß einer der Ausführungsformen 101 bis 105, dadurch gekennzeichnet, dass c 0,005 bis 0,1 ist.
107. Verfahren gemäß einer der Ausführungsformen 101 bis 105, dadurch gekennzeichnet, dass c 0,005 bis 0,05 oder 0,005 bis 0,02 ist.
108. Verfahren gemäß Ausführungsform 100, dadurch gekennzeichnet, dass das Verhältnis nₚ : nᵥ von der in der katalytisch aktiven Masse enthaltenen molaren Menge nₚ an Phosphor zu der in der katalytisch aktiven Masse enthaltenen molaren Menge nᵥ an V 0,09 bis 2,0, vorzugsweise 0,9 bis 1,5 und besonders bevorzugt 0,9 bis 1,2 beträgt.
109. Verfahren gemäß einer der Ausführungsformen 100 oder 108, dadurch gekennzeichnet, dass die in der katalytisch aktiven Masse enthaltenen, von Vanadin und Phosphor verschienen Elemente ein oder mehr als ein Element aus der Gruppe bestehend aus Lithium, Kalium, Natrium, Rubidium, Cäsium, Thallium, Molybdän, Zink, Hafnium, Zirkon, Titan, Chrom, Mangan, Nickel, Kupfer, Eisen, Bor, Silizium, Zinn, Niob, Kobalt und Wismut sind.
110. Verfahren gemäß Ausführungsform 109, dadurch gekennzeichnet, dass der Gesamtgehalt an von Vanadin und Phosphor verschiedenen Elementen in der katalytisch aktiven Masse, bezogen auf deren Gewicht, nicht mehr als 5 Gew.-% beträgt, wobei das jeweilige von Vanadin und Phosphor verschiedene Element als elektrisch neutrales Oxid gerechnet wird, in welchem das Element die gleiche Ladungszahl wie in der Aktivmasse aufweist.
111. Verfahren gemäß einer der Ausführungsformen 100 bis 110, dadurch gekennzeichnet, dass die arithmetisch mittlere Oxidationsstufe des Vanadins in der katalytisch aktiven Masse +3,9 bis + 4,4 oder +4,0 bis +4,3 beträgt.
112. Verfahren gemäß einer der Ausführungsformen 100 bis 111, dadurch gekennzeichnet, dass die spezifische BET-Oberfläche der katalytisch aktiven Masse ≥ 15 bis 50 m²/g beträgt.
113. Verfahren gemäß einer der Ausführungsformen 100 bis 112, dadurch gekennzeichnet, dass das Porengesamtvolumen der katalytisch aktiven Masse 0,1 bis 0,5 ml/g beträgt.
114. Verfahren gemäß einer der Ausführungsformen 100 bis 113, dadurch gekennzeichnet, dass das Porengesamtvolumen der katalytisch aktiven Masse 0,15 bis 0,4 ml/g beträgt.
115. Verfahren gemäß einer der Ausführungsformen 100 bis 114, dadurch gekennzeichnet, dass der wenigstens eine Oxidationskatalysator B ein Vollkatalysator oder ein Trägerkatalysator ist.
116. Verfahren gemäß der Ausführungsform 115, dadurch gekennzeichnet, dass die Geometrie des Vollkatalysators ausgewählt ist aus der Gruppe bestehend aus Kugel, Ring und Vollzylinder, sowie eine Längstausdehnung im Bereich von 1 bis 10 mm aufweist.
117. Verfahren gemäß der Ausführungsform 115, dadurch gekennzeichnet, dass die Geometrie des Vollkatalysators ein Ring (ein Hohlzylinder) ist, mit einem Außendurchmesser im Bereich von 3 bis 10 mm, einer Höhe von 1 bis 10 mm, einem Innendurchmesser von 1 bis 8 mm und einer Wanddicke von 1 bis 3 mm.
118. Verfahren gemäß einer der Ausführungsformen 100 bis 114, dadurch gekennzeichnet, dass der wenigstens eine Aldokondensationskatalysator B ein Schalenkatalysator ist, der die katalytisch aktive Masse als Schale auf die Oberfläche eines inerten Trägerformkörpers aufgebracht aufweist.
119. Verfahren gemäß Ausführungsform 118, dadurch gekennzeichnet, dass der Trägerformkörper eine Kugel oder ein Ring ist.
120. Verfahren gemäß Ausführungsform 118 oder 119, dadurch gekennzeichnet, dass die Längstausdehnung des Trägerformkörpers 1 bis 10 mm beträgt.
121. Verfahren gemäß einer der Ausführungsformen 118 bis 120, dadurch gekennzeichnet, dass der inerte Trägerformkörper aus Steatit ist.
122. Verfahren gemäß einer der Ausführungsformen 118 bis 121, dadurch gekennzeichnet, dass die Dicke der Schale aus Aktivmasse 10 bis 2000 µm, oder 10 bis 500 µm, oder 100 bis 500 µm, oder 200 bis 300 µm beträgt.
123. Verfahren gemäß einer der Ausführungsformen 1 bis 122, dadurch gekennzeichnet, dass zur Auftrennung des Produktgasgemischs B in der Trennzone T das Produktgasgemisch B, wahlweise nach direkter und/oder indirekter Abkühlung desselben, in eine mit trennwirksamen Einbauten ausgerüstete Kondensationskolonne geleitet und innerhalb der Kondensationskolonne fraktionierend kondensiert wird und die Stoffströme X, Y und Z als voneinander getrennte Fraktionen aus der Kondensationskolonne herausgeführt werden.
124. Verfahren gemäß einer der Ausführungsformen 1 bis 123, dadurch gekennzeichnet, dass zur Auftrennung des Produktgasgemischs B in der Trennzone T das Produktgasgemisch B, wahlweise nach direkter und/oder indirekter Abkühlung desselben, in einer mit trennwirksamen Einbauten ausgestatteten Absorptionskolonne im Gegenstrom zu einem bei Normaldruck höher als Acrylsäure siedenden organischen Lösungsmittel geführt und die im Produktgasgemisch B enthaltene Essigsäure und Acrylsäure unter Erhalt eines Absorbats in das Lösungsmittel absorbiert wird, während ein Stoffstrom Z die Absorptionskolonne an deren Kopf verlässt, und vom Absorbat anschließend die Stoffströme X und Y durch fraktionierende Destillation desselben in einer Rektifikationskolonne als getrennte Fraktionen abgetrennt werden.
125. Verfahren gemäß einer der Ausführungsformen 1 bis 123, dadurch gekennzeichnet, dass zur Auftrennung des Produktgasgemischs B in der Trennzone T das Produktgasgemisch B, wahlweise nach direkter und/oder indirekter Abkühlung desselben, in einer mit trennwirksamen Einbauten ausgestatteten Absorptionskolonne im Gegenstrom zu einer wässrigen Lösung als Absorptionsmittel geführt und die im Produktgasgemisch B enthaltene Essigsäure und Acrylsäure unter Erhalt eines Absorbats in das Lösungsmittel absorbiert wird während ein Stoffstrom Z die Absorptionskolonne an deren Kopf verlässt, und vom Absorbat anschließend die Stoffströme X und Y durch fraktionierende Destillation desselben in einer Rektifikationskolonne als getrennte Fraktionen abgetrennt werden.
126. Acrylsäure, für die das Verhältnis V aus der in dieser Acrylsäure enthaltenen molaren Menge n¹⁴C an ¹⁴C-Atomkernen zu der in derselben Acrylsäure enthaltenen molaren Menge n¹²C an ¹²C-Atomkernen, V = n¹⁴C : n¹²C, größer als 0 und kleiner als das entsprechende im Kohlendioxid der Atmosphäre auf der Erde vorliegende molare Verhältnis V* von ¹⁴C-Atomkernen zu ¹²C-Atomkernen ist.
127. Acrylsäure gemäß Ausführungsform 126, dadurch gekennzeichnet, dass V = (1/3)·V* ist.
128. Acrylsäure gemäß Ausführungsform 126, dadurch gekennzeichnet, dass V = (2/3)·V* ist.
129. Wenigstens 1 kg Acrylsäure enthaltende flüssige Phase P, dadurch gekennzeichnet, dass die enthaltene Acrylsäure eine Acrylsäure gemäß einer der Ausführungsformen 126 bis 128 ist.

### Beispiele

### I) Herstellung verschiedener Katalysatoren

### A) Herstellung eines Mischoxidkatalysators für die heterogen katalysierte partielle Gasphasenoxidation von Methanol zu Formaldehyd nach dem FORMOX-Verfahren

In einer eine Temperatur von 60°C aufweisenden Mischung aus 800 ml Wasser und 250 g einer 25 gew.-%igen wässrigen Ammoniaklösung wurden unter Aufrechterhaltung der 60°C 530 g Ammoniumheptamolybdattetrahydrat ((NH₄)₆Mo₇O₂₄·4H₂O) gelöst. Dabei wurde eine 60°C warme Lösung 1 erhalten.

In 1000 ml 60°C warmem Wasser wurden 808 g Eisen-(III)-nitratnonahydrat (Fe(NO₃)₃·9H₂O) unter Beibehalt der 60°C gelöst. Dabei wurde eine 60°C warme Lösung 2 erhalten.

Innerhalb von 20 min wurde die Lösung 2 unter Beibehalt der 60°C kontinuierlich in die Lösung 1 eingerührt. Anschließend wurde noch 5 min bei 60°C nachgerührt. Die erhaltene wässrige Suspension wurde anschließend bei einer Eintrittstemperatur von 340°C und einer Austrittstemperatur von 110°C im Luftstrom innerhalb von 1,5 h sprühgetrocknet (Sprühturm vom Typ Mobile Minor 2000 (MM-I) der Firma Niro A/S, Gladsaxevej 305, 2860 Soborg, Dänemark, mit einem Zentrifugalzerstäuber vom Typ F01A und einem Zerstäuberrad vom Typ SL24-50). Während der Sprühtrocknung wurde der jeweils noch nicht gesprühte Anteil der Suspension unter Beibehalt der 60°C weitergerührt.

Das so erhaltene Sprühpulver wurde, bezogen auf sein Gewicht, mit 1 Gew.-% Graphit TIMREX^{®} T44 der Firma Timcal AG (vgl. WO 2008/087116) homogen vermischt (in einem Rhönradmischer; Raddurchmesser: 650 mm, Trommelvolumen: 5 l, Drehzahl: ca. 30 U/min, Mischdauer: 30 min). Das resultierende Gemisch wurde dann in einem Walzenkompaktor vom Typ RCC 100x20 der Fa. Powtec mit 2 gegenläufigen Stahlwalzen bei einem Pressdruck von 12 bar kompaktiert und anschließend durch ein Sieb mit quadratischen Maschen einer Maschenweite von 0,8 mm gedrückt. Das resultierende Kompaktat (es wies ein Schüttgewicht von 1050 g/l und eine im Wesentlichen einheitliche Körnung von ≥ 0,4 mm und ≤ 0,8 mm auf) wurde anschließend im vorgenannten Rhönradmischer bei einer Drehzahl von ca. 30 U/min innerhalb von 30 min mit, bezogen auf sein Gewicht, 3 Gew.-% desselben Graphits vermischt und anschließend wie in der DE-A 10 2008040093 beschrieben zu ringförmigen Vollkatalysatorvorläuferformkörpern der Geometrie 5 mm x 3 mm x 3 mm (Außendurchmesser x Höhe x Innendurchmesser) mit einer Seitendrückfestigkeit von 22 ± 5 N und einer Masse von 130 mg verdichtet (Füllhöhe: 7,5 - 9 mm; Presskraft: 3,0 - 3,5 kN; Kilian Rundläufer (9-fach-Tablettiermaschine) vom Typ S100 (Fa. Kilian, D-50735 Köln). Die Tablettierung erfolgte unter Stickstoffatmosphäre.

Zur abschließenden thermischen Behandlung wurden die Katalysatorvorläuferformkörper gleichmäßig auf 4 nebeneinander angeordnete Gitternetze mit einer quadratischen Grundfläche von jeweils 150 mm x 150 mm (Schütthöhe: ca. 40 mm) aufgeteilt und in einem von Luft durchströmten Umluftofen (Firma Heraeus Instruments GmbH, D-63450 Hanau, Typ: K 750/2) wie folgt behandelt. Der Luftstrom betrug 100 Nl/h und wies anfänglich eine Temperatur von 120°C auf. Die 120°C wurden zunächst für 10 h aufrechterhalten. Dann wurde die Temperatur innerhalb von 10 h im Wesentlichen linear auf 460°C erhöht und die 460°C anschließend für weitere 4 h aufrechterhalten. Danach wurde innerhalb von 5 h auf 25°C abgekühlt.

Es wurde so ein ringförmiger Vollkatalysator-Oxidationskatalysator A erhalten, dessen Mischoxidaktivmasse die Stöchiometrie Fe₂(MoO₄)₃ aufwies. Anschließend wurden die Vollkatalysatorringe durch ein Sieb mit quadratischen Maschen einer Maschenweite von 0,8 mm gedrückt. Der Siebdurchgang (splittförmiger Oxidationskatalysator A) wies eine im Wesentlichen einheitliche Körnung (Längstausdehnung) von ≥ 0,4 mm und ≤ 0,8 mm auf.

### B) Herstellung eines Aldolkondensationskatalysators B, dessen Aktivmasse ein mit Fe(III) dotiertes Vanadin-Phosphoroxid ist

In einem mit molekularem Stickstoff (N₂-Gehalt ≥ 99,99 Vol.-%) und über Druckwasser von außen beheizbaren, ein Innenvolumen von 8 m³ aufweisenden, auf seiner Innenoberfläche emaillierten und mit Strombrechern sowie einem Impellerrührer ausgestatteten Rührkessel wurden 4602 kg iso-Butanol vorgelegt. Nach Inbetriebnahme des dreistufigen Impellerrührers wurde das iso-Butanol unter Rückfluss auf 90°C erhitzt. Unter Beibehalt dieser Temperatur wurde nun damit begonnen, über eine Förderschnecke 690 kg feinteiliges Vanadinpentoxid kontinuierlich zuzuführen. Nachdem innerhalb von 20 min 460 kg Vanadinpentoxid (V₂O₅) zugegeben waren, wurde unter Fortsetzung der Zufuhr von Vanadinpentoxid mit dem kontinuierlichen Zupumpen von 805 kg 105 gew.-%iger Phosphorsäure (vgl. DE-A 3520053) begonnen, wobei die Temperatur von 90°C beibehalten wurde (Pumprate = 160 kg/h).

Nach beendeter Zugabe der Phosphorsäure wurde das Gemisch unter Rühren und unter Rückfluss auf Temperaturen im Bereich von 100 bis 108°C erhitzt und während 14 Stunden in diesem Temperaturbereich gehalten. Anschließend wurde die erhaltene heiße Suspension innerhalb von 75 Minuten im Wesentlichen linear auf 60°C abgekühlt und 22,7 kg Fe-(III)-Phosphat-Hydrat zugegeben (Fe-Gehalt: 29,9 Gew.-%; Lieferant: Firma Dr. Paul Lohmann; frei von Fe-(II)-Verunreinigungen). Dann wurde wieder unter Rückfluss auf Temperaturen von 100 bis 108°C erhitzt und die Suspension unter weiterem Rühren für 1 Stunde bei dieser Temperatur gehalten. Anschließend wurde die ca. 105°C heiße Suspension in eine zuvor mit Stickstoff inertisierte sowie auf 100°C beheizte Druckfilternutsche abgelassen und bei einem Druck oberhalb der Filternutsche von ca. 0,35 Mpa abs. abfiltriert. Der anfallende Nutschkuchen wurde durch stetiges Einleiten von Stickstoff unter Rühren und bei 100°C innerhalb von einer Stunde trocken geblasen. Nach dem Trockenblasen wurde auf 155°C aufgeheizt und auf einen Druck von 15 kPa abs. (150 mbar abs.) evakuiert.

Unter diesen Bedingungen wurde die Trocknung bis zu einem Restgehalt an iso-Butanol von 2 Gew.-% in der getrockneten Vorläufermasse durchgeführt. Diese enthielt Fe und V im molaren Verhältnis FeN von 0,016.

Anschließend wurde das Trockenpulver in einem geneigten Drehrohr aus Edelstahl, das von 100 m³/h Luft (deren Eintrittstemperatur 150°C betrug) durchströmt wurde und innenliegende spiralförmige Wendeln aufwies, weiterbehandelt. Die Rohrlänge betrug 6,5 m, der Innendurchmesser 0,9 m und die Drehrohrwanddicke war 2,5 cm. Die Drehzahl des Drehrohres betrug 0,4 U/min. Das Trockenpulver wurde dem Drehrohrinneren in einer Menge von 60 kg/h an dessen oberem Ende zugeführt. Die spiralförmigen Wendeln gewährleisteten eine gleichförmige Rieselbewegung (abwärts) des Trockenpulvers innerhalb des Drehrohres. Die Drehrohrlänge war in fünf Heizzonen gleicher Länge aufgeteilt, die von außen temperiert wurden. Die auf der Außenwand des Drehrohres gemessenen Temperaturen der fünf Heizzonen betrugen von oben nach unten 250°C, 300°C, 345°C, 345°C und 345°C.

400 g des das Drehrohr verlassenden Pulvers wurden, bezogen auf ihr Gewicht, mit 1 Gew.-% Graphit (Asbury 3160, Fa. Timcal Ltd., vgl. WO 2008/087116) homogen vermischt (in einem Rhönradmischer mit 650 mm Raddurchmesser, 5 l Trommelvolumen, Drehzahl: 30 U/min, Mischdauer: 30 min.). Das resultierende Gemisch wurde dann mit Hilfe eines Walzenkompaktors der Fa. Powtec mit 2 gegenläufigen Stahlwalzen bei einem Pressdruck von 9 bar kompaktiert und nachfolgend durch ein Sieb mit quadratischen Siebmaschen einer Weite von 1 mm gedrückt. Das resultierende Kompaktat wies ein Schüttgewicht von 1100 g/l und eine im Wesentlichen einheitliche Körnung von ≥ 0,7 mm und ≤ 0,8 mm auf. 30 ml Schüttvolumen des Granulats wurden in einen vertikalen Rohrofen gefüllt (Rohrinnendurchmesser: 26 mm; im Zentrum des Rohres von oben nach unten verlaufende Thermohülse mit einem Außendurchmesser von 4 mm zur Aufnahme eines Thermoelements). Durch den Rohrofen wurden 25 Nl/h Luft mit einer Eintrittstemperatur von 160°C geführt. Mit einer Heizrate von 5°C/min. wurde die Temperatur des im Rohrofen befindlichen Calcinationsgutes von 25°C auf 250°C erhöht. Nach Erreichen der Temperatur von 250°C wurde die Temperatur des Calcinationsgutes mit einer Heizrate von 2°C/min auf 330°C angehoben. Diese Temperatur wurde über einen Zeitraum von 40 min. gehalten.

Dann wurde unter Beibehaltung eines Volumenstroms von 25 Nl/h von einer Luftdurchströmung auf eine Durchströmung des Rohrofens mit einem Gemisch aus 50 Vol.-% N₂ und 50 Vol.-% Wasserdampf umgestellt (dessen Eintrittstemperatur betrug 160°C) und die Temperatur des Calcinationsgutes mit einer Heizrate von 3°C/min auf 425°C angehoben. Diese Temperatur wurde über einen Zeitraum von 180°C gehalten. Dann wurde unter Beibehaltung des Volumenstroms von 25 Nl/h wieder auf eine Luftdurchströmung umgestellt (die Eintrittstemperatur des Luftstroms war 25°C). Dann wurde die Temperatur des Calcinationsgutes innerhalb von 120 min. auf 25°C abgekühlt.

Die Stöchiometrie des wie beschrieben hergestellten, mit Fe (III) dotierten Vanadin-Phosphoroxid-Aldolkondensationsvollkatalysators B war V₁P_{1,05}Fe_{0,016}Oₙ.

### II) Durchführung eines erfindungsgemäßen Verfahrens zur Herstellung von Acrylsäure aus Essigsäure und Methanol unter Verwendung der in I) hergestellten Katalysatoren (die Gehalte aller Reaktionsgaseingangsgemische und Edukte wurden gaschromatographisch bestimmt)

### 1. Gestaltung der Reaktionszone A

Die Realisierung der Reaktionszone A erfolgte in einem Rohrreaktor A (Innendurchmesser: 8 mm; Wandstärke: 1 mm; Länge: 100 mm; Material: Edelstahl DIN Werkstoff 1.4541), der von außen elektrisch beheizt werden konnte. Die Katalysatorbeschickung im Rohrreaktor A war wie folgt gestaltet:

| | |
|---|---|
| 30 mm | einer Vorschüttung aus Steatit-Splitt (1 bis 1,5 mm Längstausdehnung; Steatit C220 von CeramTec) am Reaktoreingang; und |
| 64 mm | mit 3,24 ml des splittförmigen Oxidationskatalysators A. |

Die Gehalte des Reaktionsgaseingangsgemischs A waren:

| | | |
|---|---|---|
| 9,15 | Vol.-% | Methanol, |
| 3,04 | Vol.-% | Wasser, |
| 77,76 | Vol.-% | N₂, und |
| 10,05 | Vol.-% | O₂. |

Das Reaktionsgaseingangsgemisch A (21,0 Nl/h) wurde der Vorschüttung aus Steatit-Splitt mit einer Eintrittstemperatur von 265°C zugeführt. Der Druck am Eingang in den Rohrreaktor A betrug 2 bar abs.. Die Temperatur des Rohrreaktors A wurde auf der Länge seiner Festbettbeschickung auf 265°C eingestellt (Außenwandtemperatur des Rohrreaktors A). Die Restlänge des Rohrreaktors A war unbeheizt. Die Belastung der Katalysatorbeschickung mit Reaktionsgaseingangsgemisch C war 6500 h⁻¹.

Das den Rohrreaktor A verlassende Produktgasgemisch A (22,0 Nl/h) wies folgende Gehalte auf (online GC-Analytik):

| | | |
|---|---|---|
| 0,07 | Vol.-% | Methanol, |
| 8,03 | Vol.-% | Formaldehyd, |
| 11,84 | Vol.-% | Wasser, |
| 73,71 | Vol.-% | N₂, |
| 4,83 | Vol.-% | O₂, |
| 0,40 | Vol.-% | CO₂, |
| 0,09 | Vol.-% | Ameisensäure, und |
| 0,04 | Vol.-% | Dimethylether. |

### 2. Gestaltung der Reaktionszone B

Die Realisierung der Reaktionszone B erfolgte in einem Rohrreaktor B (Innendurchmesser: 15 mm; Wandstärke: 1,2 mm; Länge: 2000 mm; Material: Edelstahl DIN Werkstoff 1.4541), der von außen elektrisch beheizt werden konnte. Die Katalysatorbeschickung im Rohrreaktor B war wie folgt gestaltet:

| | |
|---|---|
| 1000 mm | einer Vorschüttung aus Steatit-Splitt (wie in der Reaktionszone A) am Reaktoreingang; und |
| 753 mm | mit 133 ml des Aldolkondensationsvollkatalysators B. |

Aus den 22,0 Nl/h des Formaldehyd enthaltenden Produktgasgemischs A, 20,5 Nl/h eines gasförmigen Gemischs aus molekularem Stickstoff und in die Dampfphase überführter Essigsäure (99 Vol.-% Essigsäure), das die nachfolgende Gehalte

| | | |
|---|---|---|
| 90,49 | Vol.-% | N₂, und |
| 8,49 | Vol.-% | Essigsäure, |

aufwies,
und 2,6 Nl/h einer in die Dampfphase überführten Essigsäure (die den Stoffstrom Y abbildete), wurden 45,1 Nl/h des Reaktionsgaseingangsgemischs B erzeugt.

Die Gehalte der verdampften Essigsäure waren:

| | | |
|---|---|---|
| 99 | Vol.-% | Essigsäure. |

Die Gehalte des Reaktionsgaseingangsgemischs B waren:

| | | |
|---|---|---|
| 0,03 | Vol.-% | Methanol, |
| 3,91 | Vol.-% | Formaldehyd, |
| 5,76 | Vol.-% | Wasser, |
| 76,83 | Vol.-% | N₂, |
| 2,35 | Vol.-% | O₂, |
| 9,86 | Vol.-% | Essigsäure, |
| 0,19 | Vol.-% | CO₂, |
| 0,04 | Vol.-% | Ameisensäure, und |
| 0,02 | Vol.-% | Dimethylether. |

Das Reaktionsgaseingangsgemisch B wurde der Vorschüttung aus Steatit-Splitt mit einer Eintrittstemperatur von 320°C zugeführt. Der Druck am Eingang in den Rohrreaktor B betrug 1,5 bar abs.. Die Temperatur des Rohrreaktors B wurde auf der Länge seiner Festbettschüttung auf 340°C eingestellt (Außenwandtemperatur des Rohrreaktors B). Die Restlänge des Rohrreaktors B war unbeheizt. Die Belastung der Katalysatorbeschickung mit Reaktionsgaseingangsgemisch B war 340 h⁻¹ (Nl/I.h).

Das den Rohrreaktor B verlassende Produktgasgemisch B (45,1 Nl/h) wies folgende Gehalte auf (online GC-Analytik):

| | | |
|---|---|---|
| 0,03 | Vol.-% | Methanol, |
| 0,04 | Vol.-% | Formaldehyd, |
| 9,63 | Vol.-% | Wasser, |
| 76,83 | Vol.-% | N₂, |
| 2,27 | Vol.-% | O₂, |
| 3,76 | Vol.-% | Acrylsäure, |
| 6,04 | Vol.-% | Essigsäure, |
| 0,28 | Vol.-% | CO₂, |

| | | |
|---|---|---|
| 0,04 | Vol.-% | Ameisensäure, und |
| 0,02 | Vol.-% | Dimethylether. |

Bezogen auf die der Reaktionszone A zugeführte Menge an Methanol betrug die erzielte Ausbeute an Acrylsäure 88 mol-%.

Unter Einbezug einer Essigsäureherstellung durch homogen katalysierte Carbonylierung von Methanol in der Flüssigphase mit einer Selektivität der Essigsäurebildung von ≥ 99 mol-% beträgt die Ausbeute an Acrylsäure, bezogen auf die zu seiner Herstellung insgesamt eingesetzte Menge an Methanol, ca. 95,3 mol-%.

Im Hinblick auf die oben genannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

## Patentansprüche

1. Verfahren zur Herstellung von Acrylsäure aus Methanol und Essigsäure, das folgende Maßnahmen umfasst:
- durch eine erste Reaktionszone A, die mit wenigstens einem Oxidationskatalysator A beschickt ist, wird ein Strom eines die Reaktanden Methanol und molekularer Sauerstoff sowie wenigstens ein von Wasserdampf verschiedenes inertes Verdünnungsgas enthaltenden Reaktionsgaseingangsgemischs A hindurchgeführt und beim Durchströmen der Reaktionszone A im Reaktionsgaseingangsgemisch A enthaltenes Methanol heterogen katalysiert zu Formaldehyd und Wasserdampf oxidiert, so dass ein Formaldehyd, Wasserdampf, wenigstens ein von Wasserdampf verschiedenes inertes Verdünnungsgas sowie gegebenenfalls überschüssigen molekularen Sauerstoff enthaltendes Produktgasgemisch A entsteht und ein Strom an Produktgasgemisch A die Reaktionszone A verlässt, wobei dem durch die Reaktionszone A strömenden Reaktionsgasgemisch A auf seinem Weg durch die Reaktionszone A wahlweise weiterer molekularer Sauerstoff und/oder weiteres inertes Verdünnungsgas zugeführt werden kann,
- wahlweise wird der die Reaktionszone A verlassende Strom an Produktgasgemisch A einer Trennzone T* zugeführt und in der Trennzone T* im Produktgasgemisch A gegebenenfalls noch enthaltenes nicht umgesetztes Methanol vom Produktgasgemisch A abgetrennt, wobei ein Formaldehyd enthaltendes Produktgasgemisch A* verbleibt und ein Strom an Produktgasgemisch A* die Reaktionszone A verlässt,
- aus dem Strom an Produktgasgemisch A oder aus dem Strom an Produktgasgemisch A* sowie wenigstens einem weiteren, Essigsäure enthaltenden, Stoffstrom, wird ein Strom eines Essigsäure, Wasserdampf, wenigstens ein von Wasserdampf verschiedenes inertes Verdünnungsgas, Formaldehyd und gegebenenfalls molekularen Sauerstoff enthaltenden Reaktionsgaseingangsgemischs B erzeugt, in welchem die enthaltene molare Menge n_{HAc} an Essigsäure größer ist, als die in ihm enthaltene molare Menge n_{Fd} an Formaldehyd,
- durch eine zweite Reaktionszone B, die mit wenigstens einem Aldolkondensationskatalysator B beschickt ist, wird der Strom des Reaktionsgaseingangsgemischs B hindurchgeführt und beim Durchströmen der Reaktionszone B im Reaktionsgaseingangsgemisch B enthaltenes Formaldehyd mit im Reaktionsgaseingangsgemisch B enthaltener Essigsäure heterogen katalysiert zu Acrylsäure und H₂O kondensiert, so dass ein Acrylsäure, Essigsäure, Wasserdampf, wenigstens ein von Wasserdampf verschiedenes inertes Verdünnungsgas sowie gegebenenfalls molekularen Sauerstoff enthaltendes Produktgasgemisch B entsteht und ein Strom an Produktgasgemisch B die Reaktionszone B verlässt, wobei dem durch die Reaktionszone B strömenden Reaktionsgasgemisch B auf seinem Weg durch die Reaktionszone B wahlweise weiterer molekularer Sauerstoff und/oder weiteres inertes Verdünnungsgas zugeführt werden kann,
- der die Reaktionszone B verlassende Strom an Produktgasgemisch B wird einer Trennzone T zugeführt und in der Trennzone T in wenigstens drei Stoffströme X, Y und Z aufgetrennt, wobei
- der im Stoffstrom X enthaltene Acrylsäurestrom größer ist, als der in den Stoffströmen Y und Z zusammengenommen enthaltene Acrylsäurestrom,
- der im Stoffstrom Y enthaltene Essigsäurestrom größer ist, als der in den Stoffströmen X und Z zusammengenommen enthaltene Essigsäurestrom,
- der im Stoffstrom Z enthaltene Strom an von Wasserdampf verschiedenem inertem Verdünnungsgas größer ist, als der in den Stoffströmen X und Y zusammengenommen enthaltene Stoffstrom an von Wasserdampf verschiedenem inertem Verdünnungsgas,
und
- der Stoffstrom Y wird in die Reaktionszone B rückgeführt und zur Erzeugung des Reaktionsgaseingangsgemischs B mitverwendet.

2. Verfahren nach Anspruch 1, **dadurch gekennzeichnet, dass** der wenigstens eine Oxidationskatalysator A eine katalytisch aktive Masse aufweist, die ein Mischoxid der allgemeinen Formel I,
[Fe₂(MoO₄)₃]₁[M¹ₘOₙ]_{q} (I),
in der die Variablen folgende Bedeutung aufweisen:
M¹ = Mo und/oder Fe, oder
Mo und/oder Fe sowie, bezogen auf die molare Gesamtmenge aus Mo und Fe, eine molare Gesamtmenge von bis zu 10 mol-% eines oder mehrere Elemente aus der Gruppe bestehend aus Ti, Sb, Sn, Ni, Cr, Ce, Al, Ca, Mg, V, Nb, Ag, Mn, Cu, Co, Si, Na, K, TI, Zr, W, Ir, Ta, As, P und B,
q = 0 bis 5,
m = 1 bis 3,
n = 1 bis 6,
ist.

3. Verfahren nach Anspruch 1 oder 2, **dadurch gekennzeichnet, dass** das Reaktionsgaseingangsgemisch A 2 bis 15 Vol.-% Methanol enthält.

4. Verfahren nach einem der Ansprüche 1 bis 3, **dadurch gekennzeichnet, dass** das Reaktionsgaseingangsgemisch A 0 bis 20 Vol.-% H₂O enthält.

5. Verfahren nach einem der Ansprüche 1 bis 4, **dadurch gekennzeichnet, dass** das Reaktionsgaseingangsgemisch A den molekularen Sauerstoff in einer molaren Menge nₒ und das Methanol in einer molaren Menge n_{Me} enthält und das Verhältnis no : n_{Me} wenigstens 1 oder größer als 1 ist.

6. Verfahren nach einem der Ansprüche 1 bis 5, **dadurch gekennzeichnet, dass** eine Teilmenge des Stoffstroms Y zur Erzeugung des Reaktionsgaseingangsgemischs A in die Reaktionszone A rückgeführt wird.

7. Verfahren nach einem der Ansprüche 1 bis 6, **dadurch gekennzeichnet, dass** das Reaktionsgaseingangsgemisch A als wenigstens ein von Wasserdampf verschiedenes inertes Verdünnungsgas N₂ enthält.

8. Verfahren nach einem der Ansprüche 1 bis 7, **dadurch gekennzeichnet, dass** das Methanol in der Reaktionszone A bei einer Reaktionstemperatur im Bereich von 250 bis 400°C zu Formaldehyd und Wasser oxidiert wird.

9. Verfahren nach einem der Ansprüche 1 bis 8, **dadurch gekennzeichnet, dass** die in dem wenigstens einen weiteren Stoffstrom enthaltene Essigsäure eine durch homogene Katalyse katalysierte Carbonylierung von Methanol in der Flüssigphase erzeugte Essigsäure ist.

10. Verfahren nach Anspruch 9, **dadurch gekennzeichnet, dass** der Katalysator Rh in Kombination mit HI und CH₃I umfasst.

11. Verfahren nach Anspruch 9 oder 10, **dadurch gekennzeichnet, dass** die in dem wenigstens einen weiteren Stoffstrom enthaltene Essigsäure eine vom Produktgemisch der homogen katalysierten Carbonylierung von Methanol zu Essigsäure in der Flüssigphase rektifikativ abgetrennte Essigsäure ist.

12. Verfahren nach einem der Ansprüche 1 bis 11, **dadurch gekennzeichnet, dass** der wenigstens eine weitere, Essigsäure enthaltende, Stoffstrom das Produktgasgemisch einer in Abwesenheit von Halogen-haltigen Verbindungen heterogen katalysierten Gasphasencarbonylierung von Methanol zu Essigsäure ist.

13. Verfahren nach einem der Ansprüche 1 bis 12, **dadurch gekennzeichnet, dass** die Reaktionstemperatur in der Reaktionszone B 200 bis 400°C beträgt.

14. Verfahren nach einem der Ansprüche 1 bis 13, **dadurch gekennzeichnet, dass** der Gehalt des Reaktionsgaseingangsgemischs B an Formaldehyd 0,5 bis 10 Vol-% beträgt.

15. Verfahren nach einem der Ansprüche 1 bis 14, **dadurch gekennzeichnet, dass** das Reaktionsgaseingangsgemisch B die Essigsäure in einer molaren Menge n_{HAc} und das Formaldehyd in einer molaren Menge n_{Fd} enthält und das Verhältnis n_{HAc} : n_{Fd} größer als 1 und ≤ 10 ist.

16. Verfahren nach einem der Ansprüche 1 bis 15, **dadurch gekennzeichnet, dass** der Essigsäuregehalt des Reaktionsgaseingangsgemischs B 1,5 bis 20 Vol.-% beträgt.

17. Verfahren nach einem der Ansprüche 1 bis 16, **dadurch gekennzeichnet, dass** der Wasserdampfgehalt des Reaktionsgaseingangsgemischs B 30 Vol.-% nicht überschreitet und 0,5 Vol.-% nicht unterschreitet.

18. Verfahren nach einem der Ansprüche 1 bis 17, **dadurch gekennzeichnet, dass** der wenigstens eine Aldokondensationskatalysator B eine katalytisch aktive Masse aufweist, die eine Multielementoxidaktivmasse der allgemeinen Formel II,
V₁P_{b}Fe_{c}X¹_{d}X²ₑOₙ (II),
in der die Variablen folgende Bedeutung aufweisen:
X¹ = Mo, Bi, Co, Ni, Si, Zn, Hf, Zr, Ti, Cr, Mn, Cu, B, Sn und/oder Nb,
X² = Li, K, Na, Rb, Cs und/oder TI,
b = 0,9 bis 2,0
c = ≥ 0 bis 0,1,
d = ≥ 0 bis 0,1,
e = ≥ 0 bis 0,1, und
n = der stöchiometische Koeffizient des Elements Sauerstoff, der durch die stöchiometrischen Koeffezienten der von Sauerstoff verschiedenen Elemente sowie deren Ladungzahl in II bestimmt wird,
ist.

19. Verfahren nach einem der Ansprüche 1 bis 18, **dadurch gekennzeichnet, dass** zur Auftrennung des Produktgasgemischs B in der Trennzone T das Produktgasgemisch B, wahlweise nach direkter und/oder indirekter Abkühlung desselben, in eine mit trennwirksamen Einbauten ausgerüstete Kondensationskolonne geleitet und innerhalb der Kondensationskolonne fraktionierend kondensiert wird und die Stoffströme X, Y und Z als voneinander getrennte Fraktionen aus der Kondensationskolonne herausgeführt werden.

20. Verfahren nach einem der Ansprüche 1 bis 18, **dadurch gekennzeichnet, dass** zur Auftrennung des Produktgasgemischs B in der Trennzone T das Produktgasgemisch B, wahlweise nach direkter und/oder indirekter Abkühlung desselben, in einer mit trennwirksamen Einbauten ausgestatteten Absorptionskolonne im Gegenstrom zu einem bei Normaldruck höher als Acrylsäure siedenden organischen Lösungsmittel geführt und die im Produktgasgemisch B enthaltene Essigsäure und Acrylsäure unter Erhalt eines Absorbats in das Lösungsmittel absorbiert wird, während ein Stoffstrom Z die Absorptionskolonne an deren Kopf verlässt, und vom Absorbat anschließend die Stoffströme X und Y durch fraktionierende Destillation desselben in einer Rektifikationskolonne als getrennte Fraktionen abgetrennt werden.

21. Verfahren nach einem der Ansprüche 1 bis 18, **dadurch gekennzeichnet, dass** zur Auftrennung des Produktgasgemischs B in der Trennzone T das Produktgasgemisch B, wahlweise nach direkter und/oder indirekter Abkühlung desselben, in einer mit trennwirksamen Einbauten ausgestatteten Absorptionskolonne im Gegenstrom zu einer wässrigen Lösung als Absorptionsmittel geführt und die im Produktgasgemisch B enthaltene Essigsäure und Acrylsäure unter Erhalt eines Absorbats in das Lösungsmittel absorbiert wird während ein Stoffstrom Z die Absorptionskolonne an deren Kopf verlässt, und vom Absorbat anschließend die Stoffströme X und Y durch fraktionierende Destillation desselben in einer Rektifikationskolonne als getrennte Fraktionen abgetrennt werden.

22. Acrylsäure, für die das Verhältnis V aus der in dieser Acrylsäure enthaltenen molaren Menge n¹⁴C an ¹⁴C-Atomkernen zu der in derselben Acrylsäure enthaltenen molaren Menge n¹²C an ¹²C-Atomkernen, V = n¹⁴C : n¹²C, größer als 0 und kleiner als das entsprechende im Kohlendioxid der Atmosphäre auf der Erde vorliegende molare Verhältnis V* von ¹⁴C-Atomkernen zu ¹²C-Atomkernen ist.

23. Acrylsäure nach Anspruch 22, **dadurch gekennzeichnet, dass** V = (2/3) ·V* ist.

24. Wenigstens 1 kg Acrylsäure enthaltende flüssige Phase P, **dadurch gekennzeichnet, dass** die enthaltene Acrylsäure eine Acrylsäure gemäß Anspruch 22 oder 23 ist.

## Claims

1. A process for preparing acrylic acid from methanol and acetic acid, which comprises the following measures:
- a stream of a reaction gas input mixture A comprising the methanol and molecular oxygen reactants and at least one inert diluent gas other than steam is conducted through a first reaction zone A charged with at least one oxidation catalyst A and, in the course of passage through reaction zone A, methanol present in the reaction gas input mixture A is oxidized under heterogeneous catalysis to formaldehyde and steam so as to form a product gas mixture A comprising formaldehyde, steam and at least one inert diluent gas other than steam, with or without excess molecular oxygen, and a stream of product gas mixture A leaves reaction zone A, it optionally being possible to supply further molecular oxygen and/or further inert diluent gas to the reaction gas mixture A flowing through reaction zone A on its way through reaction zone A,
- optionally, the stream of product gas mixture A leaving reaction zone A is fed to a separation zone T* and any unconverted methanol still present in product gas mixture A in separation zone T* is removed from product gas mixture A to leave a formaldehyde-comprising product gas mixture A*, and a stream of product gas mixture A* leaves reaction zone A,
- a stream of a reaction gas input mixture B which comprises acetic acid, steam, at least one inert diluent gas other than steam and formaldehyde, with or without molecular oxygen, and in which the molar amount n_{HAc} of acetic acid present is greater than the molar amount n_{Fd} of formaldehyde present therein is obtained from the stream of product gas mixture A or from the stream of product gas mixture A* and at least one further stream comprising acetic acid,
- the stream of reaction gas input mixture B is passed through a second reaction zone B charged with at least one aldol condensation catalyst B and formaldehyde present in reaction gas input mixture B, as it flows through reaction zone B, is condensed with acetic acid present in reaction gas input mixture B under heterogeneous catalysis to give acrylic acid and H₂O, so as to form a product gas mixture B comprising acrylic acid, acetic acid, steam and at least one inert diluent gas other than steam, with or without molecular oxygen, and a stream of product gas mixture B leaves reaction zone B, it optionally being possible to supply further molecular oxygen and/or further inert diluent gas to the reaction gas mixture B flowing through reaction zone B on its way through reaction zone B,
- the stream of product gas mixture B leaving reaction zone B is fed to a separation zone T and separated in separation zone T into at least three streams X, Y and Z,
- the acrylic acid flow present in stream X being greater than the acrylic acid flow present in streams Y and Z together,
- the acetic acid flow present in stream Y being greater than the acetic acid flow present in streams X and Z together,
- the flow of inert diluent gas other than steam present in stream Z being greater than the flow of inert diluent gas other than steam present in streams X and Y together,
and
- stream Y is recycled into reaction zone B and used to obtain reaction gas input mixture B.

2. The process according to claim 1, wherein the at least one oxidation catalyst A comprises a catalytically active material which is a mixed oxide of the general formula I
[Fe₂(MoO₄)₃]₁[M¹ₘOₙ]_{q} (I)
in which the variables are each defined as follows:
M¹ = Mo and/or Fe, or
Mo and/or Fe and, based on the total molar amount of Mo and Fe, a total molar amount of up to 10 mol% of one or more elements from the group consisting of Ti, Sb, Sn, Ni, Cr, Ce, Al, Ca, Mg, V, Nb, Ag, Mn, Cu, Co, Si, Na, K, Tl, Zr, W, Ir, Ta, As, P and B,
q = 0 to 5,
m = 1 to 3,
n = 1 to 6.

3. The process according to either of claims 1 and 2, wherein reaction gas input mixture A comprises 2 to 15% by volume of methanol.

4. The process according to any of claims 1 to 3, wherein reaction gas input mixture A comprises 0 to 20% by volume of H₂O.

5. The process according to any of claims 1 to 4, wherein reaction gas input mixture A comprises the molecular oxygen in a molar amount n_{O} and the methanol in a molar amount n_{Me}, and the n_{O} : n_{Me} ratio is at least 1 or greater than 1.

6. The process according to any of claims 1 to 5, wherein a portion of the stream Y is recycled into reaction zone A to obtain reaction gas input mixture A.

7. The process according to any of claims 1 to 6, wherein reaction gas input mixture A comprises N₂ as the at least one inert diluent gas other than steam.

8. The process according to any of claims 1 to 7, wherein the methanol is oxidized to formaldehyde and water in reaction zone A at a reaction temperature in the range from 250 to 400°C.

9. The process according to any of claims 1 to 8, wherein the acetic acid present in the at least one further stream is acetic acid obtained by homogeneous catalysis catalyzed carbonylation of methanol in the liquid phase.

10. The process according to claim 9, wherein the catalyst comprises Rh in combination with HI and CH₃I.

11. The process according to claim 9 or 10, wherein the acetic acid present in the at least one further stream is acetic acid removed by rectification from the product mixture of the homogeneously catalyzed carbonylation of methanol to acetic acid in the liquid phase.

12. The process according to any of claims 1 to 11, wherein the at least one further stream comprising acetic acid is the product gas mixture of a heterogeneously catalyzed gas phase carbonylation of methanol to acetic acid in the absence of halogenated compounds.

13. The process according to claims 1 to 12, wherein the reaction temperature in reaction zone B is 200 to 400°C.

14. The process according to any of claims 1 to 13, wherein the formaldehyde content in reaction gas input mixture B is 0.5 to 10% by volume.

15. The process according to any of claims 1 to 14, wherein reaction gas input mixture B comprises acetic acid in a molar amount n_{HAc} and formaldehyde in a molar amount n_{Fd}, and the n_{HAc}:n_{Fd} ratio is greater than 1 and ≤ 10.

16. The process according to any of claims 1 to 15, wherein the acetic acid content of reaction gas input mixture B is 1.5 to 20% by volume.

17. The process according to any of claims 1 to 16, wherein the steam content of reaction gas input mixture B does not exceed 30% by volume and is not less than 0.5% by volume.

18. The process according to any of claims 1 to 17, wherein the at least one aldol condensation catalyst B has a catalytically active material which is a multielement oxide active material of the general formula II
V₁P_{b}Fe_{c}X¹_{d}X²ₑOₙ (II)
in which the variables are each defined as follows:
X¹ = Mo, Bi, Co, Ni, Si, Zn, Hf, Zr, Ti, Cr, Mn, Cu, B, Sn and/or Nb,
X² = Li, K, Na, Rb, Cs and/or TI,
b= 0.9 to 2.0
c= ≥ 0 to 0.1,
d= ≥ 0 to 0.1,
e= ≥ 0 to 0.1, and
n = the stoichiometric coefficient of the element oxygen, which is determined by the stoichiometric coefficients of the non-oxygen elements and the charge numbers thereof in II.

19. The process according to any of claims 1 to 18, wherein product gas mixture B is separated in separation zone T by passing product gas mixture B, optionally after direct and/or indirect cooling thereof, into a condensation column equipped with separating internals and fractionally condensing it within the condensation column and conducting streams X, Y and Z out of the condensation column as separate fractions.

20. The process according to any of claims 1 to 18, wherein product gas mixture B is separated in separation zone T by passing product gas mixture B, optionally after direct and/or indirect cooling thereof, into an absorption column equipped with separating internals in countercurrent to an organic solvent with a higher boiling point than acrylic acid at standard pressure, and absorbing the acetic acid and acrylic acid present in product gas mixture B into the solvent to obtain an absorbate, while a stream Z leaves the absorption column at the top thereof, and then removing streams X and Y from the absorbate as separate fractions by fractional distillation thereof in a rectification column.

21. The process according to any of claims 1 to 18, wherein product gas mixture B is separated in separation zone T by passing product gas mixture B, optionally after direct and/or indirect cooling thereof, into an absorption column equipped with separating internals in countercurrent to an aqueous solution as an absorbent, and absorbing the acetic acid and acrylic acid present in product gas mixture B into the solvent to obtain an absorbate, while a stream Z leaves the absorption column at the top thereof, and then removing streams X and Y as separate fractions from the absorbate by fractional distillation thereof in a rectification column.

22. Acrylic acid for which the ratio V of the molar amount n¹⁴C of ¹⁴C atomic nuclei present in this acrylic acid to the molar amount n¹²C of ¹²C atomic nuclei present in the same acrylic acid, V = n¹⁴C : n¹²C, is greater than 0 and less than the corresponding molar ratio V* of ¹⁴C atomic nuclei to ¹²C atomic nuclei present in the carbon dioxide in the earth's atmosphere.

23. Acrylic acid according to claim 22, wherein V = (2/3) V*.

24. A liquid phase P comprising at least 1 kg of acrylic acid, wherein the acrylic acid present is an acrylic acid according to claim 22 or 23.

## Revendications

1. Procédé pour la préparation d'acide acrylique à partir de méthanol et d'acide acétique, qui comprend les mesures suivantes :
- on fait passer, à travers une première zone de réaction A, qui est pourvue d'au moins un catalyseur d'oxydation A, un flux d'un mélange d'entrée gazeux réactionnel A contenant les réactifs méthanol et oxygène moléculaire ainsi qu'au moins un gaz de dilution inerte, différent de la vapeur d'eau, et lors de l'écoulement à travers la zone de réaction A, le méthanol contenu dans le mélange d'entrée gazeux réactionnel A est oxydé, sous catalyse par voie hétérogène, en formaldéhyde et en vapeur d'eau, de telle sorte qu'on obtient un mélange gazeux produit A contenant du formaldéhyde, de la vapeur d'eau, au moins un gaz de dilution inerte différent de la vapeur d'eau ainsi que le cas échéant de l'oxygène moléculaire en excès, et un flux de mélange gazeux produit A quitte la zone de réaction A, le mélange gazeux réactionnel A s'écoulant à travers la zone de réaction A pouvant être alimenté, sur son trajet au travers de la zone de réaction A, au choix, en oxygène moléculaire supplémentaire et/ou en gaz de dilution inerte supplémentaire,
- au choix, le flux de mélange gazeux produit A quittant la zone de réaction A est introduit dans une zone de séparation T* et, dans la zone de séparation T*, on sépare le méthanol non transformé le cas échéant encore contenu dans le mélange gazeux produit A du mélange gazeux produit A, un mélange gazeux produit A* contenant du formaldéhyde restant et un flux de mélange gazeux produit A* quittant la zone de réaction A,
- à partir du flux de mélange gazeux produit A ou du flux de mélange gazeux produit A* ainsi qu'à partir d'au moins un autre flux de substances, contenant de l'acide acétique, on génère un flux d'un mélange d'entrée gazeux réactionnel B contenant de l'acide acétique, de la vapeur d'eau, au moins un gaz de dilution inerte différent de la vapeur d'eau, du formaldéhyde et le cas échéant de l'oxygène moléculaire, dans lequel la quantité molaire contenue n_{HAc} d'acide acétique est supérieure à la quantité molaire n_{Fd} de formaldéhyde qu'il contient,
- on fait passer, à travers une deuxième zone de réaction B, qui est pourvue d'au moins un catalyseur de condensation d'aldol B, un flux du mélange d'entrée gazeux réactionnel B et lors de l'écoulement à travers la zone de réaction B, le formaldéhyde contenu dans le mélange d'entrée gazeux réactionnel B est condensé, sous catalyse par voie hétérogène, avec l'acide acétique contenu dans le mélange d'entrée gazeux réactionnel B en acide acrylique et en H₂O, de telle sorte qu'on obtient un mélange gazeux produit B contenant de l'acide acrylique, de l'acide acétique, de la vapeur d'eau, au moins un gaz de dilution inerte différent de la vapeur d'eau ainsi que le cas échéant de l'oxygène moléculaire, et un flux de mélange gazeux produit B quitte la zone de réaction B, le mélange gazeux réactionnel B s'écoulant à travers la zone de réaction B pouvant être alimenté, sur son trajet à travers la zone de réaction B, au choix, en oxygène moléculaire supplémentaire et/ou en gaz de dilution inerte supplémentaire,
- le flux de mélange gazeux produit B quittant la zone de réaction B est introduit dans une zone de séparation T et séparé, dans la zone de séparation T, en au moins trois flux de substances X, Y et Z,
- le flux d'acide acrylique contenu dans le flux de substances X étant supérieur au flux d'acide acrylique contenu dans les flux de substances Y et Z pris ensemble,
- le flux d'acide acétique contenu dans le flux de substances Y étant supérieur au flux d'acide acétique contenu dans les flux de substances X et Z pris ensemble,
- le flux de gaz de dilution inerte différent de la vapeur d'eau contenu dans le flux de substances Z étant supérieur au flux de gaz de dilution inerte différent de la vapeur d'eau contenu dans les flux de substances X et Y pris ensemble, et
- le flux de substances Y est recyclé dans la zone de réaction B et utilisé conjointement pour la génération du mélange d'entrée gazeux réactionnel B.

2. Procédé selon la revendication 1, **caractérisé en ce que** ledit au moins un catalyseur d'oxydation A présente une masse catalytiquement active, qui est un oxyde mixte de formule générale I,
[Fe₂(MoO₄)₃]₁[M¹ₙOₙ]_{q} (I),
dans laquelle les variables ont la signification suivante :
M¹ Mo et/ou Fe, ou
Mo et/ou Fe ainsi que, par rapport à la quantité molaire totale de Mo et de Fe, une quantité molaire totale de jusqu'à 10% en mole d'un ou de plusieurs éléments du groupe constitué par Ti, Sb, Sn, Ni, Cr, Ce, Al, Ca, Mg, V, Nb, Ag, Mn, Cu, Co, Si, Na, K, Tl, Zr, W, Ir, Ta, As, P et B,
q = 0 à 5,
m = 1 à 3,
n = 1 à 6.

3. Procédé selon la revendication 1 ou 2, **caractérisé en ce que** le mélange d'entrée gazeux réactionnel A contient 2 à 15% en volume de méthanol.

4. Procédé selon l'une quelconque des revendications 1 à 3, **caractérisé en ce que** le mélange d'entrée gazeux réactionnel A contient 0 à 20% en volume de H₂O.

5. Procédé selon l'une quelconque des revendications 1 à 4, **caractérisé en ce que** le mélange d'entrée gazeux réactionnel A contient l'oxygène moléculaire en une quantité molaire n_{O} et le méthanol en une quantité molaire n_{Me} et le rapport n_{O}:n_{Me} vaut au moins 1 ou est supérieur à 1.

6. Procédé selon l'une quelconque des revendications 1 à 5, **caractérisé en ce qu'**une quantité partielle du flux de substances Y est recyclée dans la zone de réaction A pour la génération du mélange d'entrée gazeux réactionnel A.

7. Procédé selon l'une quelconque des revendications 1 à 6, **caractérisé en ce que** le mélange d'entrée gazeux réactionnel A contient N₂ en tant qu'au moins un gaz de dilution inerte différent de la vapeur d'eau.

8. Procédé selon l'une quelconque des revendications 1 à 7, **caractérisé en ce que** le méthanol dans la zone de réaction A est oxydé à une température de réaction dans la plage de 250 à 400°C en formaldéhyde et en eau.

9. Procédé selon l'une quelconque des revendications 1 à 8, **caractérisé en ce que** l'acide acétique contenu dans ledit au moins un autre flux de substances est un acide acétique généré par carbonylation, catalysée par une catalyse homogène, de méthanol en phase liquide.

10. Procédé selon la revendication 9, **caractérisé en ce que** le catalyseur comprend du Rh en combinaison avec HI et CH₃I.

11. Procédé selon la revendication 9 ou 10, **caractérisé en ce que** l'acide acétique contenu dans ledit au moins un autre flux de substances est un acide acétique séparé par rectification du mélange produit de la carbonylation, catalysée par voie homogène, de méthanol en acide acétique en phase liquide.

12. Procédé selon l'une quelconque des revendications 1 à 11, **caractérisé en ce que** ledit au moins un autre flux de substances, contenant de l'acide acétique, est le mélange gazeux produit d'une carbonylation en phase gazeuse, catalysée par voie hétérogène en l'absence de composés halogénés, de méthanol en acide acétique.

13. Procédé selon l'une quelconque des revendications 1 à 12, **caractérisé en ce que** la température de réaction dans la zone de réaction B est de 200 à 400°C.

14. Procédé selon l'une quelconque des revendications 1 à 13, **caractérisé en ce que** la teneur en formaldéhyde du mélange d'entrée gazeux réactionnel B est de 0,5 à 10% en volume.

15. Procédé selon l'une quelconque des revendications 1 à 14, **caractérisé en ce que** le mélange d'entrée gazeux réactionnel B contient l'acide acétique en une quantité molaire n_{HAc} et le formaldéhyde en une quantité molaire n_{Fd} et le rapport n_{HAc}:n_{Fd} est supérieur à 1 et ≤ 10.

16. Procédé selon l'une quelconque des revendications 1 à 15, **caractérisé en ce que** la teneur en acide acétique du mélange d'entrée gazeux réactionnel B est de 1,5 à 20% en volume.

17. Procédé selon l'une quelconque des revendications 1 à 16, **caractérisé en ce que** la teneur en vapeur d'eau du mélange d'entrée gazeux réactionnel B ne dépasse pas 30% en volume et ne passe pas sous 0,5% en volume.

18. Procédé selon l'une quelconque des revendications 1 à 17, **caractérisé en ce que** ledit au moins un catalyseur de condensation d'aldol B présente une masse catalytiquement active, qui est une masse active oxyde d'éléments multiples de formule générale II,
V₁P_{b}Fe_{c}X¹_{d}X²ₑOₙ (II),
dans laquelle les variables ont la signification suivante :
X¹ = Mo, Bi, Co, Ni, Si, Zn, Hf, Zr, Ti, Cr, Mn, Cu, B, Sn et/ou Nb,
X² = Li, K, Na, Rb, Cs et/ou Tl,
b = 0,9 à 2,0
c = ≥ 0 à 0, 1,
d = ≥ 0 à 0, 1,
e = ≥ 0 à 0, 1, et
n = le coefficient stoechiométrique de l'élément oxygène, qui est déterminé par les coefficients stoechiométriques des éléments différents de l'oxygène ainsi que de leur valence dans II.

19. Procédé selon l'une quelconque des revendications 1 à 18, **caractérisé en ce que**, pour la séparation du mélange gazeux produit B dans la zone de séparation T, le mélange gazeux produit B est guidé, au choix après un refroidissement direct et/ou indirect de celui-ci, dans une colonne de condensation équipée d'inserts actifs en séparation et est condensé par fractionnement dans la colonne de condensation et les flux de substances X, Y et Z sont évacués de la colonne de condensation sous forme de fraction séparées les unes des autres.

20. Procédé selon l'une quelconque des revendications 1 à 18, **caractérisé en ce que**, pour la séparation du mélange gazeux produit B dans la zone de séparation T, le mélange gazeux produit B est guidé, au choix après refroidissement direct et/ou indirect de celui-ci, dans une colonne d'absorption équipée d'inserts actifs en séparation, à contre-courant par rapport à un solvant organique présentant un point d'ébullition supérieur, à pression normale, à celui de l'acide acrylique et l'acide acétique et l'acide acrylique contenus dans le mélange gazeux produit B sont absorbés dans le solvant avec obtention d'un absorbat, pendant qu'un flux de substances Z quitte la colonne d'absorption via sa tête et les flux de substances X et Y sont ensuite séparés de l'absorbat par distillation fractionnée de celui-ci dans une colonne de rectification sous forme de fractions séparées.

21. Procédé selon l'une quelconque des revendications 1 à 18, **caractérisé en ce que**, pour la séparation du mélange gazeux produit B dans la zone de séparation T, le mélange gazeux produit B est guidé, au choix après refroidissement direct et/ou indirect de celui-ci, dans une colonne d'absorption équipée d'inserts actifs en séparation, à contre-courant par rapport à une solution aqueuse comme absorbant et l'acide acétique et l'acide acrylique contenus dans le mélange gazeux produit B sont absorbés dans le solvant avec obtention d'un absorbat, pendant qu'un flux de substances Z quitte la colonne d'absorption via sa tête et les flux de substances X et Y sont ensuite séparés de l'absorbat par distillation fractionnée de celui-ci dans une colonne de rectification sous forme de fractions séparées.

22. Acide acrylique, pour lequel le rapport V de la quantité molaire n¹⁴C de noyaux d'atomes de ¹⁴C contenue dans cet acide acrylique à la quantité molaire n¹²C de noyaux d'atomes de ¹²C contenue dans le même acide acrylique, V = n¹⁴C:n¹²C, est supérieur à 0 et inférieur au rapport molaire V* de noyaux d'atomes de ¹⁴C aux noyaux d'atomes de ¹²C correspondant qui existe dans le dioxyde de carbone de l'atmosphère sur terre.

23. Acide acrylique selon la revendication 22, **caractérisé en ce que** V = (2/3)·V*.

24. Phase liquide P contenant au moins 1 kg d'acide acrylique, **caractérisé en ce que** l'acide acrylique contenu est un acide acrylique selon la revendication 22 ou 23.
